(19) **Europäisches Patentamt**
**European Patent Office**
**Office européen des brevets**

(11) **EP 4 775 198 A1**

(12) **EUROPEAN PATENT APPLICATION**

(43) Date of publication:
**15.07.2026 Bulletin 2026/29**

(21) Application number: **26151632.2**

(22) Date of filing: **13.01.2026**

(51) International Patent Classification (IPC):
*A61K 8/11* (2006.01)  *A61K 8/44* (2006.01)
*A61Q 19/00* (2006.01)  *A61Q 19/02* (2006.01)

(52) Cooperative Patent Classification (CPC):
**A61Q 19/00; A61K 8/11; A61K 8/44; A61Q 19/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **14.01.2025 EP 25305041**

(71) Applicant: **ACM**
**92110 Clichy (FR)**

(72) Inventor: **ROBERT, Christelle**
**92150 Suresnes (FR)**

(74) Representative: **Novagraaf Technologies**
**2 rue Sarah Bernhardt**
**CS90017**
**92665 Asnières-sur-Seine Cedex (FR)**

(54) **TOPICAL COSMETIC COMPOSITION TO LIGHTEN SKIN PIGMENTATION**

(57) The invention belongs to the field of skin treatment. In particular, it relates to a topical cosmetic composition comprising tranexamic acid encapsulated in a liposome and a non-therapeutic cosmetic method comprising the application to the skin of said topical cosmetic composition. The topical cosmetic composition can be used in the treatment of melanin or hyperpigmented spot, brown spots, hyperpigmented spots such as ephelides, melasma, actinic lentigo, senile lentigo and post-inflammatory hyperpigmentation. In particular, the obtained results have demonstrated that, when topically applied, the composition advantageously improves the treatment of brown spots and/or hyperpigmentation spots.

EP 4 775 198 A1

## Description

### Technical domain

The invention belongs to the field of skin treatment. In particular, it relates to a cosmetic or dermatological composition comprising tranexamic acid encapsulated in a liposome, in particular, it relates to a whitening cosmetic or dermatological composition comprising tranexamic acid encapsulated in a liposome.

The invention also belongs to a non-therapeutic cosmetic skin whitening treatment method comprising the application to the skin of said cosmetic composition

### State of the art

50% of the world's population is affected by pigmentary disorders, only 38% of people with pigmentation disorders protect their skin all year round and 73% lack knowledge, particularly about the impact of the sun on pigmentation disorders.

Pigmentation spots affect all phototypes: Light phototypes (I to III) are more susceptible to sun spots (solar lentigos) due to less natural protection against UV rays. Dark phototypes (IV to VI) are more prone to post-inflammatory (PIH) spots. These spots often appear after injury, inflammation (acne, bites, burns), or aggressive treatments.

Greater risk of developing marked melasma, notably due to hormonal fluctuations (pregnancy, contraceptives).

Fewer visible solar lentigines, as basic pigmentation often masks these spots.

Regular exposure of the skin to ultraviolet (UV) radiation, as well as other stimuli such as inflammation or mechanical friction, promotes melanin production by melanocytes. Once melanized, these melanosomes are transferred to keratinocytes neighboring the melanocytes. These melanin-bearing melanosomes remain in the keratinocyte cytoplasm for some time.

The world's population is increasingly affected by hyperpigmentation

In the middle and upper layers of the epidermis, in caucasoids, i.e. subjects with white skin, melanized melanosomes are then incorporated into lysosomes for gradual degradation.

In the epidermis of Caucasoid subjects, melanin is absent from the stratum corneum due to its progressive destruction in the superficial epidermal layers. However, in melasma and lentigos appearance of permanent, unsightly hyperpigmented spots due to melanin overload in epidermal cells.

Melasma is often considered one of the most difficult types hyperpigmentation to treat, due to the following factors :multiple factors and pathways are involved in melasma, leading to an overproduction of melanin. Treatment must therefore be more holistic. It's a chronic pathology melasma can recur chronically due to hormonal, environmental or even genetic factors and last for many years.

The appearance of hyperpigmentation is the result of excessive melanin production. Once melanin has been produced in the melanocytes, it is distributed to the keratinocytes via the melanocytes' dendritic extensions, which extend between the keratinocytes. When production levels are normal, melanin gives the skin its color and acts as a natural photoprotector. When overproduction occurs, it manifests itself as hyperpigmented lesions.

Today, there are many treatments available to correct hyperpigmentation. Skin hyperpigmentation treatments aim to reduce or eliminate pigmentation spots. Their choice depends on the type of hyperpigmentation (melasma, sun spots, post-inflammatory, etc.), phototype and skin sensitivity, for example there are chemical peels, dermatological techniques, for example laser, Intense Pulsed Light (IPL) and cryotherapy, microneedling and mesotherapy, and medicated treatments.

In particular, chemical peels exfoliate the skin's surface layers to diminish the appearance of pigmentation spots. The acids used are Glycolic acid (AHA): For light to moderate pigmentation spots., Salicylic acid (BHA): ideal for oily or acne-prone skin and Trichloroacetic acid (TCA): for deeper pigmentation spots. Chemical peels are recommended for light to medium phototypes, but must be adapted for darker skins to avoid the rebound effect.

Laser, IPL and cryotherapy are dermatological techniques for destroying targeted melanin. For example Q-Switched or picosecond laser: specifically targets melanin, ideal for lentigos or sun spots, fractional laser (Fraxel): treats deeper layers and evens out skin tone, Intense Pulsed Light (IPL): Treats diffuse pigmentation spots and redness, particularly suitable for fair skins and cryotherapy uses liquid nitrogen to destroy superficial pigmentation spots (especially lentigos).

Microneedling and mesotherapy stimulate skin regeneration and lightening. In particular, microneedling stimulates cell renewal and promotes absorption of skin-lightening serums and mesotherapy injects skin-lightening composition into the deep layers of the skin.

But these treatments are not without risk, are often costly, and must be performed or prescribed by dermatologists in their own practices for greater safety.

Whitening compositions are known in the art and are commercially available. However, their efficiency is discussed, and they can comprise compounds or ingredients that could be toxic to melanocyte.

Treatments of severe melasma are also known. For example, tranexamic acid taken orally is known to treat melasma, particularly effective on darker skins or corticoids are known for the treatment of severe melasma, often combined with other active ingredients. It has been recently demonstrated that melasma is a multifactorial hyperpigmentation whose source is not limited to the melanocyte. It also has been demonstrated that oral tranexamic acid is effective on melasma only by acting on endothelial cells, in particular by reducing the vascularization component (endothelial cells) that stimulates melanocytes.

So there is a real need in the art to find a mean or a composition that allow to reduce/treat hyperpigmented spots with a better efficiency and/or less toxicity, for example for the skin cells.

**Description of the invention**

The present invention has precisely the objectives, in particular, of meeting the aforementioned needs of the prior art, by providing a topical cosmetic composition comprising tranexamic acid encapsulated in a liposome for use in the treatment of brown skin spots and/or hyperpigmented spots.

An object of the invention is also a cosmetic non-therapeutic use of a topical cosmetic composition comprising tranexamic acid encapsulated in a liposome for treating melanin or hyperpigmented spot.

The terms "treating", "treat" and "treatment" include (i) preventing a melanin or hyperpigmented spot from occurring (e.g., prophylaxis); (ii) inhibiting, for example the production of melanin and/or degrading the melanin and/or the melanin or hyperpigmented spot or arresting its development; and/or diminishing, for example, the melanin or hyperpigmented spot. Thus, the terms "treat", "treatment", and "treating" extend to prophylaxis and include prevent, prevention, preventing, lowering, stopping or reversing the progression or severity of the melanin or hyperpigmented spot.

In the present, a "melanin spot" refers to a localized area of skin where there is an accumulation or overproduction of melanin, the natural pigment responsible for the coloration of the skin, hair and eyes. These spots, also known as hyperpigmented spots, appear as darker patches or macules on the skin's surface, and can result from various factors, such as sun exposure, aging, hormonal changes or skin inflammation.

An object of the invention is also the cosmetic non-therapeutic use of a topical composition, preferably a topical cosmetic composition, comprising tranexamic acid encapsulated in a liposome for one or more of the following applications: brightening the skin, reducing and/or treating the skin brown spots, reducing and/or treating hyperpigmented spots.

In particular, an object of the invention is the non-therapeutic cosmetic use of a topical cosmetic composition comprising tranexamic acid encapsulated in a liposome for use in the non-therapeutic cosmetic treatment of melanin or hyperpigmented spot, such as ephelides, melasma, actinic lentigo, senile lentigo and post-inflammatory hyperpigmentation (PIH).

In the present, "skin brightening" refers to improving the overall radiance, clarity and evenness of the skin tone and/of the complexion, for example improving the luminosity of the skin, make the skin more vibrant and healthy, for example by reducing dullness, uneven pigmentation and discoloration.

In the present, "ephelides" refers to small, flat, brown or tan spots on the skin. The causes of ephelides are essentially due to sun exposure and an increase in melanin production.

In the present, "melasma" refers to a benign but unsightly skin condition in the form of hyperpigmented spots appearing on sun-exposed areas, especially on the face, cleavage and neck. Melasma mainly affects women, most often during pregnancy, giving what is commonly known as the pregnancy mask. The causes of melasma are essentially due to genetic predisposition, sun exposure and/or estrogen.

In the present, "lentigo" refers to a non-malignant spot, also known as an age spot. The causes of lentigo may be due to a localized increase in the number of melanocytes, the cells that create melanin.

In the present, "actinic lentigo", also mentioned solar lentigo, is a harmless spot of darkened skin. The actinic lentigo is due to ultraviolet (UV) radiation, or long-term sun exposition.

In the present, "senile lentigo", also mentioned age spot, refers to pigmented or hyperpigmented spots on the skin which may be due to hyperplasia of melanocytes and cell aging.

In the present, "post-inflammatory hyperpigmentation" (PIH) refers to hyperpigmented skin spot occurring after an inflammation or injury to the skin, for example acne, atopic dermatitis, aesthetic procedure. The causes of lentigo may be due to an overproduction of melanin.

In the present, the expression "cosmetic, non-therapeutic use" refers to the improvement of the aesthetic appearance of the skin, complexion, scalp, hair, nails, lips or other external body surfaces. For example, for improving or maintaining the appearance, feel or comfort of the skin without any therapeutic effect. For example, the cosmetic use excludes therapeutic, prophylactic or diagnostic purpose, including but not limited to the treatment, alleviation or prevention of pathological condition, disease of the skin or other tissues.

In the present, liposome may be any adapted liposome known from one skilled in the art and/or commercially available. It may be for example any adapted liposome known from one skilled in the art as disclosed in Akbarzadeh et al.

(2013) "Liposome: classification, preparation, and applications." Nanoscale Research Letters, 8(1): 102). It may be any liposome in any form and/or ingredient acceptable to the Scientific Committee on Consumer Safety (SCCS), and/or any other liposome listed in the INCI (International Nomenclature of Cosmetic Ingredients) dictionary published by the PCPC (Personal Care Products Council) that can encapsulated tranexamic acid. It may be for example liposome commercialized by Gattefossé, Croda, Seppic, DSM, Symrise, IFF-Lucas Meyer, Mibelle, Lipoid GmbH, Formulator Sample Shop, Making Cosmetics, Aromazone, Liposoma BV, Nanovex Biotechnologies, Rahn AG and/or Dermalab It may be for example Lipoid liposome commercialized by Lipoid GmbH. It may be for example Empty Liposomes 10% and/or 5% commercialized by Formulator Sample Shop. It may be nano Liposomes commercialized by Nanovex Biotechnologies. It may be Liposomal base commercialized by Rahn AG. It may be liposome Empty commercialized by Dermalab. From this general knowledge, the skilled person knows how to determine the liposomes that may be useful for encapsulating the acid.

The liposome may be obtained by any adapted process known from one skilled in the art. It may be for example a process as disclosed in Mozafari, M.R. (2005) "Liposomes: an overview of manufacturing techniques." Cellular and Molecular Biology Letters, 10(4): 711-719.

Melanogenesis is regulated by various epidermal and dermal cells dermis, in particular the keratinocytes produce cytokines and hormones that stimulate melanocytes, causing melanocytes to descend into the dermis, the endothelial cells play a key role in melasma by producing endothelin-1 which is a powerful activator of melanogenesis.

Another object of the invention is the non-therapeutic use of a composition, preferably a topical composition, preferably a topical cosmetic composition, according to the invention to improve the aspect of the complexion and/or of the skin, for example to render the complexion more radiant, fresher, more uniform, more unified, and/or to render the skin brighter, to hydrate or rehydrate the skin.

The cosmetic or dermatological composition according to the invention may be applied on any type of skin, and especially on any type of mammalian skin. For example, the cosmetic or dermatological composition according to the invention may be applied on skin presenting any phototype. For example, it may be applied on skin with a phototype chosen from one of the phototypes II to VI. For example, the cosmetic or dermatological composition according to the invention may be applied on skin presenting any one of phototypes as described in Fitzpatrick, T. B. (1988). « The validity and practicality of sun-reactive skin types I through VI », Archives of Dermatology, 124(6), 869-871.

Unless otherwise indicated, the concentration of compounds is expressed as a percentage by weight of the total weight of the composition.

According to the invention the composition can comprise, in % by weight relative to the total weight of the composition, from 0.01 to 1% of tranexamic acid, for example from 0.05 to 0.15, for example from 0.075 to 0.12%, for example from 0.1%, for example from 0.015 to 0.05%.

According to the invention, the composition can further comprise at least one ingredient selected from the group comprising ascorbic acid, zinc salt, olive leaf extract, oleuropeine, niacinamide, retinyl palmitate, hexylresorcinol, alpha hydroxy acid (aha) and poly hydroxy acid.

According to the invention, the composition can further comprise at least one phosphatase activator.

According to the invention, the composition can further comprise at least one ingredient selected from the group comprising ascorbic acid, zinc salt, olive leaf extract, oleuropeine, niacinamide, retinyl palmitate, hexylresorcinol, alpha hydroxy acid (aha), poly hydroxy acid and phosphatase activator.

In the present, the ascorbic acid can be any ascorbic acid acceptable to the Scientific Committee on Consumer Safety (SCCS) and/or any ascorbic acid listed in the INCI (International Nomenclature of Cosmetic Ingredients) dictionary published by the PCPC (Personal Care Products Council). For example, it may be ascorbic acid commercialized by Spec-Chem, Univar.

In the present the olive leaf extract can be any vegetable olive leaf extract known to the skilled person, for example it may be an olea europaea (olive) leaf extract. In the present the olea europaea (olive) leaf extract can comprise or consist of oleuropeine. For example, it may be olive leaf extract commercialized by Sinoplasan and/or Vimergy.

In the present, the zinc salt can be any zinc salt acceptable to the Scientific Committee on Consumer Safety (SCCS) and/or any zinc salt listed in the INCI (International Nomenclature of Cosmetic Ingredients) dictionary published by the PCPC (Personal Care Products Council). For example, it may be zinc PCA. For example, it may be zinc PCA commercialized by Alexmo-Cosmetics.

In the present, the retinyl palmitate can be any alpha retinyl palmitate acceptable to the Scientific Committee on Consumer Safety (SCCS) and/or any retinyl palmitate listed in the INCI (International Nomenclature of Cosmetic Ingredients) dictionary published by the PCPC (Personal Care Products Council). The retinyl palmitate can be added to the composition of the invention in any form and/or ingredient acceptable to the Scientific Committee on Consumer Safety (SCCS). For example, it can be retinyl palmitate encapsulated in a liposome. For example, it may be retinyl palmitate commercialized by Alexmo-Cosmetics.

In the present, the alpha hydroxy acid (aha) can be any alpha hydroxy acid acceptable to the Scientific Committee on Consumer Safety (SCCS) and/or any alpha hydroxy acid listed in the INCI (International Nomenclature of Cosmetic Ingredients) dictionary published by the PCPC (Personal Care Products Council). It may be for example glycolic acid, lactic acid, mandelic acid, and citric acid. For example, it may be glycolic acid commercialized by BASF. For example, it may be lactic acid commercialized by DKSH. For example, it may be mandelic acid commercialized by BASF. For example, it may be citric acid commercialized by ADM.

In the present, the poly hydroxy acid (pha) can be any poly hydroxy acid acceptable to the Scientific Committee on Consumer Safety (SCCS) and/or any poly hydroxy acid listed in the INCI (International Nomenclature of Cosmetic Ingredients) dictionary published by the PCPC (Personal Care Products Council). It may be for example gluconolactone, Lactobionic acid. For example, it may be gluconolactone commercialized by DKSH. For example, it may be lactobionic acid commercialized by DKSH.

According to the invention the composition can comprise, in % by weight relative to the total weight of the composition, from 0.01 to 0.1% of ascorbic acid, preferably from 0.01 to 0.03%. The composition can comprise, in % by weight relative to the total weight of the composition from 0.001 to 0.05% of ascorbic acid, for example from 0.001 to 0.03% of ascorbic acid, for example from 0.01 to 0.03%.

According to the invention the composition can comprise, in % by weight relative to the total weight of the composition, from 0.01 to 0.1% of olive leaf extract, preferably of olea europaea (olive) leaf extract, preferably of oleuropeine, more preferably between 0.01 and 0.09%.

According to the invention the composition can comprise, in % by weight relative to the total weight of the composition, from 1 to 10% of niacinamide, preferably from 0.4 to 4%.

According to the invention the composition can comprise, in % by weight relative to the total weight of the composition, from 0.01 to 1% of retinyl palmitate, preferably from 0.02 to 0.1%, more preferably from 0.01 to 002%.

According to the invention the composition can comprise, in % by weight relative to the total weight of the composition, from 0.01 and 1% of zinc salt, preferably from 0.05 to 1%.

According to the invention the composition can comprise, in % by weight relative to the total weight of the composition, from 0.1 to 10% of hexylresorcinol, preferably from 0.3 to 3%.

According to the invention the composition can comprise, in % by weight relative to the total weight of the composition, from 0.0001 to 10% of alpha hydroxy acid (aha), preferably from 0.1 to 5%, more preferably from 0.3 to 3%.

According to the invention the composition can comprise, in % by weight relative to the total weight of the composition, from 0.0001 to 10% of poly hydroxy acid, preferably from 0.1 to 5%, more preferably from 0.3 to 3%.

According to the invention the composition can further comprises at least one phosphatase activator selected from the group comprising magnesium ions ($Mg^{2+}$), cupric ions ($Cu^{2+}$), zinc ions ($Zn^{2+}$), manganese ions ($Mn^{2+}$) and mixtures thereof. It may be for example the combination of at least two phosphatase activators selected from the group comprising magnesium sulfate, manganese chloride, magnesium chloride, manganese sulfate.

According to the invention the composition can comprise, in % by weight relative to the total weight of the composition, from 0.01 to 10% of phosphatase activator, preferably from 0.01 to 3%, more preferably from 0.03 to 3%.

The composition according to the present invention may also include other active ingredients that can be used in cosmetics or dermatology.

The composition according to the invention can comprise one or more adjuvants known to the person skilled in the art. These may include, for example, one or more adjuvant(s) chosen from ester-type agents, emollient agents, mineral thickening agents, organic thickening agents, whether associative or not, mineral sunscreens, perfumes, preservatives, ceramides and pseudoceramides, vitamins and provitamins, proteins, sequestering agents, alkalizing agents, acidifying agents, reducing agents, oxidizing agents, mineral fillers, colorants or any other adjuvant listed in the INCI (International Nomenclature Cosmetic Ingredients) dictionary published by the PCPC (Personnal Care Products council).

The composition can be in any form known to the skilled person in the fields of cosmetics, without any particular galenic restrictions. The cosmetic or dermatological composition can be in any form known to the skilled person in the fields of cosmetics and dermatology, without any particular galenic restrictions. The composition, preferably cosmetic composition according to the invention may be in one of the following galenic forms: ointment, cream, oil, milk, stick, pad, transdermal patch, solution, gel, spray, lotion serum or suspension. Advantageously, the cosmetic or dermatological composition is a serum.

The composition can be a topical cosmetic composition for example for application to the surface of the skin, hair, nails, or other external body parts.

The inventors have observed clear improvements when the composition is a serum, as advantageously demonstrated in the following examples.

The cosmetic or dermatological composition, preferably cosmetic composition, according to the invention may be obtained by any suitable process known to the skilled person for the manufacture of a cosmetic and/or dermatological composition. This may involve, for example, simple mixing. It may also involve, for example, a process comprising a step of incorporating an internal phase into an external phase by means of an emulsifier, for example a rotor-stator type turbine.

An object of the present invention is the non-therapeutic cosmetic use of a topical composition, preferably a cosmetic composition, of the invention for brightening the skin, reducing and/or treating the skin brown spots, reducing and/or treating hyperpigmented spots.

An object of the present invention is a non-therapeutic cosmetic method comprising applying to the skin the cosmetic composition of the invention.

For this application, the galenic forms described above can be used, for example. Application to the skin can therefore be carried out according to the galenic form used. Advantageously, the composition is a serum.

This may involve, for example, simple application to the skin, or application accompanied by massage of the skin with the composition of the present invention.

The application is preferably made with a sufficient quantity of the composition so that the entire surface of the skin to be treated is treated. For example, it may be a conventional application, such as a cream on the skin. It may also, for example, be applied to form a treatment mask.

The application can be any application desired by the user, for example a daily, twice-daily, weekly and/or twice-monthly application. It may be, for example, a once-a-day, twice-a-day or more frequent application. Preferably, the application can be twice-daily.

The inventors have demonstrated that, when topically applied, the composition of the invention advantageously and significantly reduces the spot intensity, for example composition of the invention advantageously and significantly reduce the intensity of melasma spot intensity, acne-related PIH pigment spots, post-acne marks and/or lentigo spots.

The inventors have demonstrated that, when topically applied, the composition of the invention advantageously and significantly increase skin tone uniformity, radiance and/or luminosity.

The inventors have demonstrated that, when topically applied, the composition of the invention advantageously improves the treatment of brown spots/blemishes and/or hyperpigmentation spots/blemishes. The obtained results have also surprisingly demonstrated that the composition of the invention allows an increase of at least eight times in the cutaneous penetration of tranexamic acid, until the endothelial cells. In addition, the obtained results demonstrate that the absorption of encapsulated tranexamic acid into the skin is faster than free tranexamic acid. The results also demonstrate that the composition is well tolerated and allows the treatment of treat brown spots, hyperpigmented spots. Advantageously, the composition of the invention advantageously regulates the stimulation of melanocyte and thus reduces the production of melanin.

In addition, when topically applied, the composition of the invention advantageously allows targeting all the pathways and pigments responsible, for example melanin for hyperpigmentation on all phototypes.

In addition, the composition of the invention advantageously allows synergically to eliminates melanin on the surface of the skin, in particular, the aha, pha and retinyl palmitate act on existing melanin in the surface layers of the skin, inhibits melanogenesis, in particular niacinamide block melanin transfer to keratinocytes, reduces inflammation and inhibits release of inflammatory cytokines by keratinocytes and hexylresorcinol Inhibits tyrosinase activity in melanocytes.

Advantageously, the composition of the invention advantageously allows synergically to eliminate melanin on the surface of the skin, inhibits melanogenesis, reduces the melanogenesis with the regulation of the melanocytes, the reduction of the inflammation and inhibits release of inflammatory cytokines by keratinocytes and its antioxidating effect and reduction of oxidative stress.

In other words, the composition of the invention advantageously can reduce oxidative stress, eliminates surface melanin, adsorbs melanin from surface layers, stimulates

In the present, the composition of the present invention can be used in a non-therapeutic cosmetic combination method, for example with other cosmetic composition.

"Non-therapeutic cosmetic combination method " comprise applying to the skin the cosmetic composition of the invention, and at least a second cosmetic composition as part of a specific non-therapeutic cosmetic treatment regimen, intended to provide the beneficial effect from the co-action of these cosmetic compositions. Administration of these cosmetic compositions in combination typically can be carried out over a defined time period (usually minutes, hours, days or weeks depending upon the combination selected).

"Non-therapeutic cosmetic combination method" can embrace application on the skin of these cosmetic compositions in a sequential manner, that is, wherein each cosmetic composition is applied at a different time, as well as applying the cosmetic composition on the skin in a substantially simultaneous manner. Sequential or substantially simultaneous administration of each cosmetic composition can be done by any appropriate route, including, but not limited to, topical routes.

Further advantages and/or characteristics will become apparent to the skilled person in the light of the examples given below by way of illustration.

## Brief descriptions of the figures

Figure 1 graphically represents the results of the study of the penetration of traxenamic acid encapsulated in a liposome in the skin, with "L" the portion not absorbed by the skin, "EP" the epidermis, and "D" the dermis.

Figure 2 represents a cross section of a skin sample with highlighted blood vessels. Stratum corneum (SC), epidermis (EP) and dermis (D) are noted. In this figure, red color corresponds to the blood vessels.

Figure 3 represents a cross section of a skin sample dyed with rhodamine and DAPI in which both the cell nuclei and the liposomes are highlighted. Stratum corneum (SC), epidermis (EP) and dermis (D) are noted. In this figure, the cell nuclei are colored in blue and the liposomes are colored in red.

Figure 4 represents a Fibroblast (F) with Liposomes (L) attached to it. In this figure, the fibroblasts are colored in blue and the liposomes are colored in red.

Figure 5 shows a diagram representing the methodology of the statistical analysis conducted on the data obtained in the study of example 5.

Figure 6 shows a comparison of skin samples without liposomes (Top-Left image (A), with the liposomes from Table 3 (Top-Right image (B)) and with the liposomes from Table 2, (Bottom image (C)), with the liposomes from Table 2 (C) circled. In this figure, the cell nuclei are colored in blue and the liposomes are colored in red.

Figure 7 represents a cross section of a skin sample with the endothelial cells highlighted and the liposomes highlighted and circled after a 16-hour application of table 2 liposomes. In this figure, endothelial cells are colored in green and liposomes are colored in red.

Figure 8 represents a cross section of a skin sample with the endothelial cells highlighted and the liposomes highlighted and circled after a 24-hour application of table 2 liposomes. In this figure, endothelial cells are colored in green and liposomes are colored in red.

Figure 9 shows a comparison of the same skin samples without and with endothelial cells highlighted (Left (A) and Right (B) images respectively). In this figure, endothelial cells are colored in green and liposomes are colored in red, the yellow color is the product of the red and green channels.

Figure 10 graphically represents the calibration curve for ethyl ascorbic acid.

Figure 11 graphically represents the skin penetration (i.e. the concentration) of the tested composition of the invention with the portion not absorbed by the skin (L), and with absorption in Stratum corneum (SC), in epidermis (EP) and in dermis (D) for composition B and A, both without tranexamic acid (TA).

## EXAMPLES

### EXAMPLE 1 : Example of compositions according to the present invention

The compositions described in this example are serum. They have been prepared by mixing the various ingredients described in Table 1 and table 1b below Table 1 : Cosmetic Composition

| CAS | Ingredients (INCI) | Quantity in % by weight of the total weight of the composition |
|---|---|---|
| 7732-18-5 | AQUA (WATER) | 67 to 71 |
| 504-63-2 | PROPANEDIOL | 4 to 6 |
| 93925-36-1 | C12-13 ALKYL LACTATE | 2 to 4 |
| 90-80-2 | GLUCONOLACTONE | 2 to 4 |
| 98-92-0 | NIACINAMIDE | 2 to 4 |
| 95912-86-0 | COCO-CAPRYLATE/CAPRATE | 2 to 4 |
| 31566-31-1 | GLYCERYL STEARATE | 1 to 3 |
| 56-81-5 | GLYCERIN | 1 to 3 |
| 629-96-9 | ARACHIDYL ALCOHOL | 1 to 3 |
| 136-77-6 | HEXYLRESORCINOL | 0.5 to 1.5 |
| 111-01-3 | SQUALANE | 0.5 to 1.5 |
| 661-19-8 | BEHENYL ALCOHOL | 0.5 to 1 |
| 5343-92-0 | PENTYLENE GLYCOL | 0.5 to 1 |
| 11138-66-2 | XANTHAN GUM | 0.5 to 1 |
| 1310-73-2 | SODIUM HYDROXIDE | 0.3 to 0.7 |
|  | ARACHIDYL GLUCOSIDE | 0.3 to 0.7 |

(continued)

| CAS | Ingredients (INCI) | Quantity in % by weight of the total weight of the composition |
|---|---|---|
| 6920-22-5 | 1,2-HEXANEDIOL | 0.3 to 0.7 |
| 38517-23-6 | SODIUM STEAROYL GLUTAMATE | 0.3 to 0.6 |
| 69-65-8 | MANNITOL | 0.1 to 0.4 |
| 8002-43-5 93685-90-6 | PHOSPHATIDYLCHOLINE | 0.1 to 0.4 |
| 9000-01-5 | ACACIA SENEGAL GUM | 0.1 to 0.3 |
| 8013-01-2 / 68876-77-7 | YEAST EXTRACT | 0.05 to 0.5 |
| 68797-35-3 | DIPOTASSIUM GLYCYRRHIZATE | 0.05 to 0.5 |
| 1197-18-8 | TRANEXAMIC ACID | 0.05 to 0.5 |
| 129499-78-1 | ASCORBYL GLUCOSIDE | 0.05 to 0.5 |
| 8001-25-0 | OLEA EUROPAEA (OLIVE) LEAF EXTRACT | 0.05 to 0.5 |
| 15454-75-8 | ZINC PCA | 0.05 to 0.5 |
| 659-40-5 | HEXAMIDINE DIISETHIONATE | 0.01 to 0.1 |
|  | PARFUM (FRAGRANCE) | 0.01 to 0.1 |
| 36653-82-4 | CETYL ALCOHOL | 0.01 to 0.1 |
| 34406-66-1 | POLYGLYCERYL-10 LAURATE | 0.01 to 0.1 |
| 7487-88-9 | MAGNESIUM SULFATE | 0.01 to 0.7 |
| 79-81-2 | RETINYL PALMITATE | 0.01 to 0.6 |
| 1117-86-8 | CAPRYLYL GLYCOL | 0.01 to 0.5 |
| 50-81-7 | ASCORBIC ACID | 0.001 to 0.05 |
| 10124-55-7 | MANGANESE SULFATE | 0.001 to 0.05 |
| 24634-61-5 | POTASSIUM SORBATE | 0.001 to 0.01 |
| 532-32-1 | SODIUM BENZOATE | 0.001 to 0.01 |
| 54549-25-6 / 58846-77-8 / 141464-42-8 / 68515-73-1 | DECYL GLUCOSIDE | 0.001 to 0.01 |
| 7647-14-5 | SODIUM CHLORIDE | 0.0001 to 0.005 |
| 1406-66-2 | TOCOPHEROL | 0.0001 to 0.0008 |
| 77-92-9 | CITRIC ACID | 0.0001 to 0.0008 |

Table 1b : Cosmetic Composition

| CAS | Ingredients (INCI) | Quantity in % by weight of the total weight of the composition |
|---|---|---|
| 7732-18-5 | AQUA (WATER) | 67 to 71 |
| 504-63-2 | PROPANEDIOL | 4 to 6 |
| 93925-36-1 | C12-13 ALKYL LACTATE | 2 to 4 |
| 90-80-2 | GLUCONOLACTONE | 2 to 4 |
| 98-92-0 | NIACINAMIDE | 2 to 4 |

(continued)

| CAS | Ingredients (INCI) | Quantity in % by weight of the total weight of the composition |
|---|---|---|
| 95912-86-0 | COCO-CAPRYLATE/CAPRATE | 2 to 4 |
| 31566-31-1 | GLYCERYL STEARATE | 1 to 3 |
| 56-81-5 | GLYCERIN | 1 to 3 |
| 629-96-9 | ARACHIDYL ALCOHOL | 1 to 3 |
| 136-77-6 | HEXYLRESORCINOL | 0.5 to 1.5 |
| 111-01-3 | SQUALANE | 0.5 to 1.5 |
| 661-19-8 | BEHENYL ALCOHOL | 0.5 to 1 |
| 5343-92-0 | PENTYLENE GLYCOL | 0.5 to 1 |
| 11138-66-2 | XANTHAN GUM | 0.5 to 1 |
| 1310-73-2 | SODIUM HYDROXIDE | 0.3 to 0.7 |
| | ARACHIDYL GLUCOSIDE | 0.3 to 0.7 |
| 6920-22-5 | 1,2-HEXANEDIOL | 0.3 to 0.7 |
| 38517-23-6 | SODIUM STEAROYL GLUTAMATE | 0.3 to 0.6 |
| 69-65-8 | MANNITOL | 0.1 to 0.4 |
| 8002-43-5 93685-90-6 | PHOSPHATIDYLCHOLINE | 0.1 to 0.4 |
| 9000-01-5 | ACACIA SENEGAL GUM | 0.1 to 0.3 |
| 8013-01-2 / 68876-77-7 | YEAST EXTRACT | 0.05 to 0.5 |
| 68797-35-3 | DIPOTASSIUM GLYCYRRHIZATE | 0.05 to 0.5 |
| 1197-18-8 | TRANEXAMIC ACID | 0.05 to 0.5 |
| 129499-78-1 | ASCORBYL GLUCOSIDE | 0.05 to 0.5 |
| 8001-25-0 | OLEA EUROPAEA (OLIVE) LEAF EXTRACT | 0.05 to 0.5 |
| 15454-75-8 | ZINC PCA | 0.05 to 0.5 |
| 659-40-5 | HEXAMIDINE DIISETHIONATE | 0.05 to 0.5 |
| | PARFUM (FRAGRANCE) | 0.05 to 0.5 |
| 36653-82-4 | CETYL ALCOHOL | 0.01 to 0.1 |
| 34406-66-1 | POLYGLYCERYL-10 LAURATE | 0.01 to 0.1 |
| 7487-88-9 | MAGNESIUM SULFATE | 0.01 to 0.7 |
| 79-81-2 | RETINYL PALMITATE | 0.01 to 0.6 |
| 1117-86-8 | CAPRYLYL GLYCOL | 0.01 to 0.5 |
| 50-81-7 | ASCORBIC ACID | 0.001 to 0.05 |
| 10124-55-7 | MANGANESE SULFATE | 0.001 to 0.05 |
| 24634-61-5 | POTASSIUM SORBATE | 0.008 to 0.01 |
| 532-32-1 | SODIUM BENZOATE | 0.008 to 0.01 |
| 54549-25-6 / 58846-77-8 / 141464-42-8 / 68515-73-1 | DECYL GLUCOSIDE | 0.008 to 0.01 |
| 7647-14-5 | SODIUM CHLORIDE | 0.0001 to 0.005 |

(continued)

| CAS | Ingredients (INCI) | Quantity in % by weight of the total weight of the composition |
|---|---|---|
| 1406-66-2 | TOCOPHEROL | 0.0001 to 0.0008 |
| 77-92-9 | CITRIC ACID | 0.0001 to 0.0008 |
| 68155-66-8, 54464-57-2 and 68155-67-9 | TETRAMETHYL ACETYLOCTAHYDRONAPHTHALENES | 0,001999 |

The composition of table 1 and 1b contains 92% naturally sourced ingredients.

This composition comprises tranexamic acid encapsulated in a liposome

**EXAMPLE 2** : **Study of the skin penetration of encapsulated tranexamic acid compared to non-encapsulated tranexamic acid**

The goal of this example is to compare the skin penetration capabilities of tranexamic acid encapsulated into two different liposomes compared with free tranexamic acid.

The material used were: 100 μL micropipettes, 50 mL Falcon tubes.

The reagents: Ninhydrin, Phenylacetaldehyde, PBS, Ethanol, a first composition comprising tranexamic acid encapsulated in a first liposome, as disclosed in table 2 and a second composition comprising tranexamic acid encapsulated in a second liposome as disclosed in table 3, and a Tranexamic acid solution (Free TA). The concentration of Tranexamic acid was identic in all the tested composition

The Equipment used were Franz diffusion cells.

Table 2 : composition comprising tranexamic acid encapsulated in a liposome (composition A)

| Ingredients (INCI) | Quantity in % by weight of the total weight of the composition |
|---|---|
| AQUA (WATER) | 74 to 78 |
| MANNITOL | 3 to 7 |
| PENTYLENE GLYCOL | 3 to 7 |
| GLYCERIN | 1 to 4 |
| DIPOTASSIUM GLYCYRRHIZATE | 1 to 3 |
| TRANEXAMIC ACID | 1 to 3 |
| POLYGLYCERYL-10 LAURATE | 0.5 to 2 |
| CETYL ALCOHOL | 0.5 to 2 |
| CITRIC ACID | 0.005 to 0.1 |
| PHOSPHATIDYLCHOLINE | 3 to 7 |

Table 3 : composition comprising tranexamic acid encapsulated in a liposome (composition B)

| Ingredients (INCI) | Quantity in % by weight of the total weight of the composition |
|---|---|
| Aqua | qsp 100 |
| Mannitol | 4.50 to 6.50 |
| Phosphatidylcholine | 3.20 to 5.20 |
| Glycerin | 2.90 to 3.90 |
| Tranexamic Acid | 2.00 to 3.00 |
| Cetyl Alcohol | 0.60 to 0.80 |
| Decyl Glucoside | 0.50 to 0.70 |
| Potassium Sorbate | 0.50 to 0.70 |

(continued)

| Ingredients (INCI) | *Quantity in % by weight of the total weight of the composition* |
| --- | --- |
| Sodium Benzoate | 0.50 to 0.70 |
| Niacinamide | 0.40 to 0.60 |
| Xanthan Gum | 0.10 to 0.30 |
| Sodium Chloride | 0.05 to 0.15 |

Tranexamic acid penetration profile was determined using Franz diffusion cells and dermatomized human skin samples. Skin samples were thawed and rehydrated on a 0,9% saline buffer for 20 minutes. After that time, skin discs were placed on top of the acceptor compartments of Franz diffusion cells, previously filled up to meniscus with the same saline buffer. The donor compartment was placed on top of the skin, and 100 $\mu$L of the tested product were added. The system was sealed up with Parafilm® M, to avoid evaporation, and the product was left to apply for 16 h.

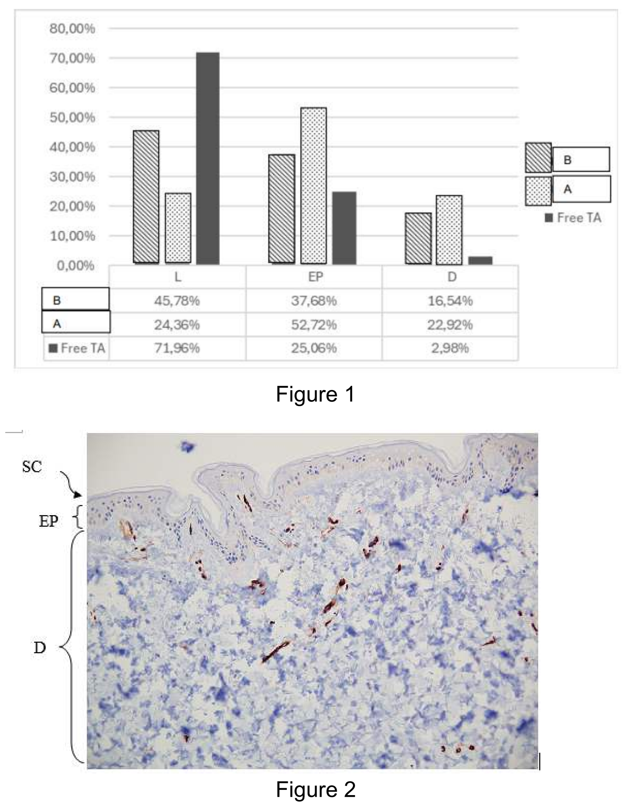

In Figure 1 the results of the penetration studies are summarized. An increased penetration for tranexamic acid encapsulated into a liposome both in the epidermis and dermis layers can be observed (around 1.4 times more), compared to free tranexamic acid. The graphic shows the percentage of tranexamic acid present in different parts of the skin. "L" represents the portion not absorbed by the skin, "EP" refers to the epidermis, and "D" denotes the dermis. As observed, the absorption of tranexamic acid into the skin is faster when encapsulated in liposomes and penetrate deeper than free tranexamic acid. This trend is consistent in both the epidermis and dermis. Specifically, in the dermis, 22.9% or 16.54% of tranexamic acid is present when encapsulated into a liposome, compared to 2.98% with free tranexamic acid. Results of dark spot treatment : Composition A : +++,Composition B : ++,Free TA : -

These results demonstrate that compositions according to the invention allow a more efficient and deeper penetration of active ingredients and also allow to treat dark spots and/or hyperpigmented spots in the skin.

## EXAMPLE 3: Study of the skin penetration of a liposome

The goal of the present example is to analyze the skin penetration capabilities of the delivery of the composition as defined in table 2 without tranexamic acid, with the help of a fluorescent dye and confocal microscopy.

The material used was : 100 $\mu$L micropipettes, 50 mL Falcon tubes.

The reagents: :1 PE CF (1,2-dioleoyl-sn-glycero-3-phosphoethanolamine-N-(carboxyfluorescein) (ammonium salt)), 4',6-diamidino-2-phenylindole (DAPI), composition as defined in table 2 without tranexamic acid, PFA (Paraformaldehyde) and Saline buffer.

The equipment used was Franz diffusion cells and Confocal microscope.

Fluorescent liposomes were synthesized including rhodamine-labelled phospholipids (18:1 PE CF) in the membrane of the liposomes. Product was characterized prior to the analysis, to assure that it was in accordance with the specifications regarding size and polydispersity index.

Frozen skin samples were hydrated and set to room temperature using a saline buffer. Once the skin was at working conditions, it was mounted on the Franz diffusion cells, and the wat er bath was set to 37°C, to simulate physiological conditions. 100 $\mu$L of fluorescent liposomes were added to receptor compartment of the Franz diffusion cells, and the compound was let to diffuse for 16 hours. After that time, the skin sample was rinsed off with ultrapure water and fixed with paraformaldehyde (PFA) at 4°C and sent to the pathology service at the University of Salamanca, for further analysis. A cross section of the skin dyed with Hematoxylin-Eosin (HE) can be observed in Figure 2.

These skin sections were dyed with DAPI, a fluorescent stain with specificity for cellular nuclei with an emission maximum at 461 nm and observed with a confocal microscope. This dye makes it possible to localize cells (blue, elongated, in the figure) throughout the tissue. The sample was also observed at the emission wavelength of rhodamine and contrasted with the HE dyed image of the sample, gaining information regarding the penetration capabilities of the liposomes.

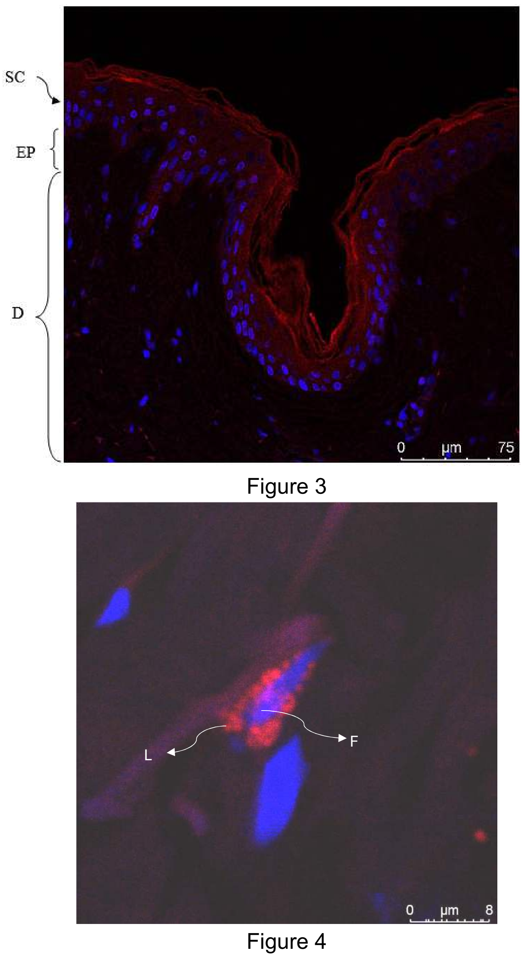

An image of a cross section of the skin dyed with rhodamine and DAPI is depicted in Figure 3. In this image, it can be observed how the biggest liposomal concentration (red elongated cells) is found on the epidermis. However, a significant number of liposomes can be also found at deeper levels, in the dermis and endothelial cells. Doing a more detailed analysis on these deeper tissues, we can observe how liposomes are specifically attached to fibroblasts, as seen in Figure 4.

These results thus demonstrate that liposomes allow an efficient penetration of active ingredients into the skin.

**Example 4: Effect of compositions according to the invention**

In this example, the efficacy of compositions mentioned in table 1 above was assessed. Study duration : 84 days, number of volunteers: 40 volunteers

Target population: Women aged 23 to 66 (mean age 49), phototype II to V, with all skin types on the face, with dull complexion and with pigmented spots on the face: 21 volunteers with melasmas with a majority epidermal component (recruitment with Wood's lamp), 10 volunteers with actinic lentigo spots (with all ages), 9 volunteers with acne-related pigmented (Post-inflammatory hyperpigmentation) PIH spots and post-acne marks.
5 volunteers with pigmented spots on the hands were also included in the study to monitor the evolution of the spots.

**Material and method:**

Depiwhite M Protective Cream SPF 50+ (a solar protective cream elaborated by the inventors) was applied in the morning to clean, dry skin all over the face (do not apply to hands).

The composition of table 1 (serum) was applied morning, directly after the application of Depiwhite M Protective Cream SPF 50+, and evening, on clean, dry skin, all over the face and hands (for 5 volunteers). 2 squeezes for 1 application. This composition of table 1 was allowed to dry for at least 5 minutes after application.

The application of DEPIWHITE.M - SPF50+ protective cream on the volunteers, before the application of the composition of table 1, aims at controlling the evolution of the spots on the skin of the volunteers during the study. In particular, the composition of DEPIWHITE.M - SPF50+ protective cream, which is a product of the inventors, is clearly and precisely known, and does not contain compounds that may physically and/or chemically interact with the composition of table 1, and thus that may distort the results of the study. The application of DEPIWHITE.M - SPF50+ protective cream on the skin of the volunteers thus allows to keep the spots in state that is convenient for the study, that is to say in a state allowing measurement/analysis of their evolution and determination of results, and at the same time allows to ensure that there will be no undesirable interaction between the protective cream and the serum of the present invention during the study. It also allows to correspond to a normal application of the composition of table 1 by common users, insofar as it is generally recommended to apply a solar protection on the skin every day, in combination with skincare and/or makeup products.

DEPIWHITE.M - SPF50+ protective cream was not applied on the hands of the volunteers, in order provide a test that demonstrates that the anti-stain effect is only due to DEPIWHITE SERUM (see example 5, point g).

**Results** :

1/ Tolerance at 28 days: The dermatologist assessed the product's tolerance at the start of the study and at D28 and no intolerance reactions was observed after 28 days of use.

Clinical scoring by the dermatologist at D0 and D28 of pigmented spots on photos. Clinical assessments were carried out by the dermatologist on photos taken on D0 and D28, using a structured scale from 0 to 5.

Assessment of spot intensity by clinical scoring : Significant mean decrease in spot intensity of 13.3% (-0.5 points). This increase was observed in 88% of volunteers.

Evaluation of spot size by clinical scoring: decrease in spot size of 9.3% (-0.3 points). This decrease was observed in 80% of volunteers.

Evaluation of number of spots by clinical scoring: Significant mean decrease in number of spots of 11.8% (-0.4 point). This reduction was observed in 80% of volunteers.

Evaluation of skin tone/ complexion uniformity by clinical scoring: Significant average increase in skin tone uniformity of +1.4 points (i.e. 107.5%). This increase was observed in 100% of volunteers. At D28, complexion was 2 times more even.

Assessment of complexion radiance by clinical scoring: Significant average increase in complexion radiance of +1.5 points (i.e. 115.7%). This increase was observed in 100% of volunteers. At D28, the complexion was 2 times more radiant.

Assessment of complexion luminosity by clinical scoring: Significant average increase in complexion luminosity of +15 points (i.e. 118.4%). This increase was observed in 100% of volunteers. At D28, the complexion was 2 times brighter.

According to the results of the clinical scoring, after 28 days of twice-daily application, it was concluded that the use of a composition described in table 1 above, according to the invention, significantly reduces the intensity and number of dark spots. It also significantly improves skin tone uniformity, radiance and luminosity.

The composition of the invention reduces the size of spots to follow at D56.

In the present, the acronym "D0" corresponds to the formulation "day 0", and refers to the first day of application of the tested product, i.e. of the composition according to the invention, and especially the composition according to example 1.

Also, the acronym "D28" corresponds to the formulation "day 28", and refers to a period of 28 days after the first day of application of the composition according to example 1.

**Example 5: Objective evaluation of the anti-stain effectiveness and of the tolerance of a composition according to the invention on a panel of volunteers.**

In this example, the anti-stain efficacy and the tolerance of an example of a composition according to example 1 (mentioned in table 1 above) was assessed using biometrological measurements, clinical scoring and self-assessment questionnaires. Study duration : 84 days. Number of volunteers: 40 volunteers

**Objectives** :

- Evaluation of the redness of spots and analysis of pigmented spots and melasma by analysis of 2D photos taken by the ColorFace® (the analyses have been carried out by QIMA Life Science)
- Evaluation of spots by analysis of 2D photos taken with the C-Cube® on the face and hands (for 5 volunteers)
- Evaluation of tolerance by dermatological control
- Evaluation of the intensity, size and number of spots, as well as the radiance, luminosity and evenness of the complexion, by means of a clinical scoring carried out by the dermatologist on the basis of the 2D photos taken by the ColorFace®.
- Evaluation of the volunteers' overall satisfaction using self-assessment questionnaires.

**Recruitment** :

Women, aged 23 to 66 (mean age : 49 ± 13), with skin types II to V, having all skin types with a dull complexion were recruited, in accordance with the inclusion and exclusion criteria defined in the protocol. In addition, 21 volunteers with melasma with a predominantly epidermal component (recruited using a Wood's lamp), 10 volunteers with actinic lentigines (of all ages), 9 volunteers with acne-related PIH pigment spots and post-acne marks, and 5 volunteers with actinic lentigo-type pigment spots on their hands, in order to monitor the evolution of the spots (these volunteers did not apply sunscreen during the study period), were recruited.

Inclusion criteria were : Female; Aged 20 years or older; Phototype II to V; All skin types; Dull complexion; 20 volunteers with melasma with a predominantly epidermal component (recruitment using a Wood's lamp); 10 volunteers with actinic lentigo spots (all ages); 10 volunteers with acne-related pigmentary PIH spots and post-acne marks; 5 volunteers with pigment spots on their hands to monitor the evolution of the spots (these volunteers did not apply sunscreen during the study period); No pigmentation-related treatment for at least 2 months. No sun exposure during the study period and in the month prior to the start of the study; Affiliated with a health insurance plan in accordance with French law on biomedical research; Informed and consenting, having undergone a general clinical examination confirming their suitability to participate in the study.

Non-inclusion criteria: Women who are pregnant or wish to become pregnant during the study or who are breastfeeding; Having planned UV exposure during the test period; Having undergone pigmentation-related treatment for at least two months; Having applied a cosmetic product to their face or hands on the morning of the appointment; Having applied products : scrubs, peels, self-tanning products, depigmenting products, or radiance treatments at a beauty salon, etc. to the face within the last month, or masks within the last 2 weeks; Having undergone cosmetic treatments at a dermatologist's office (peels, mesotherapy, injections, etc.) on the face within the last 6 months; Showing signs of recent and intense exposure to the sun or UV rays; Having a chronic or acute condition and/or undergoing topical or general treatment that may influence the results of the study, as determined by the investigator; Suffering from systemic diseases or any dermatosis that may interfere with the study, as determined by the investigator; Having a history of allergy or hypersensitivity to any of the components of the study product; Participating in another study or being in the exclusion period of another study; Having planned surgery during the study; Unable to be contacted by telephone in an emergency; Unable to comply with the requirements of the protocol; Deprived of liberty by a judicial or administrative decision or under a legal protection regime.

The volunteers included in the study did not take any concomitant treatments that could lead to a change in skin condition.

**Products:**

Description: DEPIWHITE SERUM - Intensive anti-spot concentrate Packaging : Pump bottle, Instructions for use : Apply morning and evening, on a clean and dry skin, all over the face and the hands (for 5 volunteers). 2 squeezes for 1 application. Description : DEPIWHITE.M - SPF50+ protective cream (Laboratoire ACM), Packaging : Tube, Instructions for use : Apply on a clean and dry skin, all over the face, in the morning. Apply after the serum (DEPIWHITE SERUM) and after allowing the serum to dry for at least 5 minutes.

The aim of the application of DEPIWHITE.M - SPF50+ protective cream on the volunteers, before the application of DEPIWHITE SERUM, is the same as in example 4.

**Protocol:**

The volunteers visited the laboratory 4 times during the study. All the measures and comments involved were recorded at each visit by the volunteers: during their visit at D0, at the start of the study, during their intermediate visits at D28 and D56, during their last visit at D84, at the end of the study.

At D0: Initial visit : Verification of inclusion and non-inclusion criteria, Verification of understanding of the study, Signature of informed consent, Dermatological checkup, Clinical scoring by the dermatologist of pigmentation spots (intensity of colour, size and number) and complexion (uniformity, radiance and luminosity) on the basis of the 2D ColorFace® photos. ColorFace® photos, C-Cube® photos and face and hand analysis (5 volunteers), Application of the product with a standardized quantity, Questionnaire returned after first application, Distribution of products and instructions.

From D0 to D27 in the evening: application of test products (composition) at home twice a day, morning and evening, on the face and on hands for part of the panel. Sun protection was also applied to the face in the morning (volunteers did not apply it to their hands). Two squeezes were used for each application.

At D7: Questionnaire sent with return completed, At D28: Intermediate visit : Dermatological check, Clinical scoring by the dermatologist of pigmentation spots (intensity of colour, size and number) and complexion (uniformity, radiance and luminosity) on the basis of the 2D ColorFace® photos, ColorFace® photos, C-Cube® photos + face and hand analysis (5 volunteers), Return of completed questionnaire on D28.

From D28 to D56 in the evening: application of the test products at home twice a day, morning and evening on the face and on hands for part of the panel. Sun protection was also applied to the face in the morning (the volunteers did not apply any to their hands). 2 squeezes were used for each application.

At D56: Intermediate visit : Dermatological check, Clinical scoring by the dermatologist of pigmentation spots (intensity of color, size and number) and complexion (uniformity, radiance and luminosity) on the basis of the 2D ColorFace® photos, ColorFace® photos, C-Cube® photos + face and hand analysis (5 volunteers), Return of completed questionnaire on D56.

From D56 to D83 in the evening: application of the test products at home twice a day, morning and evening on the face and on hands for part of the panel. Sun protection was also applied to the face in the morning (the volunteers did not apply any to their hands). 2 squeezes were performed for one application.

At D84: Final visit: Dermatological check, Clinical scoring by the dermatologist of pigment spots (intensity of color, size and number) and complexion (uniformity, radiance and luminosity) on the 2D ColorFace® photos, ColorFace® photos, C-Cube® photos + face and hand analysis (5 volunteers), Return of the completed D84 questionnaire.

In the present, the acronym "D56" corresponds to the formulation "day 56", and refers to a period of 56 days after the first day of application of the composition disclosed in above table 1.

The acronym "D84" corresponds to the formulation "day 84", and refers to a period of 84 days after the first day of application of the composition disclosed in above table 1.

**Methods** :

- Evaluation of tolerance and clinical scoring by dermatological control:
  The dermatologist evaluated skin condition and quality on D0, and then the tolerance of the product at D28, D56 and D84 by medical examination. Clinical scoring of pigmentation spots (coloration, size and number of spots) and complexion (radiance, uniformity and luminosity) was also carried out by the dermatologist on 2D photos taken by the ColorFace® on D0, D28, D56 and D84.

The clinical scales used all ranged from 0 to 5, with :

- for spots coloration/intensity : 0 for not intense and 5 for very intense;
- for spots size : 0 for small spots and 5 for large spots;
- for spots number : 0 for few spots and 5 for many spots;
- for complexion uniformity : 0 for non-uniform/uneven complexion and 5 for uniform complexion;
- for radiance (complexion) : 0 for dull complexion and 5 for radiant complexion;
- for luminosity (complexion) : 0 for non-luminous complexion and 5 for luminous complexion.

- 2D acquisition with ColorFace® :Redness and blotchiness were analyzed using photos taken with the ColorFace® acquisition system (QIMA Life Sciences).

Photos were taken of the face and on the 2 profiles on D0, D28, D56 and D84 with all the different filters.

The following colorimetric analyses were then carried out :

- Individual Typology Angle (ITA) measurement on pigmented area and surrounding skin on all 40 subjects;
- Delta E measurement between pigmented area and surrounding skin on the 40 subjects;
- Measurements on all the spots on the 40 subjects.
- IWA Newton (IWA°Newton) = Individual Whitening Angle : this is a measurement of skin whiteness, developed by Newtone Technologies. This parameter includes skin lightness (L*) and yellow component (b*) like ITA°, but also skin redness (a*), to consider both melanin and hemoglobin concentrations; ;
- Colorimetric measurements (L*a*b) on the entire face, on blemishes/spots/stains and on surrounding skin;
- dL* da* db*
- Analysis of hemoglobin and melanin intensity parameters on the entire face.

- Acquisition with C-Cube® :The C-Cube®, from Pixience, was used to capture images of the areas of interest (face and hands). These images enable the monitoring of a spot vs. the surrounding skin over a period of 84 days.

The C-Cube® measurements were carried out by a technician on the faces of 40 volunteers and on the hands of 5 volunteers. They were carried out on D0, D28, D56 and D84 on a stain and the surrounding skin.

The parameters analyzed were: ITA, L*a*b, delta E (ROI spot vs surrounding skin) and surface area occupied by the stain (for the 19 subjects without melasma).

[0146]    - Self-assessment questionnaires completed by volunteers

The assessment of the perceived effect (including comfort), efficacy, and cosmetic quality of the product (DEPIWIHITE SERUM) was carried out via an online questionnaire, completed after the first application and after 7, 28, 56, and 84 days of twice-daily use of the product at home.

The questionnaire was completed by volunteers using the Eval & GO software.

**Statistical methodology** :

- For instrumental measurements and clinical scoring :
  It was checked whether the data series follow or not a normal distribution using the Shapiro-Wilks W test:
- if $p > 5\%$, it was accepted the idea that the data series follow a normal distribution at a confidence level of 95%. The test used for statistical analysis will thus be the Student t-test.
- if $p < 5\%$, it was rejected the idea that the data series follows a normal distribution at a 95% confidence level. The test used for statistical analysis is thus the Wilcoxon signed-rank test

It was then determined the probability p of observing a difference before and after treatment that is at least as large as the one observed if the null hypothesis is true. The difference between the two probabilities is the effect size, which was expressed as a percentage of the total population.

- if $p < 5\%$, the null hypothesis was rejected. The alternative hypothesis H1 of a significant difference before and after product application was then accepted.
- if $p > 5\%$, the null hypothesis was accepted. The data did not reveal a significant difference before and after product application.

Significance level for Student t-test and Wilcoxon test: * Significant $p < 0.05$, ** Significant $p < 0.01$, *** Significant $p < 0.001$

For comparison with D0 of the delta E analysis at C-Cube® of the spot vs. the surrounding skin, the normality of the differences for each parameter was verified by the Shapiro-Wilk test (significance: $p < 0.05$):

- if the population followed a normal distribution ($p > 0.05$): the Student t-test was performed unilaterally for paired bilateral data in bilateral mode for comparison purposes :

  - if $p < 0.05$, there is a significant difference between the two measurement times;
  - if $p > 0.05$, there is no significant difference between the two measurement times.

- if the population did not follow a normal distribution ($p < 0.05$), the Wilcoxon test was performed unilaterally for paired bilateral data in bilateral mode for comparison purposes:

  - if $p < 0.05$, there is a significant difference between the two measurement times;
  - if $p > 0.05$, there is no significant difference between the two measurement times.

Significance level for the Student and Wilcoxon tests: Significant $p < 0.05$, ** Significant $p < 0.01$, *** Significant $p < 0.001$

- for image analysis :

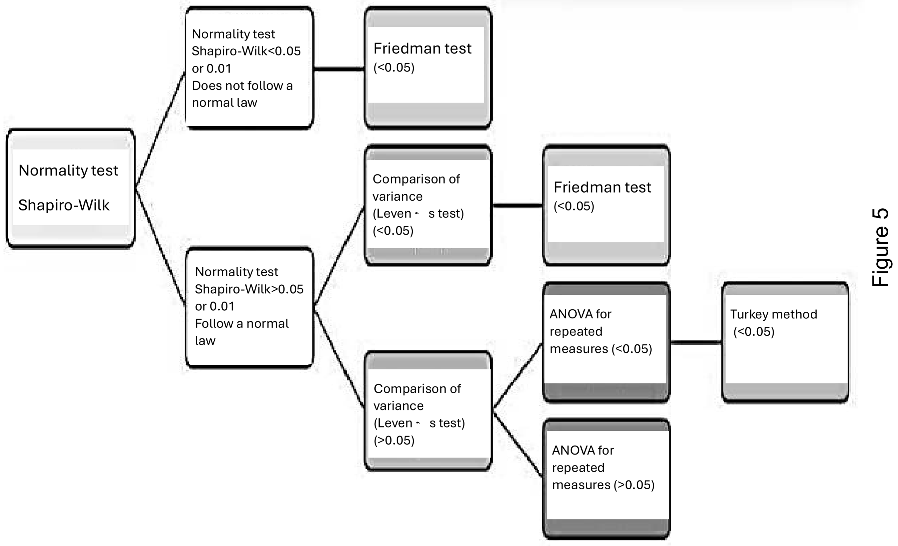

All statistical analyses were performed using the following methodology disclosed in the diagram of figure 5.
Variance comparison : the Levene test is used, the analysis of variance: ANOVA; the estimation method is based on maximum likelihood, the non-parametric test is Friedman test.

- Deviation from protocol :
  42 volunteers were initially recruited. The results were calculated on 40 volunteers at face level; on 5 volunteers at hand level ; and on 19 volunteers at face level for spot surface area.

The colorimetric analyses were adapted for the photos taken with Colorface: number and density measurements were removed, and measurements were added for the entire face: La*b*, ITA, IWA, homogeneity, and melanin intensity.

**Results and conclusion** :

No adverse events were encountered during the study.

a. Results of dermatological tolerance testing :

The dermatologist evaluated the tolerance of the product on D28, D56 and D84, with a twice-daily application, through a medical examination : no intolerance reactions were observed.
The skin acceptability of an example of a composition according to the invention was very good after 84 days of twice-daily use on volunteers with all skin types with dull complexions and epidermal melasma, actinic lentigo spots, acne-related PIH pigment spots, and post-acne marks.

b. Results of clinical scoring (n=40) :

b.1 Spots intensity :

i. general group :

Table 4 : results of clinical scoring regarding the parameter of spots intensity for the general group

| Spots intensity | D0 | D28 | Delta D28 - D0 | Variation D28 (%) | Statistics |
|---|---|---|---|---|---|
| Mean ± standard deviation | 3.4 ± 1.0 | 2.9 ± 1.0 | -0.5 ± 0.5 | -13.3 | Wilcoxon Test p-value = 0.0004*** s |
| Minimum | 2.0 | 1.5 | -1.5 | | |
| Maximum | 5.0 | 4.5 | 0.5 | | |
| Median | 3.0 | 2.5 | -0.5 | | |

| Spots intensity | D0 | D56 | Delta D56 - D0 | Variation D56 (%) | Statistics |
|---|---|---|---|---|---|
| Mean ± standard deviation | 3.4 ± 1.0 | 2.5 ± 1.0 | -0.9 ± 0.4 | -27.0 | Wilcoxon Test p-value < 0.0001*** s |
| Minimum | 2.0 | 1.0 | -2.0 | | |
| Maximum | 5.0 | 4.5 | 0.0 | | |
| Median | 3.0 | 2.0 | -1.0 | | |

| Spots intensity | D0 | D84 | Delta D84 - D0 | Variation D84 (%) | Statistics |
|---|---|---|---|---|---|
| Mean ± standard deviation | 3.4 ± 1.0 | 2.2 ± 1.0 | -1.2 ± 0.5 | -36.3 | Wilcoxon Test p-value < 0.0001*** s |
| Minimum | 2.0 | 0.5 | -2.5 | | |
| Maximum | 5.0 | 4.5 | -0.5 | | |
| Median | 3.0 | 2.0 | -1.0 | | |

Thus, for the general group of volunteers, 28 days after application of the product DEPIWHITE SERUM, it was observed a significant average reduction in the intensity of the spots of 0,5 points (i.e. -13,3%, Wilcoxon test, p-value = 0,0004) in 88% of respondents.

56 days after application of the product DEPIWHITE SERUM, it was observed a significant average reduction in the intensity of the spots of 0,9 points (i.e. -27,0%, Wilcoxon test, p-value < 0,0001) in 95% of respondents.

And 84 days after application of the product DEPIWHITE SERUM, it was observed a significant average reduction in the intensity of the spots of 1,2 points (i.e. -36,3%, Wilcoxon test, p-value < 0,0001) in 100% of respondents.

Therefore, as demonstrated above in table 4, an example of a composition according to the invention significantly reduces the intensity of the spots after 28 days, 56 days and 84 days of twice-daily application.

ii. melasma subgroup :

Table 5 : results of clinical scoring regarding the parameter of spots intensity for the melasma subgroup

| Spots intensity | D0 | D28 | Delta D28 - D0 | Variation D28 (%) | Statistics |
|---|---|---|---|---|---|
| Mean ± standard deviation | 3,7 ± 1,0 | 3,3 ± 1,1 | -0,4 ± 0,5 | -10,9 | Wilcoxon Test p-value = 0,040* s |
| Minimum | 2,0 | 1,5 | -1,5 | | |
| Maximum | 5,0 | 4,5 | 0,5 | | |
| Median | 4,0 | 3,5 | -0,5 | | |

(continued)

| Spots intensity | D0 | D56 | Delta D56 - D0 | Variation D56 (%) | Statistics |
|---|---|---|---|---|---|
| Mean ± standard deviation | 3,7 ± 1,0 | 2,8 ± 1,0 | -0,9 ± 0,4 | -25,0 | Wilcoxon Test p-value < 0,0001*** s |
| Minimum | 2,0 | 1,0 | -2,0 | | |
| Maximum | 5,0 | 4,5 | 0,0 | | |
| Median | 4,0 | 2,5 | -1,0 | | |

| Spots intensity | D0 | D84 | Delta D84 - D0 | Variation D84 (%) | Statistics |
|---|---|---|---|---|---|
| Mean ± standard deviation | 3,7 ± 1,0 | 2,5 ± 1,1 | -1,2 ± 0,5 | -32,1 | Wilcoxon Test p-value < 0,0001*** s |
| Minimum | 2,0 | 0,5 | -2,0 | | |
| Maximum | 5,0 | 4,5 | -0,5 | | |
| Median | 4,0 | 2,5 | -1,0 | | |

Thus, for the volunteers with melasma with a predominantly epidermal component, 28 days after application of the product DEPIWHITE SERUM, it was observed a significant average reduction in the intensity of melasma-type spots of 0,4 points (i.e. -10,9%, Wilcoxon test, p-value = 0,040) in 81% of respondents.

56 days after application of the product DEPIWHITE SERUM, it was observed a significant average reduction in the intensity of melasma-type spots of 0,9 points (i.e. -25,0%, Wilcoxon test, p-value < 0,000) in 95% of respondents.

And 84 days after application of the product DEPIWHITE SERUM, it was observed a significant average reduction in the intensity of melasma-type spots of 1,2 points (i.e. -32,1%, Wilcoxon test, p-value < 0,0001) in 100% of respondents.

Therefore, as demonstrated above in table 5, an example of a composition according to the invention significantly reduces the intensity of melasma-type spots after 28 days, 56 days and 84 days of twice-daily application.

iii. PIH/lentigos subgroup :

Table 6 : results of clinical scoring regarding the parameter of spots intensity for the PIH/lentigos subgroup

| Spots intensity | D0 | D28 | Delta D28 - D0 | Variation D28 (%) | Statistics |
|---|---|---|---|---|---|
| Mean ± standard deviation | 3,0 ± 0,8 | 2,5 ± 0,8 | -0,5 ± 0,3 | -16,7 | Wilcoxon Test p-value = 0,0001* s |
| Minimum | 2,0 | 1,5 | -1,5 | | |
| Maximum | 5,0 | 4,5 | 0,5 | | |
| Median | 3,0 | 2,5 | -0,5 | | |

| Spots intensity | D0 | D56 | Delta D56 - D0 | Variation D56 (%) | Statistics |
|---|---|---|---|---|---|
| Mean ± standard deviation | 3,0 ± 0,8 | 2,1 ± 0,8 | -0,9 ± 0,4 | -29,8 | Wilcoxon Test p-value < 0,0001*** s |
| Minimum | 2,0 | 1,0 | -2,0 | | |
| Maximum | 5,0 | 4,0 | 0,0 | | |
| Median | 3,0 | 2,0 | -1,0 | | |

(continued)

| Spots intensity | D0 | D84 | Delta D84 - D0 | Variation D84 (%) | Statistics |
|---|---|---|---|---|---|
| Mean ± standard deviation | 3,0 ± 0,8 | 1,7 ± 0,9 | -1,3 ± 0,5 | -42,1 | Wilcoxon Test p-value < 0,0001*** s |
| Minimum | 2,0 | 0,5 | -2,0 | | |
| Maximum | 5,0 | 4,0 | -0,5 | | |
| Median | 3,0 | 1,5 | -1,0 | | |

Thus, for the volunteers with PIH pigment spots and lentigo-type pigment spots, 28 days after application of the product DEPIWHITE SERUM, it was observed a significant average reduction in the intensity of PIH/lentigo-type spots of 0,4 points (i.e. -16,7%, Wilcoxon test, p-value = 0,0001) in 95% of respondents.

56 days after application of the product DEPIWHITE SERUM, it was observed a significant average reduction in the intensity of of PIH/lentigo-type spots of 0,9 points (i.e. -29,8%, Wilcoxon test, p-value < 0,0001) in 95% of respondents.

And 84 days after application of the product DEPIWHITE SERUM, it was observed a significant average reduction in the intensity of of PIH/lentigo-type spots of 1,3 points (i.e. -42,1%, Wilcoxon test, p-value < 0,0001) in 100% of respondents.

Therefore, as demonstrated above in table 6, an example of a composition according to the invention significantly reduces the intensity of PIH/lentigo-type spots after 28 days, 56 days and 84 days of twice-daily application.

b2. Spots size :

i. general group :

Table 7 : results of clinical scoring regarding the parameter of spots size for the general group

| Spots size | D0 | D56 | Delta D56 - D0 | Variation D56 (%) | Statistics |
|---|---|---|---|---|---|
| Mean ± standard deviation | 3,2 ± 1,1 | 2,4 ± 1,0 | -0,9 ± 0,4 | -26,4 | Wilcoxon Test p-value < 0,0001*** s |
| Minimum | 1,0 | 1,0 | -2,0 | | |
| Maximum | 5,0 | 5,0 | 0,5 | | |
| Median | 3,0 | 2,0 | -1,0 | | |
| | | | | | |
| Spots size | D0 | D84 | Delta D84 - D0 | Variation D84 (%) | Statistics |
| Mean ± standard deviation | 3,2 ± 1,1 | 2,0 ± 1,0 | -1,2 ± 0,6 | -36,8 | Wilcoxon Test p-value < 0,0001*** s |
| Minimum | 1,0 | 0,5 | -2,5 | | |
| Maximum | 5,0 | 4,5 | -0,5 | | |
| Median | 3,0 | 1,6 | -1,0 | | |

Thus, for the general group of volunteers, 56 days after application of the product DEPIWHITE SERUM, it was observed a significant average reduction in the size of the spots of 0,9 points (i.e. -26,4%, Wilcoxon test, p-value < 0.0001) in 93% of respondents.

And 84 days after application of the product DEPIWHITE SERUM, it was observed a significant average reduction in the size of the spots of 1,2 points (i.e. -36,8%, Wilcoxon test, p-value < 0.0001) in 95% of respondents.

Therefore, as demonstrated above in table 7, an example of a composition according to the invention reduce the size of the spots after 28 days of twice-daily application, and significantly reduced the size of the spots after 56 days and 84 days of twice-daily application.

ii. melasma subgroup :

Table 8 : results of clinical scoring regarding the parameter of spots size for the melasma subgroup

| Spots size | D0 | D56 | Delta D56 - D0 | Variation D56 (%) | Statistics |
|---|---|---|---|---|---|
| Mean ± standard deviation | 3,6 ± 1,2 | 2,7 ± 1,2 | -0,9 ± 0,4 | -24,0 | Wilcoxon Test p-value 0,0001*** s |
| Minimum | 1,2 | 1,0 | -2,0 | | |
| Maximum | 5,0 | 5,0 | 0,5 | | |
| Median | 4,0 | 2,5 | -1,0 | | |

| Spots size | D0 | D84 | Delta D84 - D0 | Variation D84 (%) | Statistics |
|---|---|---|---|---|---|
| Mean ± standard deviation | 3,6 ± 1,2 | 2,4 ± 1,1 | -1,2 ± 0,5 | -33,3 | Wilcoxon Test p-value < 0,0001*** s |
| Minimum | 1,2 | 0,5 | -2,5 | | |
| Maximum | 5,0 | 4,5 | 0,5 | | |
| Median | 4,0 | 2,0 | -1,5 | | |

Thus, for the volunteers with melasma with a predominantly epidermal component, 56 days after application of the product DEPIWHITE SERUM, it was observed a significant average reduction in the size of melasma-type spots of 0,9 points (i.e. - 24,0%, Wilcoxon test, p-value = 0,0001) in 95% of respondents.

And 84 days after application of the product DEPIWHITE SERUM, it was observed a significant average reduction in the size of melasma-type spots of 1,2 points (i.e. - 33,3%, Wilcoxon test, p-value < 0.0001) in 95% of respondents.

Therefore, as demonstrated above in table 8, an example of a composition according to the invention significantly reduces the size of the melasma-type spots after 56 days and 84 days of twice-daily application.

iii. PIH/lentigos subgroup :

Table 9 : results of clinical scoring regarding the parameter of spots size for the PIH/lentigos subgroup

| Spots size | D0 | D28 | Delta D28 - D0 | Variation D28 (%) | Statistics |
|---|---|---|---|---|---|
| Mean ± standard deviation | 2,8 ± 1,0 | 2,4 ± 0,8 | -0,4 ± 0,5 | -13,9 | Wilcoxon Test p-value = 0,011* s |
| Minimum | 1,0 | 1,5 | -1,5 | | |
| Maximum | 5,0 | 4,5 | 0,5 | | |
| Median | 3,0 | 2,5 | -0,5 | | |

| Spots size | D0 | D56 | Delta D56 - D0 | Variation D56 (%) | Statistics |
|---|---|---|---|---|---|
| Mean ± standard deviation | 2,8 ± 1,0 | 2,0 ± 0,7 | -0,8 ± 0,5 | -29,6 | Wilcoxon Test p-value 0,0001*** s |
| Minimum | 1,0 | 1,0 | -2,0 | | |
| Maximum | 5,0 | 4,0 | 0,5 | | |
| Median | 3,0 | 2,0 | -1,0 | | |

(continued)

| Spots size | D0 | D84 | Delta D84 - D0 | Variation D84 (%) | Statistics |
|---|---|---|---|---|---|
| Mean ± standard deviation | 2,8 ± 1,0 | 1,7 ± 0,8 | -1,2 ± 0,7 | -41,7 | Wilcoxon Test p-value < 0,0001*** s |
| Minimum | 1,2 | 0,5 | -2,5 | | |
| Maximum | 5,0 | 4,0 | 0,5 | | |
| Median | 3,0 | 1,5 | -1,0 | | |

Thus, for the volunteers with PIH pigment spots and lentigo-type pigment spots, 28 days after application of the product DEPIWHITE SERUM, it was observed a significant average reduction in the size of PIH/lentigo-type spots of 0,4 points (i.e. - 13,9%, Wilcoxon test, p-value = 0,011) in 84% of respondents.

56 days after application of the product DEPIWHITE SERUM, it was observed a significant average reduction in the size of PIH/lentigo-type spots of 0,8 points (i.e. - 29,6%, Wilcoxon test, p-value = 0,0001) in 89% of respondents.

And 84 days after application of the product DEPIWHITE SERUM, it was observed a significant average reduction in the size of PIH/lentigo-type spots of 1,2 points (i.e. - 41,7%, Wilcoxon test, p-value < 0,0001) in 95% of respondents.

Therefore, as demonstrated above in table 9, an example of a composition according to the invention significantly reduces the size of PIH/lentigo-type spots after 28 days, 56 days and 84 days of twice-daily application.

b3. Number of spots :

i. general group :

Table 10 : results of clinical scoring regarding the parameter of number of spots for the general group

| Number of spots | D0 | D28 | Delta D28 - D0 | Variation D28 (%) | Statistics |
|---|---|---|---|---|---|
| Mean ± standard deviation | 3,2 ± 1,0 | 2,8 ± 1,2 | -0,4 t 0,8 | -11,8 | Wilcoxon Test p-value = 0,0258* s |
| Minimum | 2,0 | 0,5 | -3,5 | | |
| Maximum | 5,0 | 5,5 | 1,5 | | |
| Median | 3,0 | 2,5 | -0,5 | | |

| Number of spots | D0 | D56 | Delta D56 - D0 | Variation D56 (%) | Statistics |
|---|---|---|---|---|---|
| Mean ± standard deviation | 3,2 ± 1,0 | 2,4 ± 1,1 | -0,8 ± 0,5 | -24,4 | Wilcoxon Test p-value 0,0003*** s |
| Minimum | 2,0 | 1,0 | -2,0 | | |
| Maximum | 5,0 | 5,0 | 1,0 | | |
| Median | 3,0 | 2,0 | -1,0 | | |

| Number of spots | D0 | D84 | Delta D84 - D0 | Variation D84 (%) | Statistics |
|---|---|---|---|---|---|
| Mean ± standard deviation | 3,2 ± 1,0 | 2,1 ± 1,0 | -1,1 ± 0,5 | -33,5 | Wilcoxon Test p-value < 0,0001*** s |
| Minimum | 2,0 | 0,5 | -2,5 | | |
| Maximum | 5,0 | 4,5 | 0,5 | | |
| Median | 3,0 | 2,0 | -1,0 | | |

Thus, for the general group of volunteers, 28 days after application of the product DEPIWHITE SERUM, it was observed a significant average reduction in the number of the spots of 0,4 points (i.e. -11,8%, Wilcoxon test, p-value = 0,0258) in 80% of respondents.

56 days after application of the product DEPIWHITE SERUM, it was observed a significant average reduction in the number of the spots of 0,8 points (i.e. -24,4%, Wilcoxon test, p-value = 0,0003) in 90% of respondents.

And 84 days after application of the product DEPIWHITE SERUM, it was observed a significant average reduction in the number of the spots of 1,1 points (i.e. -33,5%, Wilcoxon test, p-value < 0.0001) in 95% of respondents.

Therefore, as demonstrated above in table 10, an example of a composition according to the invention significantly reduces the number of the spots after 28 days, 56 days, and 84 days of twice-daily application.

ii. melasma subgroup :

Table 11 : results of clinical scoring regarding the parameter of number of spots for the melasma subgroup

| Number of spots | D0 | D28 | Delta D28 - D0 | Variation D28 (%) | Statistics |
|---|---|---|---|---|---|
| Mean ± standard deviation | 3,6 ± 1,0 | 3,2 ± 1,4 | -0,4 t 1,0 | | |
| Minimum | 2,0 | 0,5 | -3,5 | -11,3 | Wilcoxon Test p-value = 0,027* s |
| Maximum | 5,0 | 5,5 | 1,5 | | |
| Median | 4,0 | 2,5 | -0,5 | | |

| Number of spots | D0 | D56 | Delta D56 - D0 | Variation D56 (%) | Statistics |
|---|---|---|---|---|---|
| Mean ± standard deviation | 3,6 ± 1,0 | 2,9 ± 1,2 | -0,7 ± 0,6 | | |
| Minimum | 2,0 | 1,0 | -2,0 | -20,0 | Wilcoxon Test p-value 0,0001*** s |
| Maximum | 5,0 | 5,0 | 1,0 | | |
| Median | 4,0 | 3,0 | -1,0 | | |

| Number of spots | D0 | D84 | Delta D84 - D0 | Variation D84 (%) | Statistics |
|---|---|---|---|---|---|
| Mean ± standard deviation | 3,6 ± 1,0 | 2,5 ± 1,2 | -1,1 ± 0,6 | | |
| Minimum | 2,0 | 0,5 | -2,5 | -30,0 | Wilcoxon Test p-value < 0,0001*** s |
| Maximum | 5,0 | 4,5 | 0,5 | | |
| Median | 4,0 | 2,5 | -1,0 | | |

Thus, for the volunteers with melasma with a predominantly epidermal component, 28 days after application of the product DEPIWHITE SERUM, it was observed a significant average reduction in the number of melasma-type spots of 0,4 points (i.e. -11,3%, Wilcoxon test, p-value = 0,027) in 81% of respondents.

56 days after application of the product DEPIWHITE SERUM, it was observed a significant average reduction in the number of melasma-type spots of 0,7 points (i.e. -20,0%, Wilcoxon test, p-value = 0,0001) in 0% of respondents.

And 84 days after application of the product DEPIWHITE SERUM, it was observed a significant average reduction in the number of melasma-type spots of 1,1 points (i.e. -30,0%, Wilcoxon test, p-value < 0.0001) in 95% of respondents.

Therefore, as demonstrated above in table 11, an example of a composition according to the invention significantly reduces the number of melasma-type spots after 28 days, 56 days, and 84 days of twice-daily application.

iii. PIH/lentigos subgroup :

Table 12 : results of clinical scoring regarding the parameter of number of spots for the PIH/lentigos subgroup

| Number of spots | D0 | D28 | Delta D28 - D0 | Variation D28 (%) | Statistics |
|---|---|---|---|---|---|
| Mean ± standard deviation | 2,7 ± 0,8 | 2,4 ± 0,7 | -0,3 t 0,5 | -12,5 | Wilcoxon Test p-value = 0,003* s |
| Minimum | 2,0 | 1,5 | -1,5 | | |
| Maximum | 4,0 | 3,5 | 0,5 | | |
| Median | 3,0 | 2,5 | -0,5 | | |

| Number of spots | D0 | D56 | Delta D56 - D0 | Variation D56 (%) | Statistics |
|---|---|---|---|---|---|
| Mean ± standard deviation | 2,7 ± 0,8 | 1,9 ± 0,7 | -0,8 ± 0,4 | -30,8 | Wilcoxon Test p-value 0,0001*** s |
| Minimum | 2,0 | 1,0 | -2,0 | | |
| Maximum | 4,0 | 3,0 | 0,0 | | |
| Median | 3,0 | 2,0 | -1,0 | | |

| Number of spots | D0 | D84 | Delta D84 - D0 | Variation D84 (%) | Statistics |
|---|---|---|---|---|---|
| Mean ± standard deviation | 2,7 ± 0,8 | 1,7 ± 0,7 | -1,1 ± 0,4 | -38,5 | Wilcoxon Test p-value < 0,0001*** s |
| Minimum | 2,0 | 0,5 | -1,5 | | |
| Maximum | 4,0 | 3,0 | 0,0 | | |
| Median | 3,0 | 1,5 | -1,0 | | |

Thus, for the volunteers with PIH pigment spots and lentigo-type pigment spots, 28 days after application of the product DEPIWHITE SERUM, it was observed a significant average reduction in the number of PIH/lentigo-type spots of 0,3 points (i.e. -12,5%, Wilcoxon test, p-value = 0,0003) in 79% of respondents.

56 days after application of the product DEPIWHITE SERUM, it was observed a significant average reduction in the number of PIH/lentigo-type spots of 0,8 points (i.e. -30,8%, Wilcoxon test, p-value = 0,0001) in 89% of respondents.

And 84 days after application of the product DEPIWHITE SERUM, it was observed a significant average reduction in the number of PIH/lentigo-type spots of 1,1 points (i.e. -38,5%, Wilcoxon test, p-value < 0.0001) in 95% of respondents.

Therefore, as demonstrated above in table 12, an example of a composition according to the invention significantly reduces the number of PIH/lentigo-type spots after 28 days, 56 days, and 84 days of twice-daily application.

b4. Complexion uniformity :

i. general group :

Table 13 : results of clinical scoring regarding the parameter of complexion uniformity for the general group

| Complexion uniformity | D0 | D28 | Delta D28 - D0 | Variation D28 (%) | Statistics |
|---|---|---|---|---|---|
| Mean ± standard deviation | 1,3 ± 0,9 | 2,8 ± 1,0 | 1,4 ± 0,5 | 107,5 | Wilcoxon Test p-value < 0,0001*** s |
| Minimum | 0,0 | 0,5 | 0,5 | | |
| Maximum | 3,0 | 4,5 | 2,5 | | |
| Median | 1,0 | 2,5 | 1,5 | | |

| Complexion uniformity | D0 | D56 | Delta D56 - D0 | Variation D56 (%) | Statistics |
|---|---|---|---|---|---|
| Mean ± standard deviation | 1,3 ± 0,9 | 2,8 ± 0,9 | 1,5 ± 0,5 | 112,3 | Wilcoxon Test p-value < 0,0001*** s |
| Minimum | 0,0 | 1,0 | 0,5 | | |
| Maximum | 3,0 | 4,5 | 3,0 | | |
| Median | 1,0 | 3,0 | 1,5 | | |

| Complexion uniformity | D0 | D84 | Delta D84 - D0 | Variation D84 (%) | Statistics |
|---|---|---|---|---|---|
| Mean ± standard deviation | 1,3 ± 0,9 | 3,2 ± 0,9 | 1,8 ± 06 | 138,7 | Wilcoxon Test p-value < 0,0001*** s |
| Minimum | 0,0 | 1,5 | 0,0 | | |
| Maximum | 3,0 | 4,5 | 3,5 | | |
| Median | 1,0 | 3,0 | 2,0 | | |

Thus, for the general group of volunteers, 28 days after application of the product DEPIWHITE SERUM, it was observed a significant average increase in complexion uniformity of 1,4 points (i.e. 107,5%, Wilcoxon test, p-value < 0,0001) in 100% of respondents.

56 days after application of the product DEPIWHITE SERUM, it was observed a significant average increase in complexion uniformity of 1,5 points (i.e. 112,3%, Wilcoxon test, p-value < 0,0001) in 100% of respondents.

And 84 days after application of the product DEPIWHITE SERUM, it was observed a significant average increase in complexion uniformity of 1,8 points (i.e. 138,7%, Wilcoxon test, p-value < 0.0001) in 98% of respondents.

Therefore, as demonstrated above in table 13, an example of a composition according to the invention significantly improves complexion uniformity after 28 days, 56 days, and 84 days of twice-daily application.

ii. melasma subgroup :

Table 14 : results of clinical scoring regarding the parameter of complexion uniformity for the melasma subgroup

| Complexion uniformity | D0 | D28 | Delta D28 - D0 | Variation D28 (%) | Statistics |
|---|---|---|---|---|---|
| Mean ± standard deviation | 0,9 ± 0,8 | 2,3 ± 0,8 | 1,4 ± 0,7 | 150,0 | Wilcoxon Test p-value = 0,001*** s |
| Minimum | 0,0 | 0,5 | 0,5 | | |
| Maximum | 3,0 | 3,5 | 2,5 | | |
| Median | 1,0 | 2,5 | 1,5 | | |

(continued)

| Complexion uniformity | D0 | D56 | Delta D56 - D0 | Variation D56 (%) | Statistics |
|---|---|---|---|---|---|
| Mean ± standard deviation | 0,9 ± 0,8 | 2,4 ± 0,9 | 1,5 ± 0,6 | 168,4 | Wilcoxon Test p-value < 0,0001*** s |
| Minimum | 0,0 | 1,0 | 1,0 | | |
| Maximum | 3,0 | 4,5 | 3,0 | | |
| Median | 1,0 | 2,5 | 1,5 | | |

| Complexion uniformity | D0 | D84 | Delta D84 - D0 | Variation D84 (%) | Statistics |
|---|---|---|---|---|---|
| Mean ± standard deviation | 0,9 ± 0,8 | 2,8 ± 0,9 | 1,9 ± 0,6 | 205,3 | Wilcoxon Test p-value < 0,0001*** s |
| Minimum | 0,0 | 1,5 | 1,0 | | |
| Maximum | 3,0 | 4,5 | 3,5 | | |
| Median | 1,0 | 2,5 | 2,0 | | |

Thus, for the volunteers with melasma with a predominantly epidermal component, 28 days after application of the product DEPIWHITE SERUM, it was observed a significant average increase in complexion uniformity of 1,4 points (i.e. 150,0%, Wilcoxon test, p-value = 0,001) in 100% of respondents.

56 days after application of the product DEPIWHITE SERUM, it was observed a significant average increase in complexion uniformity of 1,5 points (i.e. 168,4%, Wilcoxon test, p-value < 0,0001) in 100% of respondents.

And 84 days after application of the product DEPIWHITE SERUM, it was observed a significant average increase in complexion uniformity of 1,9 points (i.e. 205,3%, Wilcoxon test, p-value < 0.0001) in 100% of respondents.

Therefore, as demonstrated above in table 14, an example of a composition according to the invention significantly improves complexion uniformity after 28 days, 56 days, and 84 days of twice-daily application, for users with melasma with a predominantly epidermal component.

iii. PIH/lentigos subgroup :

Table 15 : results of clinical scoring regarding the parameter of complexion uniformity for the PIH/lentigos subgroup

| Complexion uniformity | D0 | D28 | Delta D28 - D0 | Variation D28 (%) | Statistics |
|---|---|---|---|---|---|
| Mean ± standard deviation | 1,8 ± 0,9 | 3,3 ± 0,9 | 1,5 ± 0,3 | 83,8 | Wilcoxon Test p-value = 0,001*** s |
| Minimum | 0,0 | 1,5 | 0,5 | | |
| Maximum | 3,0 | 4,5 | 2,5 | | |
| Median | 2,0 | 3,5 | 1,5 | | |

| Complexion uniformity | D0 | D56 | Delta D56 - D0 | Variation D56 (%) | Statistics |
|---|---|---|---|---|---|
| Mean ± standard deviation | 1,8 ± 0,9 | 3,2 ± 0,8 | 1,4 ± 0,4 | 80,9 | Wilcoxon Test p-value < 0,0001*** s |
| Minimum | 0,0 | 1,5 | 0,5 | | |
| Maximum | 3,0 | 4,5 | 2,5 | | |
| Median | 2,0 | 3,5 | 1,5 | | |

(continued)

| Complexion uniformity | D0 | D84 | Delta D84 - D0 | Variation D84 (%) | Statistics |
|---|---|---|---|---|---|
| Mean ± standard deviation | 1,8 ± 0,9 | 3,6 ± 0,6 | 1,8 ± 06 | 101,5 | Wilcoxon Test p-value < |
| Minimum | 0,0 | 2,0 | 0,0 | | |
| Maximum | 3,0 | 4,5 | 3,5 | | 0,0001*** s |
| Median | 2,0 | 3,5 | 2,0 | | |

Thus, for the volunteers with PIH pigment spots and lentigo-type pigment spots, 28 days after application of the product DEPIWHITE SERUM, it was observed a significant average increase in complexion uniformity of 1,5 points (i.e. 83,8%, Wilcoxon test, p-value = 0,001) in 100% of respondents.

56 days after application of the product DEPIWHITE SERUM, it was observed a significant average increase in complexion uniformity of 1,4 points (i.e. 80,9%, Wilcoxon test, p-value < 0,0001) in 100% of respondents.

And 84 days after application of the product DEPIWHITE SERUM, it was observed a significant average increase in complexion uniformity of 1,8 points (i.e. 101,5%, Wilcoxon test, p-value < 0,0001) in 95% of respondents.

Therefore, as demonstrated above in table 15, an example of a composition according to the invention significantly improves complexion uniformity after 28 days, 56 days, and 84 days of twice-daily application, for users with PIH pigment spots and lentigo-type pigment spots.

b5. Radiance :

i. general group :

Table 16 : results of clinical scoring regarding the parameter of complexion radiance for the general group

| Radiance | D0 | D28 | Delta D28 - D0 | Variation D28 (%) | Statistics |
|---|---|---|---|---|---|
| Mean ± standard deviation | 1,3 ± 0,9 | 2,8 ± 0,9 | 1,5 ± 0,6 | 115,7 | Wilcoxon Test p-value < 0,0001*** s |
| Minimum | 0,0 | 1,5 | 0,5 | | |
| Maximum | 3,0 | 4,5 | 2,5 | | |
| Median | 1,0 | 2,5 | 1,5 | | |

| Radiance | D0 | D56 | Delta D56 - D0 | Variation D56 (%) | Statistics |
|---|---|---|---|---|---|
| Mean ± standard deviation | 1,3 ± 0,9 | 2,8 ± 0,8 | 1,5 ± 0,6 | 115,7 | Wilcoxon Test p-value < 0,0001*** s |
| Minimum | 0,0 | 1,0 | 0,0 | | |
| Maximum | 3,0 | 4,5 | 3,0 | | |
| Median | 1,0 | 2,8 | 1,5 | | |

| Radiance | D0 | D84 | Delta D84 - D0 | Variation D84 (%) | Statistics |
|---|---|---|---|---|---|
| Mean ± standard deviation | 1,3 ± 0,9 | 3,2 ± 0,9 | 1,9 ± 0,7 | 148,0 | Wilcoxon Test p-value < 0,0001*** s |
| Minimum | 0,0 | 1,5 | 0,5 | | |
| Maximum | 3,0 | 4,5 | 3,5 | | |
| Median | 1,0 | 3,3 | 2,0 | | |

Thus, for the general group of volunteers, 28 days after application of the product DEPIWHITE SERUM, it was observed a significant average increase in complexion radiance of 1,5 points (i.e. 115,7%, Wilcoxon test, p-value < 0,0001) in 100% of respondents.

56 days after application of the product DEPIWHITE SERUM, it was observed a significant average increase in complexion radiance of 1,5 points (i.e. 115,7%, Wilcoxon test, p-value < 0,0001) in 95% of respondents.

And 84 days after application of the product DEPIWHITE SERUM, it was observed a significant average increase in complexion radiance of 1,9 points (i.e. 148,0%, Wilcoxon test, p-value < 0.0001) in 100% of respondents.

Therefore, as demonstrated above in table 16, an example of a composition according to the invention significantly improves complexion radiance after 28 days, 56 days, and 84 days of twice-daily application.

ii. melasma subgroup :

Table 17 : results of clinical scoring regarding the parameter of complexion radiance for the melasma subgroup

| Radiance | D0 | D28 | Delta D28 - D0 | Variation D28 (%) | Statistics |
|---|---|---|---|---|---|
| Mean ± standard deviation | 0,8 ± 0,7 | 2,3 ± 0,7 | 1,5 ± 0,6 | | |
| Minimum | 0,0 | 1,5 | 0,5 | 179,4 | Wilcoxon Test p-value = 0,001*** s |
| Maximum | 3,0 | 3,5 | 2,5 | | |
| Median | 1,0 | 2,5 | 1,5 | | |

| Radiance | D0 | D56 | Delta D56 - D0 | Variation D56 (%) | Statistics |
|---|---|---|---|---|---|
| Mean ± standard deviation | 0,8 ± 0,7 | 2,4 ± 0,7 | 1,6 ± 0,8 | | |
| Minimum | 0,0 | 1,5 | 0,0 | 194,1 | Wilcoxon Test p-value 0,0001*** s |
| Maximum | 3,0 | 4,0 | 3,0 | | |
| Median | 1,0 | 2,5 | 1,5 | | |

| Radiance | D0 | D84 | Delta D84 - D0 | Variation D84 (%) | Statistics |
|---|---|---|---|---|---|
| Mean ± standard deviation | 0,8 ± 0,7 | 2,8 ± 0,8 | 2,0 ± 0,8 | | |
| Minimum | 0,0 | 1,5 | 0,5 | 244,1 | Wilcoxon Test p-value < 0,0001*** s |
| Maximum | 3,0 | 4,5 | 3,5 | | |
| Median | 1,0 | 2,5 | 2,0 | | |

Thus, for the volunteers with melasma with a predominantly epidermal component, 28 days after application of the product DEPIWHITE SERUM, it was observed a significant average increase in complexion radiance of 1,5 points (i.e. 179,4%, Wilcoxon test, p-value = 0,001) in 100% of respondents.

56 days after application of the product DEPIWHITE SERUM, it was observed a significant average increase in complexion radiance of 1,6 points (i.e. 194,1%, Wilcoxon test, p-value = 0,0001) in 90% of respondents.

And 84 days after application of the product DEPIWHITE SERUM, it was observed a significant average increase in complexion radiance of 2,0 points (i.e. 244,1%, Wilcoxon test, p-value < 0,0001) in 100% of respondents.

Therefore, as demonstrated above in table 17, an example of a composition according to the invention significantly improves complexion radiance after 28 days, 56 days, and 84 days of twice-daily application, for users with melasma with a predominantly epidermal component.

iii. PIH/lentigos subgroup :

Table 18 : results of clinical scoring regarding the parameter of complexion radiance for the PIH/lentigos subgroup

| Radiance | D0 | D28 | Delta D28 - D0 | Variation D28 (%) | Statistics |
|---|---|---|---|---|---|
| Mean ± standard deviation | 1,8 ± 0,9 | 3,3 ± 0,7 | 1,5 ± 0,6 | 83,8 | Wilcoxon Test p-value < 0,0001*** s |
| Minimum | 0,0 | 1,5 | 0,5 | | |
| Maximum | 2,0 | 4,5 | 2,5 | | |
| Median | 1,0 | 3,5 | 1,5 | | |

| Radiance | D0 | D56 | Delta D56 - D0 | Variation D56 (%) | Statistics |
|---|---|---|---|---|---|
| Mean + standard deviation | 1,8 ± 0,9 | 3,2 ± 0,8 | 1,4 ± 0,4 | 76,5 | Wilcoxon Test |
| Minimum | 0,0 | 1,5 | 0,5 | | p-value = 0,001*** s |
| Maximum | 2,0 | 4,0 | 2,0 | | |
| Median | 1,0 | 3,5 | 1,5 | | |

| Radiance | D0 | D84 | Delta D84 - D0 | Variation D84 (%) | Statistics |
|---|---|---|---|---|---|
| Mean ± standard deviation | 1,8 ± 0,9 | 3,6 ± 0,8 | 1,8 ± 06 | 100,0 | Wilcoxon Test p-value < 0,0001*** s |
| Minimum | 0,0 | 1,5 | 0,5 | | |
| Maximum | 2,0 | 4,5 | 3,0 | | |
| Median | 1,0 | 4,0 | 2,0 | | |

Thus, for the volunteers with PIH pigment spots and lentigo-type pigment spots, 28 days after application of the product DEPIWHITE SERUM, it was observed a significant average increase in complexion radiance of 1,5 points (i.e. 83,8%, Wilcoxon test, p-value =< 0,0001) in 100% of respondents.

56 days after application of the product DEPIWHITE SERUM, it was observed a significant average increase in complexion radiance of 1,4 points (i.e. 76,5%, Wilcoxon test, p-value = 0,001) in 90% of respondents.

And 84 days after application of the product DEPIWHITE SERUM, it was observed a significant average increase in complexion radiance of 1,8 points (i.e. 100,0%, Wilcoxon test, p-value < 0,0001) in 100% of respondents.

Therefore, as demonstrated above in table 18, an example of a composition according to the invention significantly improves complexion radiance after 28 days, 56 days, and 84 days of twice-daily application, for users with PIH pigment spots and lentigo-type pigment spots.

b6. Bright complexion :

i. general group :

Table 19 : results of clinical scoring regarding the parameter of complexion brightness for the general group

| Bright complexion | D0 | D28 | Delta D28 - D0 | Variation D28 (%) | Statistics |
|---|---|---|---|---|---|
| Mean ± standard deviation | 1,2 ± 0,9 | 2,7 ± 1,0 | 1,5 ± 0,6 | 118,4 | Wilcoxon Test p-value < 0,0001*** s |
| Minimum | 0,0 | 0,5 | 0,5 | | |
| Maximum | 3,0 | 4,5 | 2,5 | | |
| Median | 1,0 | 2,5 | 1,5 | | |

| Bright complexion | D0 | D56 | Delta D56 - D0 | Variation D56 (%) | Statistics |
|---|---|---|---|---|---|
| Mean ± standard deviation | 1,2 ± 0,9 | 2,7 ± 0,9 | 1,5 ± 0,6 | 118,4 | Wilcoxon Test p-value < 0,0001*** s |
| Minimum | 0,0 | 1,0 | 0,0 | | |
| Maximum | 3,0 | 4,5 | 3,0 | | |
| Median | 1,0 | 2,5 | 1,5 | | |

| Bright complexion | D0 | D84 | Delta D84 - D0 | Variation D84 (%) | Statistics |
|---|---|---|---|---|---|
| Mean ± standard deviation | 1,2 ± 0,9 | 3,2 ± 0,9 | 2,0 ± 0,7 | 160,2 | Wilcoxon Test p-value < 0,0001*** s |
| Minimum | 0,0 | 1,5 | 0,5 | | |
| Maximum | 3,0 | 4,5 | 3,5 | | |
| Median | 1,0 | 3,0 | 2,0 | | |

Thus, for the general group of volunteers, 28 days after application of the product DEPIWHITE SERUM, it was observed a significant average increase in complexion brightness of 1,5 points (i.e. 118,4%, Wilcoxon test, p-value < 0,0001) in 100% of respondents.

56 days after application of the product DEPIWHITE SERUM, it was observed a significant average increase in complexion brightness of 1,5 points (i.e. 118,4%, Wilcoxon test, p-value < 0,0001) in 98% of respondents.

And 84 days after application of the product DEPIWHITE SERUM, it was observed a significant average increase in complexion brightness of 2,0 points (i.e. 160,2%, Wilcoxon test, p-value < 0.0001) in 100% of respondents.

Therefore, as demonstrated above in table 19, an example of a composition according to the invention significantly improves complexion brightness after 28 days, 56 days, and 84 days of twice-daily application.

ii. melasma subgroup :

Table 20 : results of clinical scoring regarding the parameter of complexion brightness for the melasma subgroup

| Bright complexion | D0 | D28 | Delta D28 - D0 | Variation D28 (%) | Statistics |
|---|---|---|---|---|---|
| Mean ± standard deviation | 0,8 ± 0,7 | 2,1 ± 0,8 | 1,3 ± 0,6 | 161,8 | Wilcoxon Test p-value <= 0,001*** |
| Minimum | 0,0 | 0,5 | 0,5 | | |
| Maximum | 3,0 | 3,5 | 2,5 | | s |
| Median | 1,0 | 2,5 | 1,5 | | |

(continued)

| Bright complexion | D0 | D56 | Delta D56 - D0 | Variation D56 (%) | Statistics |
|---|---|---|---|---|---|
| Mean ± standard deviation | 0,8 ± 0,7 | 2,2 ± 0,8 | 1,4 ± 0,7 | 173,5 | Wilcoxon Test p-value <= 0,0001*** s |
| Minimum | 0,0 | 1,0 | 0,0 | | |
| Maximum | 3,0 | 4,0 | 3,0 | | |
| Median | 1,0 | 2,0 | 1,0 | | |

| Bright complexion | D0 | D84 | Delta D84 - D0 | Variation D84 (%) | Statistics |
|---|---|---|---|---|---|
| Mean ± standard deviation | 0,8 ± 0,7 | 2,8 ± 0,9 | 2,0 ± 0,7 | 241,2 | Wilcoxon Test p-value < 0,0001*** s |
| Minimum | 0,0 | 1,5 | 0,5 | | |
| Maximum | 3,0 | 4,5 | 3,5 | | |
| Median | 1,0 | 2,5 | 2,0 | | |

Thus, for the volunteers with melasma with a predominantly epidermal component, 28 days after application of the product DEPIWHITE SERUM, it was observed a significant average increase in complexion brightness of 1,3 points (i.e. 161,8%, Wilcoxon test, p-value <= 0,001) in 100% of respondents.

56 days after application of the product DEPIWHITE SERUM, it was observed a significant average increase in complexion brightness of 1,4 points (i.e. 173,5%, Wilcoxon test, p-value <= 0,0001) in 95% of respondents.

And 84 days after application of the product DEPIWHITE SERUM, it was observed a significant average increase in complexion brightness of 2,0 points (i.e. 241,2%, Wilcoxon test, p-value < 0,0001) in 100% of respondents.

Therefore, as demonstrated above in table 20, an example of a composition according to the invention significantly improves complexion brightness after 28 days, 56 days, and 84 days of twice-daily application, for users with melasma with a predominantly epidermal component.

iii. PIH/lentigos subgroup :

Table 21 : results of clinical scoring regarding the parameter of complexion brightness for the PIH/lentigos subgroup

| Bright complexion | D0 | D28 | Delta D28 - D0 | Variation D28 (%) | Statistics |
|---|---|---|---|---|---|
| Mean ± standard deviation | 1,7 ± 0,9 | 3,3 ± 0,8 | 1,6 ± 0,7 | 95,3 | Wilcoxon Test p-value = 0,0001*** s |
| Minimum | 0,0 | 1,5 | 0,5 | | |
| Maximum | 3,0 | 4,5 | 2,5 | | |
| Median | 2,0 | 3,5 | 1,5 | | |

| Bright complexion | D0 | D56 | Delta D56 - D0 | Variation D56 (%) | Statistics |
|---|---|---|---|---|---|
| Mean ± standard deviation | 1,7 ± 0,9 | 3,2 ± 0,8 | 1,5 ± 06 | 89,1 | Wilcoxon Test p-value <= 0,001*** s |
| Minimum | 0,0 | 1,5 | 0,5 | | |
| Maximum | 3,0 | 4,5 | 3,0 | | |
| Median | 2,0 | 3,0 | 1,5 | | |

(continued)

| Bright complexion | D0 | D84 | Delta D84 - D0 | Variation D84 (%) | Statistics |
|---|---|---|---|---|---|
| Mean $\pm$ standard deviation | 1,7 $\pm$ 0,9 | 3,7 $\pm$ 0,7 | 2,0 $\pm$ 0,8 | | |
| Minimum | 0,0 | 2,0 | 0,5 | 117,2 | Wilcoxon Test p-value < 0,0001*** s |
| Maximum | 3,0 | 4,5 | 3,5 | | |
| Median | 2,0 | 4,0 | 2,0 | | |

Thus, for the volunteers with PIH pigment spots and lentigo-type pigment spots, 28 days after application of the product DEPIWHITE SERUM, it was observed a significant average increase in complexion brightness of 1,6 points (i.e. 95,3%, Wilcoxon test, p-value = 0,0001) in 100% of respondents.

56 days after application of the product DEPIWHITE SERUM, it was observed a significant average increase in complexion brightness of 1,5 points (i.e. 89,1%, Wilcoxon test, p-value <= 0,0001) in 95% of respondents.

And 84 days after application of the product DEPIWHITE SERUM, it was observed a significant average increase in complexion brightness of 2,0 points (i.e. 117,2%, Wilcoxon test, p-value < 0,0001) in 100% of respondents.

Therefore, as demonstrated above in table 21, an example of a composition according to the invention significantly improves complexion brightness after 28 days, 56 days, and 84 days of twice-daily application, for users with PIH pigment spots and lentigo-type pigment spots.

Conclusion of clinical scoring :

Regarding the general group of volunteers, as demonstrated above, an example of a composition according to the invention significantly reduces the intensity and number of the spots after 28 days, 56 days and 84 days of twice-daily application.

In addition, this composition significantly improves complexion uniformity, complexion radiance and complexion brightness/luminosity after 28 days, 56 days and 84 days of twice-daily application.

Moreover, this composition significantly reduces the size of the spots after 56 days and 84 days of twice-daily application.

Regarding for the volunteers with melasma with a predominantly epidermal component, after 28 days, as demonstrated above, an example of a composition according to the invention significantly reduces the intensity and number of melasma-type spots. These reductions are confirmed after 56 and 84 days of twice-daily application.

Moreover, after 28 days, this composition significantly improves the evenness/uniformity, radiance and luminosity/brightness of the complexion. These improvements are confirmed after 56 and 84 days of twice-daily application. Furthermore, after 56 days, this composition significantly reduces the size of melasma-type spots. This result is confirmed after 84 days of twice-daily application. Regarding the volunteers with PIH pigment spots and lentigo-type pigment spots, after 28 days, as demonstrated above, an example of a composition according to the invention significantly reduces the intensity, size, and number of PIH/lentigo spots. These reductions are confirmed after 56 and 84 days of twice-daily application.

Moreover, after 28 days, this composition significantly improves the evenness/uniformity, radiance, and luminosity/brightness of the complexion. These improvements are confirmed after 56 and 84 days of twice-daily application.

c. Results of the C-Cube® (n=40) :

c1. ITA angle (ITA°)

i. general group :

Table 22 : results of C-Cube® regarding the parameter of ITA° for the general group at D28

| ITA° (spot) | D0 | D28 | Delta D28 - D0 | Variation D28 (%) | Statistics |
|---|---|---|---|---|---|
| Mean + | 23,69 $\pm$ | 27,74 $\pm$ | 4,05 $\pm$ 3,43 | 17,1 | Wilcoxon |

(continued)

| ITA° (spot) | D0 | D28 | Delta D28 - D0 | Variation D28 (%) | Statistics |
|---|---|---|---|---|---|
| standard deviation | 19,98 | 19,72 | | | Test p-value < 0,0001*** s |
| Minimum | -65,71 | -64,93 | -3,55 | | |
| Maximum | 44,83 | 49,97 | 13,71 | | |
| Median | 27,10 | 31,85 | 3,41 | | |
| | | | | | |
| ITA° (surrounding skin) | D0 | D28 | Delta D28 - D0 | Variation D28 (%) | Statistics |
| Mean $\pm$ standard deviation | 38,61 $\pm$ 20,00 | 41,85 $\pm$ 18,79 | 3,24 $\pm$ 2,96 | | Wilcoxon Test p-value < 0,0001*** s |
| Minimum | -64,26 | -56,86 | -2,62 | 8,4 | |
| Maximum | 57,60 | 59,32 | 12,43 | | |
| Median | 41,50 | 44,23 | 3,00 | | |

Table 23 : results of C-Cube® regarding the parameter of ITA° for the general group at D56

| ITA° (spot) | D0 | D56 | Delta D56 - D0 | Variation D56 (%) | Statistics |
|---|---|---|---|---|---|
| Mean $\pm$ standard deviation | 23,69 $\pm$ 19,98 | 29,86 $\pm$ 21,20 | 6,17 $\pm$ 5,07 | | Wilcoxon Test p-value < 0,0001*** s |
| Minimum | -65,71 | -73,89 | -8,18 | 26,1 | |
| Maximum | 44,83 | 56,25 | 16,87 | | |
| Median | 27,10 | 33,29 | 6,65 | | |
| | | | | | |
| ITA° (surrounding skin) | D0 | D56 | Delta D56 - D0 | Variation D56 (%) | Statistics |
| Mean $\pm$ standard deviation | 38,61 $\pm$ 20,00 | 43,93 $\pm$ 18,34 | 5,32 $\pm$ 4,72 | | Wilcoxon Test p-value < 0,0001*** s |
| Minimum | -64,26 | -53,53 | -5,71 | 13,8 | |
| Maximum | 57,60 | 59,54 | 22,21 | | |
| Median | 41,50 | 47,21 | 4,95 | | |

Table 24 : results of C-Cube® regarding the parameter of ITA° for the general group at D84

| ITA° (spot) | D0 | D84 | Delta D84 - D0 | Variation D84 (%) | Statistics |
|---|---|---|---|---|---|
| Mean $\pm$ standard deviation | 23,69 $\pm$ 19,98 | 30,89 $\pm$ 20,05 | 7,19 $\pm$ 4,34 | | Wilcoxon Test p-value < 0,0001*** s |
| Minimum | -65,71 | -65,90 | -0,19 | 30,4 | |
| Maximum | 44,83 | 53,16 | 18,59 | | |
| Median | 27,10 | 35,39 | 6,18 | | |
| | | | | | |
| ITA° (surrounding skin) | D0 | D84 | Delta D84 - D0 | Variation D84 (%) | Statistics |
| Mean $\pm$ standard deviation | 38,61 $\pm$ 20,00 | 43,90 $\pm$ 20,02 | 5,29 $\pm$ 3,44 | | Wilcoxon Test p-value < 0,0001*** s |
| Minimum | -64,26 | -63,63 | -2,90 | 13,7 | |
| Maximum | 57,60 | 61,54 | 13,71 | | |
| Median | 41,50 | 46,99 | 5,66 | | |
| Statistics spot vs surrounding skin | / | / | Student Test p-value = 0,0145* S | / | / |

Effectiveness on spots:

For the general group of volunteers, 28 days after application of the product DEPIWHITE SERUM, it was observed a significant average increase in ITA angle in the spots of 17,1% (Wilcoxon test, p-value < 0,0001), illustrating a significant lightening of the color of the spots in 90% of respondents.
56 days after application of the product DEPIWHITE SERUM, it was observed a significant average increase in ITA angle in the spots of 26,1% (Wilcoxon test, p-value < 0,0001), illustrating a significant lightening of the color of the spots in 90% of respondents.
And 84 days after application of the product DEPIWHITE SERUM, it was observed a significant average increase in ITA angle in the spots of 30,4% (Wilcoxon test, p-value < 0,0001), illustrating a significant lightening of the color of the spots in 95% of respondents.

Effectiveness on surrounding skin:

28 days after application of the product DEPIWHITE SERUM, it was observed a significant average increase in ITA angle in the surrounding skin of 8,4% (Wilcoxon test, p-value < 0,0001), illustrating a significant lightening of the color of the surrounding skin in 88% of respondents.
56 days after application of the product DEPIWHITE SERUM, it was observed a significant average increase in ITA angle in the surrounding skin of 13,8% (Wilcoxon test, p-value < 0,0001), illustrating a significant lightening of the color of the surrounding skin in 88% of respondents.
And 84 days after application of the product DEPIWHITE SERUM, it was observed a significant average increase in ITA angle in the surrounding skin of 13,7% (Wilcoxon test, p-value < 0,0001), illustrating a significant lightening of the color of the surrounding skin in 95% of respondents.

Inter-zone effectiveness:

84 days after application of the product DEPIWHITE SERUM, between the spots and the surrounding skin, it was observed a significant difference in ITA angle (Student test, p-value = 0,0145), with a significant greater effect on the spots.
Therefore, as demonstrated above in tables 22 to 24, an example of a composition according to the invention significantly lightens (in view of ITA angle parameter) the color of spots and surrounding skin after 28 days, 56 days and 84 days of twice-daily application. Moreover, it was observed a significant difference in the ITA angle between the spots and the surrounding skin, with significantly greater lightening of the spots than the surrounding skin after 84 days of twice-daily application.

ii. melasma subgroup (n=21) :

Table 25 : results of C-Cube® regarding the parameter of ITA° for the melasma subgroup at D28

| ITA° (spot) | D0 | D28 | Delta D28 - D0 | Variation D28 (%) | Statistics |
|---|---|---|---|---|---|
| Mean ± standard deviation | 17.09 ± 23.00 | 20.71 ± 24.03 | 3.62 ± 3.37 | 21.2 | Wilcoxon Test p-value < 0,0007*** s |
| Minimum | -65,71 | -64,93 | -3,55 | | |
| Maximum | 35.54 | 43.51 | 9.50 | | |
| Median | 24.54 | 27.75 | 3.37 | | |

| ITA° (surrounding skin) | D0 | D28 | Delta D28 - D0 | Variation D28 (%) | Statistics |
|---|---|---|---|---|---|
| Mean ± standard deviation | 31.06 ± 24.69 | 34.71 ± 23.07 | 3.65 ± 3.39 | 11.8 | Wilcoxon Test p-value 0,0003*** s |
| Minimum | -64,26 | -56,86 | -2,62 | | |
| Maximum | 51.14 | 54.16 | 12,43 | | |
| Median | 37.15 | 40.86 | 3.71 | | |

Table 26 : results of C-Cube® regarding the parameter of ITA° for the melasma subgroup at D56

| ITA° (spot) | D0 | D56 | Delta D56 - D0 | Variation D56 (%) | Statistics |
|---|---|---|---|---|---|
| Mean ± standard deviation | 17.09 ± 23.00 | 22.81 ± 26.26 | 5.72 ± 5.09 | 33.5 | Wilcoxon Test p-value 0,0004*** s |
| Minimum | -65,71 | -73,89 | -8,18 | | |
| Maximum | 35.54 | 46.62 | 13.22 | | |
| Median | 24.54 | 30.35 | 6,38 | | |

| ITA° (surrounding skin) | D0 | D56 | Delta D56 - D0 | Variation D56 (%) | Statistics |
|---|---|---|---|---|---|
| Mean ± standard deviation | 31.06 ± 24.69 | 37.30 ± 22.77 | 6.24 ± 5.25 | 20.1 | Wilcoxon Test p-value 0,0003*** s |
| Minimum | -64,26 | -53.53 | -5,71 | | |
| Maximum | 51.14 | 59.54 | 22.21 | | |
| Median | 37.15 | 43.65 | 5.17 | | |

Table 27 : results of C-Cube® regarding the parameter of ITA° for the melasma subgroup at D84

| ITA° (spot) | D0 | D84 | Delta D84 - D0 | Variation D84 (%) | Statistics |
|---|---|---|---|---|---|
| Mean ± standard deviation | 17.09 ± 23.00 | 24.59 ± 25.07 | 7.50 ± 4.78 | 43.9 | Wilcoxon Test p-value 0,0001*** s |
| Minimum | -65.71 | -65.90 | -2.90 | | |
| Maximum | 35.54 | 52.59 | 13.03 | | |
| Median | 24.54 | 32.02 | 6.13 | | |

| ITA° (surrounding skin) | D0 | D84 | Delta D84 - D0 | Variation D84 (%) | Statistics |
|---|---|---|---|---|---|
| Mean ± standard deviation | 31.06 ± 24.69 | 36.90 ± 25.30 | 5.84 ± 3.71 | 18.8 | Wilcoxon Test p-value 0,0001*** s |
| Minimum | -64.26 | -63.63 | -2.90 | | |
| Maximum | 51.14 | 57.21 | 13.03 | | |
| Median | 37.15 | 42.31 | 6.13 | | |

Thus, for the volunteers with melasma with a predominantly epidermal component, 28 days after application of the product DEPIWHITE SERUM, it was observed :

- a significant average increase in ITA angle in the spots of 21.2% (Wilcoxon test, p-value = 0,0007) in 90% of respondents; and
- a significant average increase in ITA angle in the surrounding skin of 11.8% (Wilcoxon test, p-value = 0,0003) in 86% of respondents.

Moreover, 56 days after application of the product DEPIWHITE SERUM, it was observed :

- a significant average increase in ITA angle in the spots of 33.5% (Wilcoxon test, p-value = 0,0004) in 90% of respondents; and
- a significant average increase in ITA angle in the surrounding skin of 20.1% (Wilcoxon test, p-value = 0,0003) in 95% of respondents.

Furthermore, 84 days after application of the product DEPIWHITE SERUM, it was observed :

- a significant average increase in ITA angle in the spots of 43.9% (Wilcoxon test, p-value = 0.0001) in 90% of respondents; and
- a significant average increase in ITA angle in the surrounding skin of 18.8% (Wilcoxon test, p-value = 0,0001) in 95% of respondents.

Therefore, as demonstrated above in tables 25 to 27, an example of the composition according to the invention significantly lightens (via the ITA angle parameter) the color of melasma-type spots and surrounding skin after 28 days, 56 days and 84 days of twice-daily application.

iii. PIH/lentigo subgroup (n=19) :

Table 28 : results of C-Cube® regarding the parameter of ITA° for the PIH/lentigo subgroup at D28

| ITA° (spot) | D0 | D28 | Delta D28 - D0 | Variation D28 (%) | Statistics |
|---|---|---|---|---|---|
| Mean ± standard deviation | 30.99 ± 9.25 | 35.52 ± 8.92 | 4.53 ± 3.53 | 14.6 | Student Test p-value 0,0000*** s |
| Minimum | 16.02 | 19.74 | -0.29 | | |
| Maximum | 44.83 | 49.97 | 13.71 | | |
| Median | 32.74 | 36.88 | 3.45 | | |

| ITA° (surrounding skin) | D0 | D28 | Delta D28 - D0 | Variation D28 (%) | Statistics |
|---|---|---|---|---|---|
| Mean ± standard deviation | 46.96 ± 7.03 | 49.74 ± 6.97 | 2.79 ± 2.40 | 5.9 | Student Test p-value 0,0001*** s |
| Minimum | 31.31 | 32.29 | -1.19 | | |
| Maximum | 57.60 | 59.32 | 8.39 | | |
| Median | 47.71 | 50.49 | 2.32 | | |

Table 29 : results of C-Cube® regarding the parameter of ITA° for the PIH/lentigo subgroup at D56

| ITA° (spot) | D0 | D56 | Delta D56 - D0 | Variation D56 (%) | Statistics |
|---|---|---|---|---|---|
| Mean ± standard deviation | 30.99 ± 9.25 | 37.66 ± 9.23 | 6.67 ± 5.15 | 21.5 | Student Test p-value 0,0000*** s |
| Minimum | 16.02 | 23.71 | -4.36 | | |
| Maximum | 44.83 | 56.25 | 16.87 | | |
| Median | 32.74 | 39.50 | 6.93 | | |

| ITA° (surrounding skin) | D0 | D56 | Delta D56 - D0 | Variation D56 (%) | Statistics |
|---|---|---|---|---|---|
| Mean ± standard deviation | 46.96 ± 7.03 | 51.26 ± 6.68 | 4.30 ± 3.95 | 9.2 | Student Test p-value 0,0002*** s |
| Minimum | 31.31 | 36.30 | -2.73 | | |
| Maximum | 57.60 | 59.36 | 9.78 | | |
| Median | 47.71 | 53.94 | 4.83 | | |

Table 30 : results of C-Cube® regarding the parameter of ITA° for the PIH/lentigo subgroup at D84

| ITA° (spot) | D0 | D84 | Delta D84 - D0 | Variation D84 (%) | Statistics |
|---|---|---|---|---|---|
| Mean ± standard deviation | 30.99 ± 9.25 | 37.84 ± 8.71 | 6.85 ± 3.88 | 22.1 | Student Test p-value 0,0000*** s |
| Minimum | 16.02 | 23.71 | 1.40 | | |
| Maximum | 44.83 | 56.25 | 15.27 | | |
| Median | 32.74 | 39.50 | 6.08 | | |

(continued)

| ITA° (surrounding skin) | D0 | D84 | Delta D84 - D0 | Variation D84 (%) | Statistics |
|---|---|---|---|---|---|
| Mean ± standard deviation | 46.96 ± 7.03 | 51.64 ± 6.10 | 4.68 ± 3.08 | 10.0 | Student Test p-value 0,0000*** s |
| Minimum | 31.31 | 39.36 | -0.18 | | |
| Maximum | 57.60 | 61.54 | 13.71 | | |
| Median | 47.71 | 52.09 | 4.83 | | |

Thus, for the volunteers with PIH pigment spots and lentigo-type pigment spots, 28 days after application of the product DEPIWHITE SERUM, it was observed :

- a significant average increase in ITA angle in the spots of 14.6% (Student test, p-value = 0,0000) in 89% of respondents; and
- a significant average increase in ITA angle in the surrounding skin of 5.9% (Student test, p-value = 0,0001) in 89% of respondents.

Moreover, 56 days after application of the product DEPIWHITE SERUM, it was observed :

- a significant average increase in ITA angle in the spots of 21.5% (Student test, p-value = 0,0000) in 89% of respondents; and
- a significant average increase in ITA angle in the surrounding skin of 9.2% (Student test, p-value = 0,0002) in 79% of respondents.

Furthermore, 84 days after application of the product DEPIWHITE SERUM, it was observed :

- a significant average increase in ITA angle in the spots of 22.1% (Student test, p-value = 0.0000) in 100% of respondents; and
- a significant average increase in ITA angle in the surrounding skin of 10.0% (Student test, p-value = 0,0000) in 95% of respondents.

Therefore, as demonstrated above in tables 28 to 30, an example of the composition according to the invention significantly lightens the color of PIH/lentigo-type spots and surrounding skin after 28 days, 56 days and 84 days of twice-daily application.

c2. L parameter (L*) - results vs D0

Table 31 : results of C-Cube® regarding the parameter L* for the general group at D28

| L* (spot) | D0 | D28 | Delta D28 - D0 | Variation D28 (%) | Statistics |
|---|---|---|---|---|---|
| Mean ± standard deviation | 59,22 ± 7,03 | 60,61 ± 7,33 | 1,39 ± 1,69 | 2,4 | Wilcoxon Test p-value < 0,0001*** s |
| Minimum | 28,17 | 27,59 | -2,38 | | |
| Maximum | 67,46 | 68,96 | 5,09 | | |
| Median | 60,46 | 62,35 | 1,20 | | |
| | | | | | |
| L* (surrounding skin) | D0 | D28 | Delta D28 - D0 | Variation D28 (%) | Statistics |
| Mean ± standard deviation | 64,36 ± 7,16 | 65,12 ± 6,64 | 0,76 ± 1,66 | 1,2 | Wilcoxon Test p-value = 0,0029** s |
| Minimum | 28,86 | 31,22 | -3,73 | | |
| Maximum | 72,49 | 71,57 | 4,50 | | |
| Median | 65,34 | 66,62 | 0,64 | | |

(continued)

| L* (surrounding skin) | D0 | D28 | Delta D28 - D0 | Variation D28 (%) | Statistics |
|---|---|---|---|---|---|
| Statistics spot vs surrounding skin | / | / | Student Test p-value = 0,0561 NS | / | / |

Table 32 : results of C-Cube® regarding the parameter L* for the general group at D56

| L* (spot) | D0 | D56 | Delta D56 - D0 | Variation D56 (%) | Statistics |
|---|---|---|---|---|---|
| Mean ± standard deviation | 59,22 ± 7,03 | 61,24 ± 7,62 | 2,02 ± 2,20 | 3,4 | Wilcoxon Test p-value < 0,0001*** s |
| Minimum | 28,17 | 25,34 | -2,83 | | |
| Maximum | 67,46 | 70,73 | 6,22 | | |
| Median | 60,46 | 62,68 | 2,06 | | |

| L* (surrounding skin) | D0 | D56 | Delta D56 - D0 | Variation D56 (%) | Statistics |
|---|---|---|---|---|---|
| Mean ± standard deviation | 64,36 ± 7,16 | 65,84 ± 6,52 | 1,48 ± 1,94 | 2,3 | Wilcoxon Test p-value < 0,0001*** s |
| Minimum | 28,86 | 31,50 | -2,67 | | |
| Maximum | 72,49 | 71,68 | 7,56 | | |
| Median | 65,34 | 67,26 | 1,44 | | |
| Statistics spot vs surrounding skin | / | / | Student Test p-value = 0,1643 NS | / | / |

Table 33 : results of C-Cube® regarding the parameter L* for the general group at D84

| L* (spot) | D0 | D84 | Delta D84 - D0 | Variation D84 (%) | Statistics |
|---|---|---|---|---|---|
| Mean ± standard deviation | 59,22 ± 7,03 | 61,87 ± 7,10 | 2,65 ± 1,80 | 4,5 | Wilcoxon Test p-value < 0,0001*** s |
| Minimum | 28,17 | 28,65 | -1,27 | | |
| Maximum | 67,46 | 68,58 | 5,91 | | |
| Median | 60,46 | 63,15 | 2,62 | | |

| L* (surrounding skin) | D0 | D84 | Delta D84 - D0 | Variation D84 (%) | Statistics |
|---|---|---|---|---|---|
| Mean ± standard deviation | 64,36 ± 7,16 | 65,99 ± 6,91 | 1,64 ± 1,49 | 2,5 | Wilcoxon Test p-value |
| Minimum | 28,86 | 29,81 | -1,64 | | |
| Maximum | 72,49 | 72,61 | 4,67 | | < 0,0001*** s |
| Median | 65,34 | 67,05 | 1,40 | | |
| Statistics spot vs surrounding skin | / | / | Student Test p-value = 0,0024* NS | / | / |

Effectiveness on spots:

28 days after application of the product DEPIWHITE SERUM, it was observed a significant average increase in L* parameter (corresponding to clarity) in the spots of 2,4% (Wilcoxon test, p-value < 0,0001), illustrating a significant increase in clarity of the spots in 83% of respondents.

56 days after application of the product DEPIWHITE SERUM, it was observed a significant average increase in L* parameter in the spots of 3,4% (Wilcoxon test, p-value < 0,0001), illustrating a significant increase in clarity of the spots in 78% of respondents.

And 84 days after application of the product DEPIWHITE SERUM, it was observed a significant average increase in L* parameter in the spots of 4,5% (Wilcoxon test, p-value < 0,0001), illustrating a significant increase in clarity of the spots in 93% of respondents.

Effectiveness on surrounding skin:

28 days after application of the product DEPIWHITE SERUM, it was observed a significant average increase in L* parameter in the surrounding skin of 1,2% (Wilcoxon test, p-value = 0,0029), illustrating a significant increase in clarity of the surrounding skin in 75% of respondents.

56 days after application of the product DEPIWHITE SERUM, it was observed a significant average increase in L* parameter in the surrounding skin of 2,3% (Wilcoxon test, p-value < 0,0001), illustrating a significant increase in clarity of the surrounding skin in 85% of respondents.

And 84 days after application of the product DEPIWHITE SERUM, it was observed a significant average increase in L* parameter in the surrounding skin of 2,5% (Wilcoxon test, p-value < 0,0001), illustrating a significant increase in clarity of the surrounding skin in 90% of respondents.

Inter-zone effectiveness:

28 days after application of the product DEPIWHITE SERUM, between the spots and the surrounding skin, it was observed a non-significant difference in L* parameter, with a greater effect on the spots (Student test, p-value = 0,0561).

56 days after application of the product DEPIWHITE SERUM, between the spots and the surrounding skin, it was observed a non-significant difference in L* parameter, despite a greater effect on the spots (Student test, p-value = 0,1643).

And 84 days after application of the product DEPIWHITE SERUM, between the spots and the surrounding skin, it was observed a significant difference in L* parameter, with a significant greater effect on the spots (Student test, p-value = 0,0024).

Therefore, as demonstrated above in tables 31 to 33, an example of a composition according to the invention significantly increases clarity (L* parameter) of spots and surrounding skin after 28 days, 56 days and 84 days of twice-daily application. Moreover, it was observed a greater effect on spots after 28 days and 56 days of twice-daily application. In addition, a significant difference was observed in the L* parameter between the spots and the surrounding skin, with a significantly greater effect on the spots after 84 days of twice-daily application.

c3. a parameter (a*) - results vs D0

Table 34 : results of C-Cube® regarding the parameter a* for the general group at D28

| a* (spot) | D0 | D28 | Delta D28 - D0 | Variation D28 (%) | Statistics |
|---|---|---|---|---|---|
| Mean ± standard deviation | 18,66 ± 2,70 | 17,85 ± 2,67 | -0,81 ± 1,87 | -4,3 | Student Test p-value = 0,0092** s |
| Minimum | 13,48 | 13,22 | -4,38 | | |
| Maximum | 24,32 | 25,19 | 4,07 | | |
| Median | 18,57 | 17,34 | -0,78 | | |

Table 35 : results of C-Cube® regarding the parameter a* for the general group at D56

| a* (spot) | D0 | D56 | Delta D56 - D0 | Variation D56 (%) | Statistics |
|---|---|---|---|---|---|
| Mean ± standard deviation | 18,66 ± 2,70 | 17,48 ± 2,81 | -1,18 ± 2,50 | -6,3 | Student Test p-value = 0,0050** |
| Minimum | 13,48 | 9,45 | -6,35 | | |
| Maximum | 25,32 | 22,28 | 4,30 | | s |
| Median | 18,57 | 17,17 | -0,70 | | |

Table 36 : results of C-Cube® regarding the parameter a* for the general group at D84

| a* (spot) | D0 | D84 | Delta D84 - D0 | Variation D84 (%) | Statistics |
|---|---|---|---|---|---|
| Mean ± standard deviation | 18,66 ± 2,70 | 16,64 ± 2,22 | -2,02 ± 2,16 | -10,8 | Student Test p-value < 0,0001*** s |
| Minimum | 13,48 | 12,62 | -7,74 | | |
| Maximum | 25,32 | 22,14 | 2,06 | | |
| Median | 18,57 | 16,73 | -1,67 | | |

| a* (surrounding skin) | D0 | D84 | Delta D84 - D0 | Variation D84 (%) | Statistics |
|---|---|---|---|---|---|
| Mean ± standard deviation | 15,63 ± 2,65 | 14,67 ± 2,43 | -0,96 ± 1,82 | -6,1 | Wilcoxon Test p-value = 0,0017** s |
| Minimum | 11,05 | 11,12 | -5,05 | | |
| Maximum | 24,65 | 22,10 | 3,71 | | |
| Median | 15,88 | 14,36 | -0,69 | | |
| Statistics spot vs surrounding skin | / | / | Student Test p-value = 0,0097** s | / | / |

Effectiveness on spots:

28 days after application of the product DEPIWHITE SERUM, it was observed a significant average decrease in a* parameter (corresponding to redness) in the spots of 4,3% (Student test, p-value = 0,0092), illustrating a significant decrease in redness of the spots in 73% of respondents.

56 days after application of the product DEPIWHITE SERUM, it was observed a significant average decrease in a* parameter in the spots of 6,3% (Student test, p-value = 0,0050), illustrating a significant decrease in redness of the spots in 75% of respondents.

And 84 days after application of the product DEPIWHITE SERUM, it was observed a significant average decrease in a* parameter in the spots of 10,8% (Student test, p-value < 0,0001), illustrating a significant decrease in redness of the spots in 88% of respondents.

Effectiveness on surrounding skin:

84 days after application of the product DEPIWHITE SERUM, it was observed a significant average decrease in a* parameter in the surrounding skin of 6,1% (Wilcoxon test, p-value = 0,0017), illustrating a significant decrease in redness of the surrounding skin in 70% of respondents.

Inter-zone effectiveness:

84 days after application of the product DEPIWHITE SERUM, between the spots and the surrounding skin, it was observed a significant difference in a* parameter, with a significant greater effect on the spots (Student test, p-value = 0,0097).

Therefore, as demonstrated above in tables 34 to 36, an example of a composition according to the invention significantly decreases redness (a* parameter) of spots after 28 days, 56 days and 84 days of twice-daily application. Moreover, this composition reduces redness on the surrounding skin after 56 days; and significantly reduces redness on the surrounding skin after 84 days of twice-daily application.

In addition, a significant difference was observed in redness between the spots and the surrounding skin, with a greater reduction in redness of the spots than of the surrounding skin after 28 days and 84 days of twice-daily application. Furthermore, a slightly greater effect on the reduction in redness of the spots than of the surrounding skin after 56 days of twice-daily application was observed.

c4. b* parameter - results vs D0

Table 37 : results of C-Cube® regarding the parameter b* for the general group at D28

| b* (spot) | D0 | D28 | Delta D28 - D0 | Variation D28 (%) | Statistics |
|---|---|---|---|---|---|
| Mean ± standard deviation | 20,29 ± 3,06 | 19,62 ± 2,89 | -0,67 ± 1,49 | -3,3 | Wilcoxon Test p-value = 0,0065** s |
| Minimum | 9,66 | 10,31 | -3,01 | | |
| Maximum | 25,69 | 24,70 | 3,11 | | |
| Median | 20,81 | 19,70 | -0,94 | | |

| b* (surrounding skin) | D0 | D28 | Delta D28 - D0 | Variation D28 (%) | Statistics |
|---|---|---|---|---|---|
| Mean ± standard deviation | 19,98 ± 2,60 | 16,03 ± 2,40 | -0,95 ± 1,26 | -5,6 | Student Test p-value < 0,0001*** s |
| Minimum | 9,97 | 11,38 | -3,26 | | |
| Maximum | 21,59 | 20,00 | 2,19 | | |
| Median | 17,25 | 16,14 | -0,75 | | |

Table 38 : results of C-Cube® regarding the parameter b* for the general group at D56

| b* (spot) | D0 | D56 | Delta D56 - D0 | Variation D56 (%) | Statistics |
|---|---|---|---|---|---|
| Mean ± standard deviation | 20,29 ± 3,06 | 18,87 ± 3,44 | -1,42 ± 2,05 | -7,0 | Wilcoxon Test p-value 0,0002*** s |
| Minimum | 9,66 | 6,39 | -6,14 | | |
| Maximum | 25,69 | 25,57 | 4,09 | | |
| Median | 20,81 | 19,22 | -1,54 | | |

| b* (surrounding skin) | D0 | D56 | Delta D56 - D0 | Variation D56 (%) | Statistics |
|---|---|---|---|---|---|
| Mean ± standard deviation | 16,98 ± 2,60 | 15,67 ± 2,28 | -1,32 ± 1,86 | -7,8 | Student Test p-value < 0,0001*** s |
| Minimum | 9,97 | 11,82 | -4,68 | | |
| Maximum | 21,59 | 20,37 | 3,68 | | |
| Median | 17,25 | 15,26 | -1,15 | | |

Table 39 : results of C-Cube® regarding the parameter b* for the general group at D84

| b* (spot) | D0 | D84 | Delta D84 - D0 | Variation D84 (%) | Statistics |
|---|---|---|---|---|---|
| Mean ± standard deviation | 20,29 ± 3,06 | 19,17 ± 3,29 | -1,12 ± 1,57 | -5,5 | Wilcoxon Test p-value 0,0002*** s |
| Minimum | 9,66 | 9,33 | -4,99 | | |
| Maximum | 25,69 | 24,57 | 1,73 | | |
| Median | 20,81 | 19,57 | -1,02 | | |

| b* (surrounding skin) | D0 | D84 | Delta D84 - D0 | Variation D84 (%) | Statistics |
|---|---|---|---|---|---|
| Mean ± standard deviation | 16,98 ± 2,60 | 15,60 ± 2,36 | -1,38 ± 1,34 | -8,1 | Student Test p-value < 0,0001*** s |
| Minimum | 9,97 | 9,86 | -4,08 | | |
| Maximum | 21,59 | 19,88 | 1,57 | | |
| Median | 17,25 | 15,77 | -1,22 | | |

Effectiveness on spots:

28 days after application of the product DEPIWHITE SERUM, it was observed a significant average decrease in b* parameter (corresponding to yellow hues) in the spots of 3,3% (Wilcoxon test, p-value = 0,0065), illustrating a significant decrease in the yellow hues of the spots in 73% of respondents.

56 days after application of the product DEPIWHITE SERUM, it was observed a significant average decrease in b* parameter in the spots of 7,0% (Wilcoxon test, p-value = 0,0002), illustrating a significant decrease in the yellow hues of the spots in 83% of respondents.

And 84 days after application of the product DEPIWHITE SERUM, it was observed a significant average decrease in b* parameter in the spots of 5,5% (Wilcoxon test, p-value = 0,0002), illustrating a significant decrease in the yellow hues of the spots in 75% of respondents.

Effectiveness on surrounding skin:

28 days after application of the product DEPIWHITE SERUM, it was observed a significant average decrease in b* parameter in the surrounding skin of 5,6% (Student test, p-value < 0,0001), illustrating a significant decrease in the yellow hues of the surrounding skin in 80% of respondents.

56 days after application of the product DEPIWHITE SERUM, it was observed a significant average decrease in b* parameter in the surrounding skin of 7,8% (Student test, p-value < 0,0001), illustrating a significant decrease in the yellow hues of the surrounding skin in 75% of respondents.

And 84 days after application of the product DEPIWHITE SERUM, it was observed a significant average decrease in b* parameter in the surrounding skin of 8,1% (Student test, p-value < 0,0001), illustrating a significant decrease in the yellow hues of the surrounding skin in 93% of respondents.

Therefore, as demonstrated above in tables 37 to 39, an example of a composition according to the invention significantly decreases the yellow hues (B* parameter) of spots and surrounding skin after 28 days, 56 days and 84 days of twice-daily application.

c5. surface area of spots on the face (n = 19 : 9 PIH acne spot + 10 actinic lentigo)

Table 40 : results of C-Cube® regarding the parameter of surface area of spots at D28

| Surface area in mm$^2$ | D0 | D28 | Delta D28 - D0 | Variation D28 (%) | Statistics |
|---|---|---|---|---|---|
| Mean ± standard deviation | 19,00 ± 28,32 | 17,57 ± 26,87 | -1,43 ± 2,45 | -7,5 | Wilcoxon Test p-value = 0,0016** s |
| Minimum | 0,90 | 0,80 | -7,60 | | |
| Maximum | 89,90 | 82,60 | 0,50 | | |
| Median | 2,60 | 2,40 | -0,40 | | |

Table 41 : results of C-Cube® regarding the parameter of surface area of spots at D56

| Surface area in mm$^2$ | D0 | D56 | Delta D56 - D0 | Variation D56 (%) | Statistics |
|---|---|---|---|---|---|
| Mean ± standard deviation | 19,00 ± 28,32 | 16,15 ± 25,48 | -2,85 ± 4,43 | -15,0 | Wilcoxon Test p-value = 0,0002*** s |
| Minimum | 0,90 | 0,70 | -17,00 | | |
| Maximum | 89,90 | 82,00 | 0,00 | | |
| Median | 2,60 | 1,60 | -0,90 | | |

Table 42 : results of C-Cube® regarding the parameter of surface area of spots at D84

| Surface area in mm$^2$ | D0 | D84 | Delta D84 - D0 | Variation D84 (%) | Statistics |
|---|---|---|---|---|---|
| Mean ± standard deviation | 19,00 ± 28,32 | 16,25 ± 25,12 | -2,75 ± 3,74 | | Wilcoxon Test p-value 0,0003*** s |
| Minimum | 0,90 | 0,30 | -12,40 | -14,5 | |
| Maximum | 89,90 | 80,50 | 0,60 | | |
| Median | 2,60 | 1,50 | -1,00 | | |

28 days after application of the product DEPIWHITE SERUM, it was observed a significant average decrease of the surface area of the spots of 7,5% (i.e., -1,43 mm$^2$) (Wilcoxon test, p-value = 0,0016) in 84% of respondents.

56 days after application of the product DEPIWHITE SERUM, it was observed a significant average decrease of the surface area of the spots of 15,0% (i.e., -2,85 mm$^2$) (Wilcoxon test, p-value = 0,0002) in 95% of respondents.

And 84 days after application of the product DEPIWHITE SERUM, it was observed a significant average decrease of the surface area of the spots of 14,5% (i.e., -2,75 mm$^2$) (Wilcoxon test, p-value = 0,0003) in 95% of respondents.

Therefore, as demonstrated above in tables 40 to 42, an example of a composition according to the invention significantly decreases the surface area/size of spots after 28 days, 56 days and 84 days of twice-daily application.

d. Variation in contrast between spots and surrounding skin (n=40) :

d.1. L parameter :

Table 45 : results regarding the contrast spots/surrounding skin, for the parameter L at D84

| L Spot vs. surrounding skin difference | D0 | D84 | Contrast variation Delta D84 - D0 | Variation D84 (%) | Statistics |
|---|---|---|---|---|---|
| Mean ± standard deviation | 5,14 ± 3,31 | 4,12 ± 3,32 | -1,02 ± 1,99 | | Wilcoxon Test p-value = 0,002** s |
| Minimum | 0,69 | -1,63 | -5,66 | -19,8 | |
| Maximum | 18,96 | 18,29 | 3,62 | | |
| Median | 4,46 | 3,35 | -0,67 | | |

84 days after application of the product DEPIWHITE SERUM, it was observed a significant average decrease in contrast L between the spots and the surrounding skin of 20% (Wilcoxon test, p-value = 0,002) in 70% of respondents.

Therefore, as demonstrated above in tables 43 to 45, an example of a composition according to the invention significantly decreases the contrast of L parameter between the spots and the surrounding skin after 84 days of twice-daily application.

d.2. E delta :

Table 48 : results regarding the contrast spots/surrounding skin, for the parameter E delta at D84

| E delta Spot vs. surrounding skin difference | D0 | D84 | Contrast variation Delta D84 - D0 | Variation D84 (%) | Statistics |
|---|---|---|---|---|---|
| Mean ± standard | 7,35 ± 3,68 | 6,55 ± 3,46 | -0,80 ± 2,34 | -10,9 | Wilcoxon Test |

(continued)

| E delta Spot vs. surrounding skin difference | D0 | D84 | Contrast variation Delta D84 - D0 | Variation D84 (%) | Statistics |
|---|---|---|---|---|---|
| deviation | | | | | p-value = 0,0485* s |
| Minimum | 1,18 | 0,93 | -6,25 | | |
| Maximum | 20,02 | 19,91 | 2,82 | | |
| Median | 6,30 | 5,76 | -0,30 | | |

84 days after application of the product DEPIWHITE SERUM, it was observed a significant average decrease decrease in the color difference between the spots and the surrounding skin of 10.9% (Wilcoxon test, p-value = 0,0485) in 63% of respondents.

Therefore, as demonstrated above in tables 46 to 48, an example of a composition according to the invention significantly decreases the color difference between the spots and the surrounding skin of after 84 days of twice-daily application.

d.3. ITA angle (ITA°) :

i. general group :

Table 51 : results regarding the contrast spots/surrounding skin, for the ITA° parameter at D84

| ITA° Spot vs. surrounding skin difference | D0 | D84 | Contrast variation Delta D84 - D0 | Variation D84 (%) | Statistics |
|---|---|---|---|---|---|
| Mean ± standard deviation | 14.92 ± 9.05 | 13.02 ± 8.80 | -1.90 ± 4.71 | -12.8 | Wilcoxon Test p-value = 0,022* s |
| Minimum | 1.45 | 0.49 | -12.52 | | |
| Maximum | 52.04 | 49.50 | 8.80 | | |
| Median | 13.09 | 11.79 | -0.57 | | |

84 days after application of the product DEPIWHITE SERUM, it was observed a significant average decrease in ITA contrast between the spots and the surrounding skin of 12.8% (Wilcoxon test, p-value = 0,022) in 65% of respondents.

Therefore, as demonstrated above in tables 49 to 51, an example of a composition according to the invention significantly decreases the ITA contrast between the spots and the surrounding skin of after 84 days of twice-daily application.

ii. PIH/lentigo subgroup :

Table 53 : results regarding the contrast spots/surrounding skin, for the ITA° parameter, for the PIH/lentigo subgroup, at D28

| ITA° Contrast variation spot vs. surrounding skin | D0 | D28 | Delta D28 - D0 | Variation D28 (%) | Statistics |
|---|---|---|---|---|---|
| Mean ± standard deviation | 15.97 ± 7.73 | 14.22 ± 8.35 | -1.74 ± 3.58 | -10.9 | Student Test p-value = 0,0476* S |
| Minimum | 4.57 | 1.64 | -9.77 | | |
| Maximum | 37.01 | 38.68 | 3.29 | | |
| Median | 13.49 | 14.45 | -2.17 | | |

Table 54 : results regarding the contrast spots/surrounding skin, for the ITA° parameter, for the PIH/lentigo subgroup, at D56

| ITA° Contrast variation spot vs. surrounding skin | D0 | D56 | Delta D56 - D0 | Variation D56 (%) | Statistics |
|---|---|---|---|---|---|
| Mean ± standard deviation | 15.97 ± 7.73 | 13.60 ± 6.69 | -2.37 ± 5.08 | -14.8 | Student Test p-value = 0,0287*S |
| Minimum | 4.57 | 3.11 | 10.43 | | |
| Maximum | 37.01 | 28.92 | 9.19 | | |
| Median | 13.49 | 13.28 | -2.15 | | |

Table 55 : results regarding the contrast spots/surrounding skin, for the ITA° parameter, for the PIH/lentigo subgroup, at D84

| ITA° Contrast variation spot vs. surrounding skin | D0 | D84 | Delta D84 - D0 | Variation D84 (%) | Statistics |
|---|---|---|---|---|---|
| Mean ± standard deviation | 15.97 ± 7.73 | 13.79 ± 6.26 | -2.17 ± 3.91 | -13.6 | Student Test p-value = 0,0262*S |
| Minimum | 4.57 | 1.46 | -11.33 | | |
| Maximum | 37.01 | 29.11 | 2.06 | | |
| Median | 13.49 | 12.79 | -0.36 | | |

Thus, for the volunteers with PIH pigment spots and lentigo-type pigment spots, 28 days after application of the product DEPIWHITE SERUM, it was observed a significant average reduction in ITA angle contrast between the PIH/lentigo-type spots and the surrounding skin of 10.9% (Student test, p-value = 0,0476) in 68% of respondents.

56 days after application of the product DEPIWHITE SERUM, it was observed a significant average reduction in ITA angle contrast between the PIH/lentigo-type spots and the surrounding skin of 14.8% (Student test, p-value = 0.0287) in 74% of respondents.

And 84 days after application of the product DEPIWHITE SERUM, it was observed a significant average reduction in ITA angle contrast between the PIH/lentigo-type spots and the surrounding skin of 13.6% (Student test, p-value = 0,0262) in 68% of respondents.

Therefore, as demonstrated above in tables 53 to 55, an example of a composition according to the invention significantly reduces the ITA contrast between the PIH/lentigo-type spots and the surrounding skin after 28 days, 56 days and 84 days of twice-daily application.

d.4. a* parameter :

Table 56 : results regarding the contrast spots/surrounding skin, for the parameter a*, at D28

| a* Spot vs. surrounding skin difference | D0 | D28 | Contrast variation Delta D28 - D0 | Variation D28 (%) | Statistics |
|---|---|---|---|---|---|
| Mean ± standard deviation | -3.03 ± 2.47 | -2.24 ± 2.17 | 0.79 ± 2.33 | 26.0 | Student Test p-value = 0,039 S* |
| Minimum | -9.27 | -8.42 | -4.68 | | |
| Maximum | 1.72 | 1.42 | 6.02 | | |
| Median | -2.77 | -1.82 | 0.93 | | |

Table 58 : results regarding the contrast spots/surrounding skin, for the parameter a*, at D84

| a* Spot vs. surrounding skin difference | D0 | D84 | Contrast variation Delta D84 - D0 | Variation D84 (%) | Statistics |
|---|---|---|---|---|---|
| Mean ± standard deviation | -3.03 ± 2.47 | -1.97 ± 2.35 | 1.06 ± 2.46 | -34.9 | Student Test p-value = 0,0097** |
| Minimum | -9.27 | -5.53 | -4.74 | | |
| Maximum | 1.72 | 3.62 | 5.56 | | s |
| Median | -2.77 | -2.01 | 0.70 | | |

28 days after application of the product DEPIWHITE SERUM, it was observed a significant average decrease in $a\mu$ contrast between the spots and the surrounding skin of 26.0% (Student test, p-value = 0,039) in 40% of respondents.

And 84 days after application of the product DEPIWHITE SERUM, it was observed a significant average decrease in a* contrast between the spots and the surrounding skin of 34.9% (Student test, p-value = 0,0097) in 33% of respondents.

Therefore, as demonstrated above in tables 56 to 58, an example of a composition according to the invention significantly decreases redness contrast between the spots and the surrounding skin of after 28 days and 84 days of twice-daily application.

e. Results on skin color parameters based on ColorFace photos (n = 40) :

e.1. Color parameters for the entire face

Table 59 : results regarding the skin color parameters for the face (ColorFace photos)

| | L* | a* | b* | ITA° | ITA°Newtone | H76 |
|---|---|---|---|---|---|---|
| D0 | 58.14 ± 8.82 | 16.72 ± 2.45 | 18 ± 4.15 | 25 ± 20.08 | 65.96 ± 7.34 | 4.86 ± 0.7 |
| D28 | 59.19 ± 8.76 | 16.15 ± 2.06 | 17.2 ± 3.88 | 28.5 ± 20.43 | 67.18 ± 7.19 | 4.87 ± 0.58 |
| D28 - D0 | 1.05 ± 1.09 | -0.56 ± 1.11 | -0.8 ± 0.94 | 3.5 ± 2.95 | 1.22 ± 1.07 | 0.01 ± 0.39 |
| Evolution over time (%) | 1.81% | -3.37% | -4.44% | 13.98% | 1.85% | 0.10% |
| P value | S (0.001) | NS (0.092) | S (0) | S (0.001) | S (0.002) | NS (0.822) |
| Statistical significance | Friedman - Nemenyi p < 0.0001 | Friedman - Nemenyi p = 0.000 | Anova - Tukey p < 0.0001 | Friedman - Nemenyi p < 0.0001 | Friedman - Nemenyi p < 0.0001 | Friedman - Nemenyi p = 0.182 |
| D56 | 59.5 ± 8.58 | 16.04 ± 1.97 | 16.39 ± 3.89 | 30.46 ± 20.39 | 67.85 ± 7.1 | 4.78 ± 0.53 |
| D56 - D0 | 1.36 ± 1.29 | -0.67 ± 0.96 | -1.62 ± 1.3 | 5.46 ± 3.71 | 1.88 ± 1.32 | -0.08 ± 0.32 |
| Evolution over time (%) | 2.34% | -4.02% | -8.97% | 21.83% | 2.86% | -1.66% |
| P value | S (<0.0001) | S (0.003) | S (<0.0001) | S (<0.0001) | S (<0.0001) | NS (0.62) |
| Statistical significance | Friedman - Nemenyi p < 0.0001 | Friedman - Nemenyi p = 0.000 | Anova - Tukey p < 0.0001 | Friedman - Nemenyi p < 0.0001 | Friedman - Nemenyi p < 0.0001 | Friedman - Nemenyi p = 0.182 |
| D84 | 59.83 ± 8.6 | 15.87 ± 1.84 | 16.29 ± 3.84 | 31.34 ± 20.63 | 68.11 ± 7.06 | 4.83 ± 0.66 |
| D84 - D0 | 1.69 ± 1.51 | -0.85 ± 1.24 | -1.71 ± 1.64 | 6.34 ± 4.53 | 2.15 ± 1.67 | -0.03 ± 0.44 |

(continued)

|  | L* | a* | b* | ITA° | ITA°Newtone | H76 |
|---|---|---|---|---|---|---|
| Evolution over time (%) | 2.91% | -5.08% | -9.52% | 25.35% | 3.25% | -0.67% |
| P value | S (<0.0001) | S (0) | S (<0.0001) | S (<0.0001) | S (<0.0001) | NS (0.9) |
| Statistical significance | Friedman - Nemenyi p < 0.0001 | Friedman - Nemenyi p = 0.000 | Anova - Tukey p < 0.0001 | Friedman - Nemenyi p < 0.0001 | Friedman - Nemenyi p < 0.0001 | Friedman - Nemenyi p = 0.182 |

Thus, after 28 days of repeated application of the product DEPIWHITE SERUM, the results show an increase in brightness (L*) associated with a decrease in skin yellowness (b*), leading to an increase in the ITA parameter and an increase in the skin lightening ratio (IWA°Newtone).

After 56 days of repeated application of the product, the results show a decrease in skin redness (a*).

Therefore, as demonstrated above in table 59, an example of a composition according to the invention lightens the complexion after 28 days of twice-daily application.

e.2. Melanin analysis :

Table 60 : results regarding the melanin analysis (ColorFace photos)

|  | Color relative to melanin |
|---|---|
| D0 | 84.48 ± 27.22 |
| D28 | 81 ± 26.4 |
| D28 - D0 | -3.48 ± 4.28 |
| Evolution over time (%) | 4.12% |
| P value | S (0.011) |
| Statistical significance | Friedman - Nemenyi p < 0.0001 |
| D56 | 77.85 ± 26.75 |
| D56 - D0 | -6.63 ± 5.56 |
| Evolution over time (%) | -7.85% |
| P value | S (<0.0001) |
| Statistical significance | Friedman - Nemenyi p < 0.0001 |
| D84 | 77.26 ± 26.61 |
| D84 - D0 | -7.22 ± 6.75 |
| Evolution over time (%) | -8.55% |
| P value | S (<0.0001) |
| Statistical significance | Friedman - Nemenyi p < 0.0001 |

Thus, as demonstrated above in table 60, after 28 days of twice-daily application of an example of a composition according to the invention, results show a reduction in color compared to the melanin parameter, illustrating a reduction in skin pigmentation.

f. Results on color parameters in spots in ColorFace photos (n = 40)

These image analyses were carried out on a different spot or area from that of C-cube, for technical reasons.

f.1. In spots :

i. general group :

Table 61 : results regarding color parameters of spots (ColorFace photos) for the general group

| | L* pigmented spot | a* pigmented spot | b* pigmented spot | ITA° pigmented spot | ITA°Newtone pigmented spot |
|---|---|---|---|---|---|
| D0 | 53.85 ± 10.04 | 18.72 ± 2.61 | 22.08 ± 4.76 | 10.82 ± 21.35 | 60.6 ± 7.39 |
| D28 | 55.08 ± 10.13 | 17.95 ± 2.4 | 21.27 ± 4.49 | 13.96 ± 21.78 | 62.11 ± 7.27 |
| D28 - D0 | 1.23 ± 1.96 | -0.77 ± 1.41 | -0.81 ± 1.34 | 3.13 ± 4.42 | 1.51 ± 1.33 |
| Evolution over time (%) | 2.29% | -4.12% | -3.66% | 28.93% | 2.50% |
| P value | S (0.049) | S (0.003) | S (0.003) | S (0.049) | S (0.001) |
| Statistical significance | Friedman - Nemenyi p < 0.0001 | Anova - Tukey p < 0.0001 | Anova - Tukey p < 0.0001 | Friedman - Nemenyi p < 0.0001 | Friedman - Nemenyi p < 0.0001 |
| D56 | 55.56 ± 10.25 | 17.9 ± 2.14 | 20.48 ± 4.4 | 15.61 ± 22.58 | 62.82 ± 7.3 |
| D56 - D0 | 1.71 ± 2.3 | -0.83 ± 1.44 | -1.59 ± 1.42 | 4.79 ± 6.29 | 2.22 ± 1.69 |
| Evolution over time (%) | 3.18% | -4.41% | -7.22% | 44.21% | 3.66% |
| P value | S (<0.0001) | S (0.001) | S (<0.0001) | S (<0.0001) | S (<0.0001) |
| Statistical significance | Friedman - Nemenyi p < 0.0001 | Anova - Tukey p < 0.0001 | Anova - Tukey p < 0.0001 | Friedman - Nemenyi p < 0.0001 | Friedman - Nemenyi p < 0.0001 |
| D84 | 56 ± 10.4 | 17.51 ± 2.25 | 20.27 ± 4.43 | 16.49 ± 22.95 | 63.31 ± 7.31 |
| D84 - D0 | 2.15 ± 2.5 | -1.21 ± 1.6 | -1.81 ± 1.86 | 5.67 ± 6.03 | 2.71 ± 2.03 |
| Evolution over time (%) | 4.00% | -6.45% | -8.20% | 52.38% | 4.47% |
| P value | S (<0.0001) | S (<0.0001) | S (<0.0001) | S (<0.0001) | S (<0.0001) |
| Statistical significance | Friedman - Nemenyi p < 0.0001 | Anova - Tukey p < 0.0001 | Anova - Tukey p < 0.0001 | Friedman - Nemenyi p < 0.0001 | Friedman - Nemenyi p < 0.0001 |

Thus, for the general group of volunteers, as demonstrated above in table 61, after 28 days of repeated application of the product DEPIWHITE SERUM, results show an increase in brightness (L*) associated with a decrease in redness (a*), leading to an increase in the ITA parameter and an increase in the skin lightening ratio (IWA Newtone) in spots.

ii. melasma subgroup :

Table 62 : results regarding color parameters of spots (ColorFace photos) for the melasma subgroup

| | L* melasma spot | a* melasma spot | b* melasma spot | ITA° melasma spot | IWA°Newtone melasma spot |
|---|---|---|---|---|---|
| D0 | 50.03 ± 10.83 | 19 ± 2.66 | 22.78 ± 5.33 | 2.04 ± 21.29 | 57.77 ± 8.08 |
| D28 | 51.17 ± 10.93 | 18.34 ± 2.53 | 21.8 ± 5.08 | 5.13 ± 22.25 | 59.39 ± 8.24 |
| D28 - D0 | 1.14 ± 2.05 | -0.66 ± 1.54 | -0.98 ± 1.41 | 3.09 ± 4.9 | 1.62 ± 1.45 |
| Evolution over time (%) | 2.28% | -3.46% | -4.32% | 151.11% | 2.80% |
| P value | NS (0.407) | NS (0.108) | S (0.023) | NS (0.407) | S (0.045) |

(continued)

| | L* melasma spot | a* melasma spot | b* melasma spot | ITA° melasma spot | IWA°Newtone melasma spot |
|---|---|---|---|---|---|
| Statistical significance | Friedman - Nemenyi p < 0.0001 | Friedman - Nemenyi p = 0.022 | Friedman - Nemenyi p < 0.0001 | Friedman - Nemenyi p < 0.0001 | Friedman - Nemenyi p < 0.0001 |
| D56 | 52.06 ± 10.94 | 18.19 ± 2.21 | 21.02 ± 4.86 | 8.01 ± 22.89 | 60.37 ± 8.36 |
| D56 - D0 | 2.04 ± 1.74 | -0.81 ± 1.26 | -1.76 ± 1.43 | 5.97 ± 5.06 | 2.6 ± 1.67 |
| Evolution over time (%) | 4.07% | -4.27% | -7.73% | 292.08% | 4.50% |
| P value | S (0) | NS (0.108) | S (0) | S (0) | S (<0.0001) |
| Statistical significance | Friedman - Nemenyi p < 0.0001 | Friedman - Nemenyi p = 0.022 | Friedman - Nemenyi p < 0.0001 | Friedman - Nemenyi p < 0.0001 | Friedman - Nemenyi p < 0.0001 |
| D84 | 52.25 ± 11.15 | 17.89 ± 2.42 | 20.52 ± 4.87 | 8.15 ± 23.24 | 60.91 ± 8.35 |
| D84 - D0 | 2.22 ± 2.45 | -1.11 ± 1.59 | -2.26 ± 1.86 | 6.11 ± 6.03 | 3.14 ± 2.17 |
| Evolution over time (%) | 4.44% | -5.85% | -9.94% | 298.84% | 5.43% |
| P value | S (0) | S (0.023) | S (<0.0001) | S (0) | S (<0.0001) |
| Statistical significance | Friedman - Nemenyi p < 0.0001 | Friedman - Nemenyi p = 0.022 | Friedman - Nemenyi p < 0.0001 | Friedman - Nemenyi p < 0.0001 | Friedman - Nemenyi p < 0.0001 |

Thus, for the volunteers with melasma with a predominantly epidermal component, as demonstrated above in table 62, after 28 days of repeated application of the product DEPIWHITE SERUM, the results show an increase in whiteness (IWA°Newtone) in melasma-type spots.

After 56 days of repeated application of the product, the results show an increase in brightness (L*) associated with a decrease in skin pigmentation (ITA°).

In addition, after 84 days of repeated application of the product, the results show a decrease in skin redness (a*) in melasma-type spots.

iii. PIH/lentigo subgroup :

Table 63 : results regarding color parameters of spots (ColorFace photos) for the PIH/lentigo subgroup

| | L* PIH and Lentigo spot | a* PIH and Lentigo spot | b* PIH and Lentigo spot | ITA° PIH and Lentigo spot | IWA°Newtone PIH and Lentigo spot |
|---|---|---|---|---|---|
| D0 | 58.07 ± 6.96 | 18.42 ± 2.51 | 21.31 ± 3.89 | 20.53 ± 16.72 | 63.73 ± 4.95 |
| D28 | 59.41 ± 6.97 | 17.52 ± 2.16 | 20.69 ± 3.65 | 23.71 ± 16.43 | 65.13 ± 4.33 |
| D28 - D0 | 1.34 ± 1.85 | -0.9 ± 1.23 | -0.61 ± 1.24 | 3.18 ± 3.83 | 1.4 ± 1.17 |
| Evolution over time (%) | 2.30% | -4.88% | -2.87% | 15.49% | 2.19% |
| P value | S (0.035) | S (0.027) | NS (0.362) | S (0.047) | S (0) |
| Statistical significance | Anova - Tukey p = 0.001 | Anova - Tukey p = 0.001 | Friedman - Nemenyi p = 0.002 | Anova - Tukey p = 0.001 | Anova - Tukey p < 0.0001 |
| D56 | 59.43 ± 7.77 | 17.58 ± 2.02 | 19.89 ± 3.74 | 24.01 ± 18.98 | 65.53 ± 4.58 |
| D56 - D0 | 1.36 ± 2.75 | -0.84 ± 1.62 | -1.41 ± 1.38 | 3.48 ± 7.19 | 1.8 ± 1.62 |

(continued)

| | L* PIH and Lentigo spot | a* PIH and Lentigo spot | b* PIH and Lentigo spot | ITA° PIH and Lentigo spot | IWA°Newtone PIH and Lentigo spot |
|---|---|---|---|---|---|
| **Evolution over time (%)** | 2.33% | -4.56% | -6.62% | 16.94% | 2.83% |
| **P value** | S (0.032) | S (0.044) | S (0.004) | S (0.025) | S (<0.0001) |
| **Statistical significance** | Anova - Tukey p = 0.001 | Anova - Tukey p = 0.001 | Friedman - Nemenyi p = 0.002 | Anova - Tukey p = 0.001 | Anova - Tukey p < 0.0001 |
| **D84** | 60.15 ± 7.6 | 17.1 ± 1.97 | 20 ± 3.88 | 25.72 ± 18.71 | 65.97 ± 4.69 |
| **D84 - D0** | 2.08 ± 2.55 | -1.32 ± 1.61 | -1.31 ± 1.72 | 5.19 ± 6 | 2.24 ± 1.74 |
| **Evolution over time (%)** | 3.58% | -7.14% | -6.15% | 25.26% | 3.51% |
| **P value** | S (0) | S (0) | S (0.007) | S (0) | S (<0.0001) |
| **Statistical significance** | Anova - Tukey p = 0.001 | Anova - Tukey p = 0.001 | Friedman - Nemenyi p = 0.002 | Anova - Tukey p = 0.001 | Anova - Tukey p < 0.0001 |

Thus, for the volunteers with PIH pigment spots and lentigo-type pigment spots, after 28 days of repeated application of the product DEPIWHITE SERUM, the results show an increase in brightness (L*) associated with a decrease in skin redness (a*), leading to a decrease in skin pigmentation (ITA°) and an increase in skin whiteness ratio (IWA °Newtone) in PIH/lentigo-type spots.

f.2. In surrounding skin :

i. general group :

Table 64 : results regarding color parameters of surrounding skin (ColorFace photos) for the general group

| | L* surrounding skin | a* surrounding skin | b* surrounding skin | ITA° surrounding skin | IWA°Newtone surrounding skin |
|---|---|---|---|---|---|
| **D0** | 58.05 ± 10 | 16.78 ± 2.65 | 18.13 ± 4.77 | 24.37 ± 23.1 | 65.7 ± 7.84 |
| **D28** | 58.96 ± 10.07 | 16.2 ± 2.37 | 17.3 ± 4.63 | 27.61 ± 23.54 | 66.89 ± 7.73 |
| **D28 - D0** | 0.9 ± 1.64 | -0.58 ± 1.31 | -0.83 ± 1.11 | 3.24 ± 4.83 | 1.19 ± 1.25 |
| **Evolution over time (%)** | 1.55% | -3.48% | -4.59% | 13.31% | 1.81% |
| **P value** | NS (0.092) | NS (0.113) | S (0.001) | S (0.024) | S (0) |
| **Statistical significance** | Friedman - Nemenyi p < 0.0001 | Friedman - Nemenyi p = 0.024 | Anova - Tukey p < 0.0001 | Friedman - Nemenyi p < 0.0001 | Friedman - Nemenyi p < 0.0001 |
| **D56** | 59.25 ± 9.97 | 16.23 ± 2.03 | 16.57 ± 4.59 | 29.32 ± 23.59 | 67.41 ± 7.49 |
| **D56 - D0** | 1.2 ± 1.62 | -0.55 ± 1.32 | -1.56 ± 1.4 | 4.96 ± 5.02 | 1.71 ± 1.45 |
| **Evolution over time (%)** | 2.06% | -3.27% | -8.61% | 20.34% | 2.60% |
| **P value** | S (0.001) | NS (0.309) | S (<0.0001) | S (<0.0001) | S (<0.0001) |
| **Statistical significance** | Friedman - Nemenyi p < 0.0001 | Friedman - Nemenyi p = 0.024 | Anova - Tukey p < 0.0001 | Friedman - Nemenyi p < 0.0001 | Friedman - Nemenyi p < 0.0001 |

(continued)

| | L* surrounding skin | a* surrounding skin | b* surrounding skin | ITA° surrounding skin | IWA°Newtone surrounding skin |
|---|---|---|---|---|---|
| **D84** | 59.77 ± 10.07 | 16 ± 2.16 | 16.52 ± 4.48 | 30.16 ± 23.43 | 67.75 ± 7.57 |
| **D84 - D0** | 1.72 ± 1.95 | -0.78 ± 1.56 | -1.61 ± 1.93 | 5.8 ± 5.83 | 2.05 ± 1.91 |
| **Evolution over time (%)** | 2.96% | -4.63% | -8.87% | 23.79% | 3.12% |
| **P value** | S (<0.0001) | S (0.019) | S (<0.0001) | S (<0.0001) | S (<0.0001) |
| **Statistical significance** | Friedman - Nemenyi p < 0.0001 | Friedman - Nemenyi p = 0.024 | Anova - Tukey p < 0.0001 | Friedman - Nemenyi p < 0.0001 | Friedman - Nemenyi p < 0.0001 |

Thus, for the general group of volunteers, as demonstrated above in table 64, after 28 days of repeated application of the product DEPIWHITE SERUM, the results show an increase in the ITA parameter.

After 56 days of repeated application of the product, the results show an increase in brightness (L*) on the surrounding skin.

And after 84 days of repeated application of the product, the results show a decrease in redness (a*) on the surrounding skin.

ii. melasma subgroup :

Table 65 : results regarding color parameters of surrounding skin (ColorFace photos) for the melasma subgroup

| | L* surrounding skin | a* surrounding skin | b* surrounding skin | ITA° surrounding skin | IWA°Newtone surrounding skin |
|---|---|---|---|---|---|
| **D0** | 54.33 ± 10.97 | 17.63 ± 2.83 | 19.82 ± 4.56 | 13.95 ± 22.51 | 62.26 ± 8.49 |
| **D28** | 55.1 ± 11.05 | 17.01 ± 2.31 | 18.84 ± 4.27 | 16.93 ± 23.45 | 63.62 ± 8.53 |
| **D28 - D0** | 0.77 ± 1.91 | -0.62 ± 1.62 | -0.98 ± 1.2 | 2.98 ± 5.02 | 1.36 ± 1.5 |
| **Evolution over time (%)** | 1.43% | -3.51% | -4.96% | 21.39% | 2.19% |
| **P value** | NS (0.774) | NS (0.168) | S (0.01) | NS (0.407) | S (0.032) |
| **Statistical significance** | Friedman - Nemenyi p = 0.000 | Anova - Tukey p = 0.023 | Anova - Tukey p < 0.0001 | Friedman - Nemenyi p < 0.0001 | Friedman - Nemenyi p < 0.0001 |
| **D56** | 55.75 ± 10.9 | 16.89 ± 2.1 | 18.17 ± 4.17 | 19.52 ± 23.74 | 64.39 ± 8.42 |
| **D56 - D0** | 1.41 ± 1.6 | -0.74 ± 1.24 | -1.65 ± 1.3 | 5.58 ± 5.18 | 2.13 ± 1.41 |
| **Evolution over time (%)** | 2.60% | -4.20% | -8.34% | 39.98% | 3.43% |
| **P value** | S (0.032) | NS (0.069) | S (<0.0001) | S (0.003) | S (<0.0001) |
| **Statistical significance** | Friedman - Nemenyi p = 0.000 | Anova - Tukey p = 0.023 | Anova - Tukey p < 0.0001 | Friedman - Nemenyi p < 0.0001 | Friedman - Nemenyi p < 0.0001 |
| **D84** | 56.18 ± 11.14 | 16.76 ± 2.31 | 17.83 ± 4.03 | 20.6 ± 24.13 | 64.81 ± 8.63 |
| **D84 - D0** | 1.85 ± 2.05 | -0.87 ± 1.6 | -1.99 ± 1.93 | 6.65 ± 6.5 | 2.56 ± 2.08 |
| **Evolution over time (%)** | 3.41% | -4.95% | -10.06% | 47.68% | 4.11% |
| **P value** | S (0) | S (0.023) | S (<0.0001) | S (0) | S (<0.0001) |

(continued)

| | L* surrounding skin | a* surrounding skin | b* surrounding skin | ITA° surrounding skin | IWA°Newtone surrounding skin |
|---|---|---|---|---|---|
| Statistical significance | Friedman - Nemenyi p = 0.000 | Anova - Tukey p = 0.023 | Anova - Tukey p < 0.0001 | Friedman - Nemenyi p < 0.0001 | Friedman - Nemenyi p < 0.0001 |

Thus, for the volunteers with melasma with a predominantly epidermal component, as demonstrated above in table 65, after 28 days of repeated application of the product DEPIWHITE SERUM, the results show an increase in skin whiteness (IWA∞Newtone) on the skin surrounding the melasma.

After 56 days of repeated application of the product, the results show an increase in brightness (L*) associated with a decrease in skin pigmentation (ITA°) on the skin surrounding the melasma.

Moreover, after 56 days of repeated application of the product, more than 50% of the volunteers responded in terms of the evolution of skin redness on the skin surrounding melasma (a*), with a p-value close to significance (0.069).

And after 84 days of repeated application of the product, the results show a decrease in skin redness (a*) on the skin surrounding melasma.

iii. PIH/lentigo subgroup :

Table 66 : results regarding color parameters of surrounding skin (ColorFace photos) for the PIH/lentigo subgroup

| | L* surrounding skin | a* surrounding skin | b* surrounding skin | ITA° surrounding skin | IWA°Newtone surrounding skin |
|---|---|---|---|---|---|
| D0 | 62.17 ± 6.75 | 15.84 ± 2.05 | 16.26 ± 4.28 | 35.88 ± 17.62 | 69.51 ± 4.71 |
| D28 | 63.21 ± 6.64 | 15.29 ± 2.09 | 15.59 ± 4.41 | 39.41 ± 17.11 | 70.51 ± 4.54 |
| D28 - D0 | 1.04 ± 1.25 | -0.55 ± 0.85 | -0.67 ± 0.97 | 3.53 ± 3.53 | 1 ± 0.85 |
| Evolution over time (%) | 1.67% | -3.46% | -4.10% | 9.85% | 1.44% |
| P value | S (0.025) | NS (0.233) | NS (0.149) | NS (0.06) | S (0.01) |
| Statistical significance | Anova - Tukey p = 0.001 | Anova - Tukey p = 0.109 | Anova - Tukey p < 0.0001 | Friedman - Nemenyi p = 0.000 | Friedman - Nemenyi p = 0.000 |
| D56 | 63.12 ± 7.02 | 15.5 ± 1.68 | 14.79 ± 4.37 | 40.15 ± 18.02 | 70.75 ± 4.32 |
| D56 - D0 | 0.95 ± 1.61 | -0.34 ± 1.38 | -1.46 ± 1.49 | 4.27 ± 4.76 | 1.24 ± 1.34 |
| Evolution over time (%) | 1.53% | -2.13% | -8.99% | 11.91% | 1.78% |
| P value | S (0.046) | NS (0.64) | S (<0.0001) | S (0.015) | S (0.003) |
| Statistical significance | Anova - Tukey p = 0.001 | Anova - Tukey p = 0.109 | Anova - Tukey p < 0.0001 | Friedman - Nemenyi p = 0.000 | Friedman - Nemenyi p = 0.000 |
| D84 | 63.74 ± 6.79 | 15.17 ± 1.61 | 15.07 ± 4.51 | 40.73 ± 17.31 | 70.99 ± 4.28 |
| D84 - D0 | 1.57 ± 1.82 | -0.67 ± 1.5 | -1.18 ± 1.85 | 4.85 ± 4.82 | 1.48 ± 1.51 |
| Evolution over time (%) | 2.52% | -4.23% | -7.27% | 13.52% | 2.13% |
| P value | S (0) | NS (0.1) | S (0.002) | S (0) | S (0) |
| Statistical significance | Anova - Tukey p = 0.001 | Anova - Tukey p = 0.109 | Anova - Tukey p < 0.0001 | Friedman - Nemenyi p = 0.000 | Friedman - Nemenyi p = 0.000 |

Thus, for the volunteers with PIH pigment spots and lentigo-type pigment spots, as demonstrated in table 66, after 28 days of repeated application of the product DEPIWHITE SERUM, the results show an increase in brightness (L*) associated with an increase in skin whiteness (IWA°Newtone) on the surrounding skin.

After 56 days of repeated application of the product, the results show a decrease in skin pigmentation (ITA°) on the surrounding skin.

f.3. Contrasts results :

i. general group :

Table 67 : results regarding the contrasts of the color parameters spots VS surrounding skin (ColorFace photos) for the general group

| | L* contrast | a* contrast | b* contrast | ITA° contrast | dE76 |
|---|---|---|---|---|---|
| D0 | 4.23 ± 2.11 | 2.2 ± 1.4 | 4.13 ± 2.19 | 13.54 ± 6.86 | 6.67 ± 2.56 |
| D28 | 3.88 ± 1.77 | 2.08 ± 1.22 | 4.23 ± 2.31 | 13.66 ± 6.68 | 6.45 ± 2.39 |
| D28 - D0 | -0.35 ± 0.92 | -0.12 ± 0.64 | 0.1 ± 0.74 | 0.11 ± 2.46 | -0.23 ± 0.9 |
| Evolution over time (%) | -8.27% | -5.47% | 2.42% | 0.83% | -3.38% |
| P value | NS (0.113) | NS (0.9) | NS (0.768) | NS (0.973) | NS (0.484) |
| Statistical significance | Friedman - Nemenyi p < 0.0001 | Friedman - Nemenyi p = 0.005 | Anova Tukey p = 0.376 | Friedman - Nemenyi p = 0.966 | Anova Tukey p = 0.027 |
| D56 | 3.7 ± 1.67 | 2.03 ± 1.15 | 4.21 ± 2.32 | 13.71 ± 6.45 | 6.3 ± 2.3 |
| D56 - D0 | -0.53 ± 1.31 | -0.18 ± 0.75 | 0.07 ± 0.76 | 0.17 ± 3.59 | -0.37 ± 1.28 |
| Evolution over time (%) | -12.60% | -8.06% | 1.75% | 1.27% | -5.58% |
| P value | S (<0.0001) | NS (0.456) | NS (0.897) | NS (0.994) | NS (0.09) |
| Statistical significance | Friedman - Nemenyi p < 0.0001 | Friedman - Nemenyi p = 0.005 | Anova Tukey p = 0.376 | Friedman - Nemenyi p = 0.966 | Anova Tukey p = 0.027 |
| D84 | 3.8 ± 1.91 | 1.86 ± 1.13 | 4.07 ± 2.24 | 13.67 ± 6.32 | 6.22 ± 2.38 |
| D84 - D0 | -0.43 ± 1.3 | -0.34 ± 0.78 | -0.07 ± 0.77 | 0.13 ± 3.4 | -0.45 ± 1.26 |
| Evolution over time (%) | -10.19% | -15.58% | -1.60% | 0.93% | -6.76% |
| P value | S (0.001) | S (0.005) | NS (0.92) | NS (1) | S (0.025) |
| Statistical significance | Friedman - Nemenyi p < 0.0001 | Friedman - Nemenyi p = 0.005 | Anova Tukey p = 0.376 | Friedman - Nemenyi p = 0.966 | Anova Tukey p = 0.027 |

Thus, as demonstrated above in table 67, after 56 days of repeated application of the product DEPIWHITE SERUM, the results show a decrease in light contrast (L*). And after 84 days of repeated application of the product, results show a decrease in skin redness contrast (a*) and the visibility of pigment spots (dE76).

ii. melasma subgroup :

Table 68 : results regarding the contrasts of the color parameters spots VS surrounding skin (ColorFace photos) for the melasma subgroup

| | L* contrast | a* contrast | b* contrast | ITA° contrast | dE76 |
|---|---|---|---|---|---|
| D0 | 4.31 ± 2.27 | 1.82 ± 1.06 | 3.31 ± 1.83 | 11.9 ± 6.27 | 6.13 ± 2.2 |

(continued)

|  | L* contrast | a* contrast | b* contrast | ITA° contrast | dE76 |
|---|---|---|---|---|---|
| D28 | 3.94 ± 2.03 | 1.82 ± 1.07 | 3.27 ± 1.92 | 11.8 ± 5.77 | 5.78 ± 2.24 |
| D28 - D0 | -0.37 ± 0.8 | 0 ± 0.41 | -0.04 ± 0.58 | -0.1 ± 2.01 | -0.34 ± 0.8 |
| Evolution over time (%) | -8.51% | -0.26% | -1.18% | -0.88% | -5.58% |
| P value | NS (0.705) | NS (1) | NS (0.991) | NS (0.999) | NS (0.21) |
| Statistical signifi-cance | Friedman - Ne-menyi p = 0.027 | Friedman - Nemenyi p = 0.419 | Anova Tukey p = 0.570 | Friedman - Nemenyi p = 0.653 | Anova Tukey p = 0.004 |
| D56 | 3.68 ± 1.88 | 1.78 ± 1.02 | 3.21 ± 1.77 | 11.51 ± 5.34 | 5.5 ± 2.13 |
| D56 - D0 | -0.62 ± 0.81 | -0.05 ± 0.5 | -0.09 ± 0.6 | -0.39 ± 2.38 | -0.63 ± 0.85 |
| Evolution over time (%) | -14.42% | -2.58% | -2.76% | -3.29% | -10.21% |
| P value | S (0.023) | NS (0.89) | NS (0.899) | NS (1) | S (0.003) |
| Statistical signifi-cance | Friedman - Ne-menyi p = 0.027 | Friedman - Nemenyi p = 0.419 | Anova Tukey p = 0.570 | Friedman - Nemenyi p = 0.653 | Anova Tukey p = 0.004 |
| D84 | 3.94 ± 2.28 | 1.67 ± 0.93 | 3.13 ± 1.76 | 12.45 ± 6.49 | 5.62 ± 2.37 |
| D84 - D0 | -0.37 ± 0.77 | -0.15 ± 0.5 | -0.18 ± 0.79 | 0.54 ± 2.17 | -0.5 ± 0.71 |
| Evolution over time (%) | -8.52% | -8.46% | -5.33% | 4.57% | -8.23% |
| P value | NS (0.179) | NS (0.479) | NS (0.539) | NS (0.705) | S (0.025) |
| Statistical signifi-cance | Friedman - Ne-menyi p = 0.027 | Friedman - Nemenyi p = 0.419 | Anova Tukey p = 0.570 | Friedman - Nemenyi p = 0.653 | Anova Tukey p = 0.004 |

Thus, as demonstrated above in table 68, for the volunteers with melasma with a predominantly epidermal component, after 56 days of repeated application of the product DEPIWHITE SERUM, the results show a decrease in skin brightness contrast of melasma (L*) associated with a decrease in the visibility of melasma (dE76).

iii. PIH/lentigo subgroup :

Table 69 : results regarding the contrasts of the color parameters spots VS surrounding skin (ColorFace photos) for the PIH/lentigo subgroup

|  | L* contrast | a* contrast | b* contrast | ITA° contrast | dE76 |
|---|---|---|---|---|---|
| D0 | 4.16 ± 1.91 | 2.63 ± 1.6 | 5.05 ± 2.19 | 15.35 ± 7.03 | 7.28 ± 2.77 |
| D28 | 3.82 ± 1.41 | 2.38 ± 1.3 | 5.3 ± 2.22 | 15.71 ± 7.01 | 7.18 ± 2.33 |
| D28 - D0 | -0.33 ± 1.04 | -0.25 ± 0.8 | 0.25 ± 0.87 | 0.35 ± 2.86 | -0.1 ± 0.99 |
| Evolution over time (%) | -8.00% | -9.47% | 5.03% | 2.30% | -1.33% |
| P value | NS (0.612) | NS (0.746) | NS (0.388) | NS (0.968) | NS (0.983) |
| Statistical significance | Anova Tukey p = 0.264 | Friedman - Nemenyi p = 0.007 | Anova Tukey p = 0.257 | Anova Tukey p = 0.504 | Anova Tukey p = 0.487 |
| D56 | 3.72 ± 1.41 | 2.31 ± 1.23 | 5.3 ± 2.36 | 16.15 ± 6.7 | 7.19 ± 2.16 |
| D56 - D0 | -0.44 ± 1.69 | -0.32 ± 0.93 | 0.25 ± 0.86 | 0.79 ± 4.48 | -0.09 ± 1.59 |
| Evolution over time (%) | -10.53% | -12.25% | 5.02% | 5.18% | -1.27% |

(continued)

| | L* contrast | a* contrast | b* contrast | ITA° contrast | dE76 |
|---|---|---|---|---|---|
| P value | NS (0.378) | NS (0.512) | NS (0.89) | NS (0.731) | NS (0.985) |
| Statistical significance | Anova Tukey p = 0.264 | Friedman - Nemenyi p = 0.007 | Anova Tukey p = 0.257 | Anova Tukey p = 0.504 | Anova Tukey p = 0.487 |
| D84 | 3.65 ± 1.36 | 2.07 ± 1.28 | 5.11 ± 2.26 | 15.02 ± 5.83 | 6.89 ± 2.21 |
| D84 - D0 | -0.5 ± 1.71 | -0.55 ± 0.96 | 0.06 ± 0.72 | -0.34 ± 4.33 | -0.39 ± 1.67 |
| Evolution over time (%) | -12.09% | -21.04% | 1.11% | -2.18% | -5.39% |
| P value | NS (0.258) | S (0.004) | NS (0.985) | NS (0.972) | NS (0.459) |
| Statistical significance | Anova Tukey p = 0.264 | Friedman - Nemenyi p = 0.007 | Anova Tukey p = 0.257 | Anova Tukey p = 0.504 | Anova Tukey p = 0.487 |

Thus, as demonstrated above in table 69, for the volunteers with PIH pigment spots and lentigo-type pigment spots, after 84 days of repeated application of the product DEPIWHITE SERUM, the results show a reduction in skin redness contrast (a*).

Conclusion regarding the color parameters in spots in ColorFace photos :

The results above show that after 84 days of twice-daily application, an example of a composition according to the invention allows to reduce the color difference (Delta E) between spots and surrounding skin, thus proven. the anti-dark spot effectiveness of the composition of the invention.

Moreover, the results above allow to conclude that an example of a composition according to the invention allows the reduction of the color difference between melasma-type spots and the surrounding skin after 56 days of twice-daily application.

Furthermore, the results above allow to conclude that an example of a composition according to the invention allows the reduction of the skin redness contrast between PIH/lentigo-type spots and the surrounding skin after 84 days of twice-daily application.

g. Hands analysis (n=5) :

g.1. ITA angle parameter (ITA°) :

Table 70 : results regarding hand analysis, for the ITA° parameter

| N° Datacapt | ITA° of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 7.05 | 13.54 | 14.50 | 22.21 | 6.49 | 7.45 | 15.16 | 92 | 106 | 215 |
| BIO-EC-0005 | 18.26 | 36.49 | 41.20 | 41.31 | 18.23 | 34.15 | 23.05 | 100 | 187 | 126 |
| BIO-EC-0006 | -21.71 | 11.02 | 12.10 | 14.81 | 32.72 | -6.16 | 36.52 | -151 | 28 | -168 |
| BIO-EC-0007 | 11.77 | 23.34 | 17.90 | 24.14 | 11.56 | 39.61 | 12.37 | 98 | 336 | 105 |
| BIO-EC-0008 | 20.28 | 28.78 | 37.39 | 39.31 | 8.49 | 25.62 | 19.02 | 42 | 126 | 94 |

(continued)

| N° Datacapt | ITA° of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| Mean | 7.13 | 22.63 | 24.62 | 28.36 | 15.50 | 20.13 | 21.22 | | | |
| Stand. dev. | 16.96 | 10.58 | 13.62 | 11.48 | 10.60 | 19.09 | 9.45 | 217.3 | 282.2 | 297.5 |
| Min | -21.71 | 11.02 | 12.10 | 14.81 | 6.49 | -6.16 | 12.37 | | | |
| Max | 20.28 | 36.49 | 41.20 | 41.31 | 32.72 | 39.61 | 36.52 | | | |
| Median | 11.77 | 23.34 | 17.90 | 24.14 | 11.56 | 25.62 | 19.02 | | | |
| | | | | | 100% | 80% | 100% | | | |

| N° Datacapt | ITA° of the surrounding skin | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 34.91 | 42.31 | 39.30 | 44.80 | 7.40 | 4.39 | 9.88 | 21 | 13 | 28 |
| BIO-EC-0005 | 46.98 | 44.70 | 55.76 | 56.07 | -2.28 | 20.85 | 9.09 | -5 | 44 | 19 |
| BIO-EC-0006 | 20.09 | 29.28 | 36.62 | 39.93 | 9.19 | -10.35 | 19.84 | 46 | -52 | 99 |
| BIO-EC-0007 | 33.72 | 44.00 | 36.93 | 40.05 | 10.28 | 16.85 | 6.33 | 30 | 50 | 19 |
| BIO-EC-0008 | 42.79 | 53.66 | 56.32 | 56.12 | 10.87 | 22.60 | 13.34 | 25 | 53 | 31 |
| Mean | 35.70 | 42.79 | 44.99 | 47.39 | 7.09 | 10.87 | 11.70 | | | |
| Stand. dev. | 10.32 | 8.75 | 10.14 | 8.18 | 5.40 | 13.83 | 5.19 | 19.9 | 30.4 | 32.8 |
| Min | 20.09 | 29.28 | 36.62 | 39.93 | -2.28 | -10.35 | 6.33 | | | |
| Max | 46.98 | 53.66 | 56.32 | 56.12 | 10.87 | 22.60 | 19.84 | | | |
| Median | 34.91 | 44.00 | 39.30 | 44.80 | 9.19 | 16.85 | 9.88 | | | |
| | | | | | 80% | 80% | 100% | | | |

g.2. L* parameter :

Table 71 : results regarding hand analysis, for the L* parameter

| N° Datacapt | L* of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 51.84 | 53.90 | 53.52 | 56.57 | 2.05 | 1.67 | 4.73 | 4 | 3 | 9 |
| BIO-EC-0005 | 55.52 | 61.34 | 63.41 | 62.08 | 5.82 | 11.57 | 6.56 | 10 | 21 | 12 |
| BIO-EC-0006 | 42.49 | 54.08 | 54.83 | 55.85 | 11.59 | -0.69 | 13.36 | 27 | -2 | 31 |

(continued)

| N° Datacapt | L* of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0007 | 53.42 | 58.02 | 56.28 | 58.33 | 4.59 | 13.80 | 4.91 | 9 | 26 | 9 |
| BIO-EC-0008 | 56.40 | 60.04 | 62.71 | 62.27 | 3.64 | 9.28 | 5.87 | 6 | 16 | 10 |
| Mean | 51.93 | 57.47 | 58.15 | 59.02 | 5.54 | 7.13 | 7.09 | | | |
| Stand. dev. | 5.57 | 3.40 | 4.59 | 3.02 | 3.65 | 6.32 | 3.59 | 10.7 | 13.7 | 13.6 |
| Min | 42.49 | 53.90 | 53.52 | 55.85 | 2.05 | -0.69 | 4.73 | | | |
| Max | 56.40 | 61.34 | 63.41 | 62.27 | 11.59 | 13.80 | 13.36 | | | |
| Median | 53.42 | 58.02 | 56.28 | 58.33 | 4.59 | 9.28 | 5.87 | | | |
| | | | | | 100% | 80% | 100% | | | |

| N° Datacapt | L* of the surrounding skin | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 60.95 | 64.20 | 64.04 | 66.74 | 3.26 | 3.09 | 5.79 | 5 | 5 | 10 |
| BIO-EC-0005 | 65.04 | 65.64 | 67.62 | 66.61 | 0.61 | 6.68 | 1.57 | 1 | 10 | 2 |
| BIO-EC-0006 | 55.41 | 59.71 | 64.12 | 62.54 | 4.30 | -0.92 | 7.13 | 8 | -2 | 13 |
| BIO-EC-0007 | 59.23 | 68.36 | 63.18 | 66.63 | 9.13 | 7.77 | 7.40 | 15 | 13 | 12 |
| BIO-EC-0008 | 64.06 | 64.52 | 68.64 | 69.38 | 0.46 | 9.41 | 5.32 | 1 | 15 | 8 |
| Mean | 60.94 | 64.49 | 65.52 | 66.38 | 3.55 | 5.20 | 5.44 | | | |
| Stand. dev. | 3.87 | 3.13 | 2.44 | 2.45 | 3.54 | 4.13 | 2.33 | 5.8 | 8.5 | 8.9 |
| Min | 55.41 | 59.71 | 63.18 | 62.54 | 0.46 | -0.92 | 1.57 | | | |
| Max | 65.04 | 68.36 | 68.64 | 69.38 | 9.13 | 9.41 | 7.40 | | | |
| Median | 60.95 | 64.52 | 64.12 | 66.63 | 3.26 | 6.68 | 5.79 | | | |
| | | | | | 100% | 80% | 100% | | | |

g.3. a* parameter :

Table 72 : results regarding hand analysis, for the a* parameter

| N° Datacapt | a* of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 19.23 | 14.81 | 17.32 | 14.84 | -4.42 | -1.91 | -4.39 | -23 | -10 | -23 |

(continued)

| N° Datacapt | a* of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0005 | 16.57 | 17.53 | 12.39 | 14.10 | 0.96 | -6.84 | -2.47 | 6 | -41 | -15 |
| BIO-EC-0006 | 17.23 | 18.17 | 17.35 | 18.06 | 0.94 | 0.77 | 0.83 | 5 | 4 | 5 |
| BIO-EC-0007 | 17.07 | 15.09 | 17.12 | 14.64 | -1.98 | -0.11 | -2.43 | -12 | -1 | -14 |
| BIO-EC-0008 | 17.13 | 14.12 | 12.81 | 12.72 | -3.01 | -4.26 | -4.41 | -18 | -25 | -26 |
| Mean | 17.45 | 15.95 | 15.40 | 14.87 | -1.50 | -2.47 | -2.57 | | | |
| Stand. dev. | 1.03 | 1.79 | 2.56 | 1.97 | 2.40 | 3.11 | 2.14 | -8.6 | -14.2 | -14.8 |
| Min | 16.57 | 14.12 | 12.39 | 12.72 | -4.42 | -6.84 | -4.41 | | | |
| Max | 19.23 | 18.17 | 17.35 | 18.06 | 0.96 | 0.77 | 0.83 | | | |
| Median | 17.13 | 15.09 | 17.12 | 14.64 | -1.98 | -1.91 | -2.47 | | | |
| | | | | | 60% | 80% | 80% | | | |

| N° Datacapt | a* of the surrounding skin | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 17.07 | 13.75 | 17.59 | 13.27 | -3.32 | 0.52 | -3.80 | -19 | 3 | -22 |
| BIO-EC-0005 | 11.34 | 14.02 | 13.25 | 11.84 | 2.68 | -3.82 | 0.50 | 24 | -34 | 4 |
| BIO-EC-0006 | 18.67 | 17.96 | 14.94 | 14.37 | -0.72 | 3.60 | -4.31 | -4 | 19 | -23 |
| BIO-EC-0007 | 17.40 | 8.71 | 15.83 | 10.03 | -8.69 | -2.85 | -7.37 | -50 | -16 | -42 |
| BIO-EC-0008 | 13.57 | 11.34 | 11.17 | 10.75 | -2.22 | -6.23 | -2.81 | -16 | -46 | -21 |
| Mean | 15.61 | 13.15 | 14.56 | 12.05 | -2.46 | -1.75 | -3.56 | | | |
| Stand. dev. | 3.05 | 3.44 | 2.46 | 1.78 | 4.16 | 3.85 | 2.83 | -15.7 | -11.2 | -22.8 |
| Min | 11.34 | 8.71 | 11.17 | 10.03 | -8.69 | -6.23 | -7.37 | | | |
| Max | 18.67 | 17.96 | 17.59 | 14.37 | 2.68 | 3.60 | 0.50 | | | |
| Median | 17.07 | 13.75 | 14.94 | 11.84 | -2.22 | -2.85 | -3.80 | | | |
| | | | | | 80% | 60% | 80% | | | |

g.4. b* parameter :

Table 73 : results regarding hand analysis, for the b* parameter

| N° Datacapt | b* of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 16.86 | 17.10 | 14.49 | 16.34 | 0.24 | -2.37 | -0.52 | 1 | -14 | -3 |
| BIO-EC-0005 | 17.32 | 15.45 | 15.32 | 13.73 | -1.87 | -1.54 | -3.59 | -11 | -9 | -21 |
| BIO-EC-0006 | 18.78 | 22.84 | 23.68 | 23.61 | 4.06 | 6.36 | 4.83 | 22 | 34 | 26 |
| BIO-EC-0007 | 17.91 | 18.88 | 19.85 | 18.69 | 0.98 | 1.07 | 0.78 | 5 | 6 | 4 |
| BIO-EC-0008 | 17.76 | 18.53 | 16.66 | 14.90 | 0.77 | -1.25 | -2.86 | 4 | -7 | -16 |
| Mean | 17.73 | 18.56 | 18.00 | 17.46 | 0.83 | 0.46 | -0.27 | 4.7 | 2.6 | -1.5 |
| Stand. dev. | 0.72 | 2.75 | 3.78 | 3.91 | 2.13 | 3.54 | 3.35 | | | |
| Min | 16.86 | 15.45 | 14.49 | 13.73 | -1.87 | -2.37 | -3.59 | | | |
| Max | 18.78 | 22.84 | 23.68 | 23.61 | 4.06 | 6.36 | 4.83 | | | |
| Median | 17.76 | 18.53 | 16.66 | 16.34 | 0.77 | -1.25 | -0.52 | | | |

| N° Datacapt | b* of the surrounding skin | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 15.67 | 15.59 | 17.12 | 16.87 | -0.07 | 1.46 | 1.21 | 0 | 9 | 8 |
| BIO-EC-0005 | 14.01 | 15.83 | 12.02 | 11.14 | 1.82 | -3.65 | -2.87 | 13 | -26 | -20 |
| BIO-EC-0006 | 15.01 | 17.43 | 19.04 | 15.11 | 2.42 | 5.02 | 0.11 | 16 | 33 | 1 |
| BIO-EC-0007 | 13.92 | 19.09 | 17.57 | 19.86 | 5.16 | 2.56 | 5.94 | 37 | 18 | 43 |
| BIO-EC-0008 | 15.21 | 10.63 | 12.44 | 13.00 | -4.58 | -1.48 | -2.21 | -30 | -10 | -15 |
| Mean | 14.76 | 15.71 | 15.64 | 15.20 | 0.95 | 0.78 | 0.43 | 6.4 | 5.3 | 2.9 |
| Stand. dev. | 0.77 | 3.17 | 3.19 | 3.38 | 3.62 | 3.41 | 3.49 | | | |
| Min | 13.92 | 10.63 | 12.02 | 11.14 | -4.58 | -3.65 | -2.87 | | | |
| Max | 15.67 | 19.09 | 19.04 | 19.86 | 5.16 | 5.02 | 5.94 | | | |
| Median | 15.01 | 15.83 | 17.12 | 15.11 | 1.82 | 1.46 | 0.11 | | | |

g.5. parameter of surface area of spots :

Table 74 : results regarding hand analysis, for the parameter of surface area of spots

| N° Datacapt | Surface (in mm²) of spot | | | | Delta D28-D0 | Delta D56-D0 | Delta D84-D0 | % Variation D28 | % Variation D56 | % Variation D84 |
|---|---|---|---|---|---|---|---|---|---|---|
| | D0 | D28 | D56 | D84 | | | | | | |
| BIO-EC-0001 | 10.80 | 10.50 | 9.20 | 7.00 | -0.30 | -1.60 | -3.80 | -3 | -15 | -35 |
| BIO-EC-0005 | 2.40 | 1.40 | 1.90 | 2.00 | -1.00 | -0.50 | -0.40 | -42 | -21 | -17 |
| BIO-EC-0006 | 1.00 | 0.70 | 0.60 | 0.70 | -0.30 | -0.40 | -0.30 | -30 | -40 | -30 |
| BIO-EC-0007 | 48.30 | 41.60 | 43.00 | 44.70 | -6.70 | -5.30 | -3.60 | -14 | -11 | -7 |
| BIO-EC-0008 | 19.60 | 17.90 | 19.00 | 18.90 | -1.70 | -0.60 | -0.70 | -9 | -3 | -4 |
| Mean | 16.42 | 14.42 | 14.74 | 14.66 | -2.00 | -1.68 | -1.76 | | | |
| Stand. dev. | 19.32 | 16.76 | 17.41 | 18.26 | 2.69 | 2.08 | 1.78 | -12.2 | -10.2 | -10.7 |
| Min | 1.00 | 0.70 | 0.60 | 0.70 | -6.70 | -5.30 | -3.80 | | | |
| Max | 48.30 | 41.60 | 43.00 | 44.70 | -0.30 | -0.40 | -0.30 | | | |
| Median | 10.80 | 10.50 | 9.20 | 7.00 | -1.00 | -0.60 | -0.70 | | | |
| | | | | | | | | 100% | 100% | 100% |

g.6. E Delta parameter :

Table 75 : results regarding hand analysis, for the E delta parameter

| N° Datacapt | (Dn - D0) | | | | | | |
|---|---|---|---|---|---|---|---|
| | ΔE*ab - spot vs surrounding skin | | | | Delta | | |
| | D0 | D28 | D56 | D84 | D28 - D0 | D56 - D0 | D84 - D0 |
| BIO-EC-0001 | 9.44 | 10.47 | 10.85 | 10.3 | 1.04 | 1.41 | 0.87 |
| BIO-EC-0005 | 11.35 | 5.56 | 5.42 | 5.69 | -5.79 | -5.93 | -5.66 |
| BIO-EC-0006 | 13.54 | 7.81 | 10.67 | 11.43 | -5.73 | -2.87 | -2.11 |
| BIO-EC-0007 | 7.05 | 12.16 | 7.37 | 9.57 | 5.11 | 0.32 | 2.52 |
| BIO-EC-0008 | 8.82 | 9.50 | 7.46 | 7.62 | 0.67 | -1.36 | -1.21 |
| Mean | 10.04 | 9.10 | 8.35 | 8.92 | -0.94 | -1.69 | -1.12 |
| Stand. dev. | 2.49 | 2.53 | 2.34 | 2.28 | 4.73 | 2.88 | 3.11 |
| Min | 7.05 | 5.56 | 5.42 | 5.69 | -5.79 | -5.93 | -5.66 |
| Max | 13.54 | 12.16 | 10.85 | 11.43 | 5.11 | 1.41 | 2.52 |
| Median | 9.44 | 9.50 | 7.46 | 9.57 | 0.67 | -1.36 | -1.21 |
| | | | | | 40% | 60% | 60% |
| % Variation | | | | | -9.4 | -16.8 | -11.1 |

Conclusion regarding the effect of the product on the hands :

As demonstrated above, on tables 70 to 75, an example of a composition according to the invention allows to lighten (ITA° and L* parameters) the color of the spots and the surrounding skin on the hands after 28 days, 56 days, and 84 days of twice-daily application.

Moreover, these results show that a composition according to the invention allows to reduce the surface area/size of spots on the hands after 28 days, 56 days and 84 days of twice-daily application.

These results are obtained on hands in which the serum of the present invention has been applied, with no previous application of solar protection. Therefore, these results demonstrate that the advantageous decrease of pigmentation is only due to the serum of the present invention, and is not due to the solar protective cream applied on the other part of the skin of the volunteers, nor to the combination of the serum of the present invention and the solar protective cream.

h. Results of the self-assessment questionnaire (n = 40) :

Table 76 : self-assessment questionnaire

| Questions | Opinion/feeling of the volunteers (in %), for each frequence of application | | | | |
|---|---|---|---|---|---|
| | D0 | D7 | D28 | D56 | D84 |
| The texture is pleasant | 93 | / | / | / | |
| The fragrance is pleasant | 76 | / | / | / | |
| The texture penetrates quickly | 98 | / | / | / | |
| The product has a non-sticky finish on the skin | 70 | / | / | / | |
| The product has a non-greasy finish on the skin | 90 | / | / | / | |
| The product can be a good base for makeup | 80 | / | / | / | |
| The product gives me a feeling of efficiency | 65 | / | / | / | |
| The complexion is more radiant | 63 | 58 | 100 | 78 | 80 |
| The complexion is fresher | 63 | / | / | / | |
| The complexion is more even/uniform | 58 | 78 | 88 | 95 | 90 |
| (Instantly) skin is more bright/luminous | 58 | 55 | 90 | 80 | 81 |
| The skin is brightened | / | 53 | 93 | 75 | 78 |
| The stains/spots are faded | / | / | 70 | 78 | 88 |
| The edges of the spots are | / | / | 73 | 75 | 83 |
| blurred | | | | | |
| The skin is visibly more hydrated | 83 | 73 | / | / | |
| The skin feels comfortable after applying the product | 90 | / | / | / | |
| The product does not cause any irritation | 80 | / | / | / | |
| The product is suitable for my skin type | 88 | 80 | / | / | |
| The product is effective | / | 50 | 100 | 85 | 85 |
| The product effectively corrects blemishes/spots/stains | | | 70 | 7 | 86 |
| The complexion is visibly more even | / | 60 | 100 | 93 | 83 |
| The stains are less visible | / | / | 83 | 80 | 85 |
| There are fewer stains | / | / | 95 | 68 | 68 |
| The intensity of the spots has decreased | / | / | 88 | 83 | 88 |
| The size of the spots is reduced | / | / | 80 | 75 | 83 |
| The color of the spots is lightened | / | / | 75 | 80 | 85 |
| The product promotes the disappearance of spots | / | / | 80 | 70 | 78 |

(continued)

| Questions | Opinion/feeling of the volunteers (in %), for each frequence of application | | | | |
|---|---|---|---|---|---|
| | D0 | D7 | D28 | D56 | D84 |
| The product lightens hyperpigmented areas of the face | / | / | 83 | 70 | 83 |
| The product suits my skin tone | / | 88 | / | / | |
| The product reduces stubborn stains | / | / | 80 | 70 | 78 |
| The product prevents the appearance of new spots (for a long time) | / | / | 93 | 58 | 75 |
| The product has a long-lasting effect on the complexion | / | / | / | / | 80 |
| The product has a long-lasting effect on the spots | / | / | / | / | 85 |
| I am satisfied with this product | 70 | 68 | 88 | 73 | 85 |
| I could recommend this product to someone | 68 | 65 | 85 | 70 | 83 |

Moreover, after 84 days of application :

- 93% of volunteers found the product very pleasant or pleasant;
- 100% of volunteers found the product appearance very pleasant or pleasant;
- 100% of volunteers found the product texture very pleasant or pleasant;
- 100% of volunteers found the product color very pleasant or pleasant;
- 98% of volunteers found the product scent very pleasant or pleasant.

Therefore, as demonstrated above in the questionnaire of table 76, 85% of volunteers were satisfied with the product/serum after 84 days of use, and 78% of volunteers would purchase the serum regardless of its price. Moreover, 84% of volunteers would recommend the serum after 84 days of use.

Conclusion of the test concerning the evaluation of the anti-stain effectiveness of the composition of the invention:

The aim of this test was to objectively evaluate, on a panel of 40 volunteers, the anti-spot (dark spot) efficacy and tolerance of a composition according to the invention through biometric measurements, clinical scoring, and self-assessment questionnaires over 84 days of twice-daily application at home.

Thus, based on dermatological testing and as demonstrated above in this example 5, it can be concluded that the skin acceptability of a composition according to the invention is very good after 84 days of twice-daily use on volunteers with all skin types, with dull complexions and melasma with an epidermal component, with actinic lentigo spots, PIH pigment spots related to post-acne marks.

Moreover, based on the instrumental results and clinical scores presented hereabove, the results demonstrate that after only 28 days, the composition according to the invention significantly reduces the appearance of dark spots, for example. their intensity, number and size. The composition according to the invention also advantageously significantly improves the radiance, evenness/uniformity and luminosity of the complexion, after 28 days.

In addition, for example after 84 days, the composition according to the invention significantly reduces the color difference between the spots and the surrounding skin.

Therefore, it has been demonstrated above that the composition according to the invention has an advantageous anti-spot effect after 28 days of twice-daily application. These tests have thus shown that the composition according to the invention allows the treatment of brown skin spots and/or hyperpigmented spots, i.e. allows to whiten the skin.

## Example 6: Non-comedoaenicitv and skin tolerance/cutaneous acceptability test (clinical test)

In this example, the non-comedogenicity and the skin tolerance of the same example of a composition according to the invention as disclosed in example 1 above was assessed. Area of application : Face. Study duration: 28 days

**Objectives** :

To evaluate for the investigational product:

- its capacity to maintain skin in good condition (cutaneous acceptability) by clinical examination under dermatological control;
- its comedogenic potential by counting of retentional and inflammatory lesions by the technician under dermatological control;
- subjectively its properties, its efficacy and its future use by analysis of the subjects' answers to a subjective evaluation questionnaire.

**Recruitment** :

A panel of 30 volunteers (16 women and 14 men) satisfying the inclusion and non-inclusion criteria with combination to oily skin (33% oily skin, 67% combination skin), phototype III and IV (17% III, 83% IV), aged 18 to 39 (mean age : 28 $\pm$ 1).

Inclusion criteria: gender: female and/or male; age: between 18 and 40 years old; volunteers with combination to oily skin prone to acne on the face; healthy subject; subject who has given free, informed, and express written consent; subject willing to adhere to the study protocol and procedures.

Non-inclusion criteria: for women, pregnant or breastfeeding or planning to become pregnant during the study; skin condition pathology in the measurement area (other than acne); any topical or systemic treatment taken in the weeks prior to the test that may interfere with the assessment of the skin acceptability of the product being studied; acne treatment (topical or systemic): retinoids: oral in the last 6 months, topical in the previous month, antibiotics: oral in the last 3 months, topical in the previous month, zinc in the last 3 months ; start, discontinuation, or change in hormone treatment (including birth control pills) within the last 3 months; use of Androcur® within the last 3 months; start, discontinuation or change in treatment with cyproterone or drospirenone or with a dual indication for contraception and acne (such as Diane 35® or Jasmine® or generics) in the last 6 months; anti-acne cosmetics in the last 2 weeks; any other topical or systemic treatment taken in the weeks prior to the test that may interfere with the assessment of the skin acceptability of the study product (at the investigator's discretion) ;volunteers who have visited a beautician for skin cleansing in the month prior to and during the study; volunteers who have manipulated their acne lesions; volunteers who have undergone surgery under general anesthesia in the previous month; excessive exposure to sunlight or UV rays in the previous month; volunteers participating in another clinical trial during the study period.

The volunteers included in the study did not take any concomitant treatments that could lead to a change in skin condition.

**Product:**

Description: DEPIWHITE SERUM - Intensive anti-spot concentrate. Instructions for use : Apply morning and evening, on the face. 1 or 2 squeezes for 1 application.

**Protocol:**

At DO : at the test center; informing subjects about the objectives of the study, the procedures, and the risks of the study through verbal explanation and the use of an information sheet; verification of inclusion and exclusion criteria; clinical examination of the skin in the application area by the technician under dermatological supervision; counting of retention and inflammatory lesions by the technician under dermatological supervision; questioning of subjects about their usual feelings of discomfort; distribution to subjects: the investigational product with an explanation of its use, the daily monitoring form to record any feelings of discomfort or medication taken.

From DO to D27 (at home) : application of the test product by the subject.

At D28: at the test center; checking the completed daily monitoring form and the investigational product; clinical examination of the skin in the application area by the technician under dermatological supervision; counting of retention and inflammatory lesions by the technician under dermatological supervision; questioning of subjects about any discomfort they felt and/or observed during the study; completion of the subjective evaluation questionnaire by the subjects.

**Calculation formulas** : comedogenic potential
Gross variations (Δ) are calculated using the following formula :

$$\Delta = ZT_{ti} - ZT_{t0}$$

with:

ZT: value obtained on the area treated with the tested product

t0: before application of the product
ti: at each kinetic time point after application of the tested product

**Statistical method** :

- Skin acceptability : only descriptive analyses (mean, SEM, frequency, etc.) were calculated.
- Comedogenic potential :
Normality of differences : determined by Shapiro-Wilk test ($\alpha$ = 0.01).

In function of the results : application of Student test (T test), for paired data, or application of Wilcoxon signed-rank test.
If p-value $\leq$ 0.05 : statistically significant difference.

- Subjective evaluation questionnaire : descriptive analyses (mean, SEM, frequency, etc.) were calculated.

In addition, in order to assess the significance of the responses to the subjective evaluation questionnaire, a 95% confidence interval is determined using the Wilson method and then compared to the theoretical proportion of 50%.
If p-value $\leq$ 0.05 : the proportion of positive responses is significantly higher than 50%.

**Methods** :

- Skin tolerance :

Before (D0) and after using the product (D28), the technician conducted an examination of the area under study under dermatological supervision to assess each of the following parameters: erythema, edema, dryness, desquamation and roughness.
On Day 0, volunteers were also asked about their usual functional and physical signs: tightness, tingling, itching, burning sensation, heat, redness/erythema, edema, dryness, desquamation and roughness.
At the end of the study, the skin acceptability of the product was assessed by taking into account the relevant elements reported by the volunteer (functional and physical signs) and those observed during the examination (clinical signs). A comparison of these results then allowed a conclusion to be drawn r
garding the final skin acceptability of the product studied.

- Comedogenic potential :

The comedogenic potential was assessed after 28 days of application, by comparing the number of elements on the face before application (D0). On D0 and D28, the technician, under dermatological supervision, counted the number of open comedones and microcysts (retention lesions) as well as papules and pustules (inflammatory lesions) on the entire face (except for the nasal pyramid, the edge of the scalp, the lip contour and the chin fold).
The variations (D28-D0) were then calculated for each type of lesion. Statistics were compiled to determine the significance of any variation.
Non-comedogenic product : does not cause a significant increase in the number of retention elements.
Non-acneogenic product : does not cause a significant increase in the number of inflammatory elements.

- Subjective assessment questionnaire :

A questionnaire, prepared by the promoter, is completed by volunteers at the end of the study in order to subjectively evaluate the characteristics of the product studied, its effectiveness, and its subsequent use.
In order to evaluate the significance of the responses, a 95% confidence interval is determined for each question using the Wilson method and then compared to the theoretical proportion of 50%.

**Results and conclusion:**

No adverse events were encountered during the study.

- Skin tolerance :

No abnormal clinical signs were observed at D28 and no subjects reported functional or physical signs at D28.

As demonstrated above, none of the 30 volunteers reported any functional or physical signs or presented any relevant clinical signs on day 28. Thus, it has been demonstrated that an example of a composition according to the invention is very well tolerated by the skin.

- Comedogenic potential :

Table 80 : results regarding the comedogenic potential of the composition of the invention

| | Number of subjects | D0 Mean ± SEM | D28 Mean ± SEM | ΔD28 Mean ± SEM | Statistical analysis | | |
|---|---|---|---|---|---|---|---|
| | | | | | p | Statistical tests | Significance |
| Open comedones | 30 | 7.3 ± 0.6 | 7.0 ± 0.5 | -0.4 ± 0.1 | 0.0010 | Wilcoxon | Yes |
| Microcysts | 30 | 6.5 ± 0.5 | 5.7 ± 0.5 | -0.9 ± 0.2 | < 0.0001 | Wilcoxon | Yes |
| Total retention elements | 30 | 13.9 ± 0.7 | 12.6 ± 0.7 | -1.2 ± 0.2 | < 0.0001 | Wilcoxon | Yes |

Therefore, as demonstrated above in table 80, an example of a composition according to the invention did not cause a significant increase in the number of blackheads and microcysts after 28 days of use. It can therefore be considered non-comedogenic and non-acnegenic.

- Subjective assessment questionnaire :

Table 81 : self-assessment questionnaire, at D28

| | After 28 days | | |
|---|---|---|---|
| | | Significance | |
| | % of positive responses | Lower limit > 50% | IC 95% |
| The texture is pleasant | 100 | Yes | (89%-100%) |
| Agree | 100 | Yes | (89%-100%) |
| Somewhat agree | 0 | No | (0%-11%) |
| The scent is pleasant | 100 | Yes | (89%-100%) |
| Agree | 80 | Yes | (63%-90%) |
| Somewhat agree | 20 | No | (10%-37%) |
| The product is easy to apply | 100 | Yes | (89%-100%) |
| Agree | 100 | Yes | (89%-100%) |
| Somewhat agree | 0 | No | (0%-11%) |
| The product penetrates quickly | 100 | Yes | (89%-100%) |
| Agree | 100 | Yes | (89%-100%) |
| Somewhat agree | 0 | No | (0%-11%) |
| The product has a non-sticky finish on the skin | 100 | Yes | (89%-100%) |
| Agree | 100 | Yes | (89%-100%) |
| Somewhat agree | 0 | No | (0%-11%) |

(continued)

| | After 28 days | | |
| --- | --- | --- | --- |
| | | Significance | |
| | % of positive responses | Lower limit > 50% | IC 95% |
| The product has a non-greasy finish on the skin | 100 | Yes | (89%-100%) |
| Agree | 100 | Yes | (89%-100%) |
| Somewhat agree | 0 | No | (0%-11%) |
| The product mattifies the skin | 100 | Yes | (89%-100%) |
| Agree | 97 | Yes | (83%-99%) |
| Somewhat agree | 3 | No | (1%-17%) |
| The product can be a good makeup base (n=16) | 100 | Yes | (89%-100%) |
| Agree | 100 | Yes | (89%-100%) |
| Somewhat agree | 0 | No | (0%-11%) |
| A cream can be applied over the product | 100 | Yes | (89%-100%) |
| Agree | 97 | Yes | (83%-99%) |
| Somewhat agree | 3 | No | (1%-17%) |
| The complexion is more radiant | 97 | Yes | (84%-100%) |
| Agree | 97 | Yes | (83%-99%) |
| Somewhat agree | 0 | No | (0%-11%) |
| The complexion is fresher | 97 | Yes | (84%-100%) |
| Agree | 97 | Yes | (83%-99%) |
| Somewhat agree | 0 | No | (0%-11%) |
| The complexion is more uniform | 97 | Yes | (84%-100%) |
| Agree | 97 | Yes | (83%-99%) |
| Somewhat agree | 0 | No | (0%-11%) |
| The complexion is more unified | 97 | Yes | (84%-100%) |
| Agree | 97 | Yes | (83%-99%) |
| Somewhat agree | 0 | No | (0%-11%) |
| The skin is brighter | 97 | Yes | (84%-100%) |
| Agree | 97 | Yes | (83%-99%) |
| Somewhat agree | 0 | No | (0%-11%) |
| The skin is visibly more hydrated | 100 | Yes | (89%-100%) |
| Agree | 97 | Yes | (83%-99%) |
| Somewhat agree | 3 | No | (1%-17%) |
| The skin feels comfortable after applying the product | 100 | Yes | (89%-100%) |
| Agree | 100 | Yes | (89%-100%) |
| Somewhat agree | 0 | No | (0%-11%) |
| The product does not cause any irritation | 100 | Yes | (89%-100%) |
| Agree | 100 | Yes | (89%-100%) |
| Somewhat agree | 0 | No | (0%-11%) |
| The product is suitable | 100 | Yes | (89%-100%) |
| for my skin type | | | |
| Agree | 100 | Yes | (89%-100%) |
| Somewhat agree | 0 | No | (0%-11%) |
| The product is suitable for sensitive skin | 100 | Yes | (89%-100%) |
| Agree | 100 | Yes | (89%-100%) |
| Somewhat agree | 0 | No | (0%-11%) |

(continued)

| | After 28 days | | |
| --- | --- | --- | --- |
| | % of positive responses | Significance | |
| | | Lower limit > 50% | IC 95% |
| The product is suitable for combination to oily skin | 100 | Yes | (89%-100%) |
| Agree | 100 | Yes | (89%-99%) |
| Somewhat agree | 0 | No | (0%-11%) |
| Would like to continue using this product | 97 | Yes | (83%-99%) |
| After this test and regardless of the price, would buy this product | 97 | Yes | (83%-99%) |

Thus, in view of the results of table 81, the inventors have demonstrated hereabove that an example of a composition according to the invention is very well tolerated by the skin; is non-comedogenic and non-acnegenic; and is appreciated by volunteers for its features and effectiveness.

Therefore, it has been demonstrated that the claimed composition may bear the statements "tolerance tested under dermatological control" and "non-comedogenic and non-acnegenic".

### Example 7 : Study of the skin penetration of delivery systems

The goal of the present example is to compare the skin penetration capabilities of two delivery systems, composition/liposomes of table 2 and composition/liposomes of table 3 without tranexamic acid, with the help of a fluorescent dye and confocal microscopy.

**Materials**

- 100 µL micropipettes
- 50 mL Falcon tubes

**Reagents** :

- 18:1 PE CF (1,2-dioleoyl-sn-glycero-3-phosphoethanolamine-N-(carboxyfluorescein) (ammonium salt))
- 4',6-diamidino-2-phenylindole (DAPI)
- composition of table 2 without tranexamic acid (Composition A TA-)
- composition of table 3 without tranexamic acid (Composition B TA-)
- PFA (Paraformaldehyde)
- Saline buffer

**Equipment** :

- Franz diffusion cells
- Confocal microscope

**Procedure** :

Fluorescent liposomes of table 2 without tranexamic acid and liposomes of table 3 without tranexamic acid were synthesized including rhodamine-labelled phospholipids (18:1 PE CF) in the membrane of the liposomes. Products were characterized prior to the analysis, to assure that they were in accordance with the specifications regarding size and polydispersity index.

Frozen skin samples were hydrated and set to room temperature using a saline buffer. Once the skin was at working conditions, it was mounted on the Franz diffusion cells, and the water bath was set to 37°C, to simulate physiological conditions. 100 μL of the selected product were added to receptor compartment of the Franz diffusion cells, and the compound was let to diffuse for different times (8, 16 and 24 h), to study the release profile of each delivery system. After those times, the skin sample was rinsed off with ultrapure water and fixed with paraformaldehyde (PFA) at 4°C and sent to the pathology service at the University of Salamanca, for further analysis.

These skin sections were dyed with DAPI, a fluorescent stain with specificity for cellular nuclei with an emission maximum at 461 nm (blue) and observed with a confocal microscope. This dye will allow us to localize cells throughout the tissue. Also, a CD31-specific antibody was added to the sample to locate the endothelial cells. This antibody was labelled with a green chromophore, so all three fluorescent dyes could be observed simultaneously.

**Results and conclusions** :

### • Delivery system penetration capabilities comparison

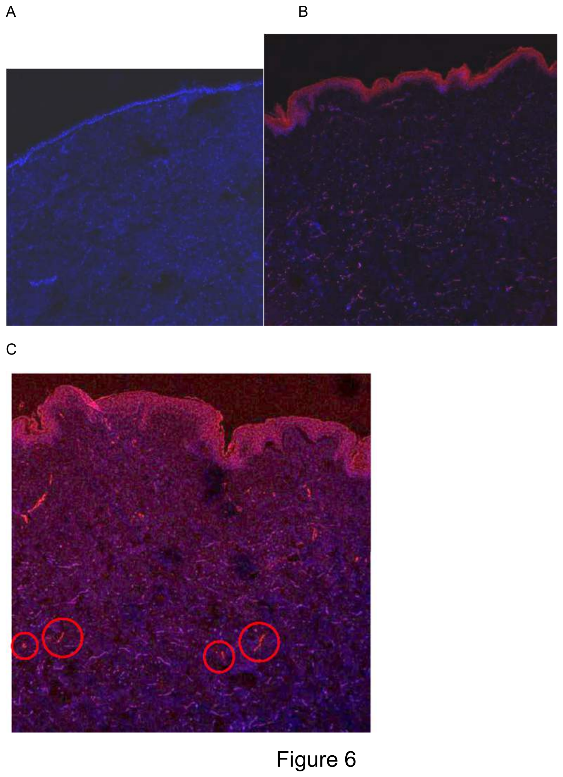

In Figure 6, a comparison of a skin sample without liposomes (A, nuclei in blue to differentiate layers), one with table 3 liposomes (B) and one with table 2 liposomes (C) is shown. It can be observed how the table 2 liposomes penetrate deeper layers of the skin, reaching the dermis (elongated cells in circles image C), while the table 3 liposome stays at the epidermis layer (image B). This comes to show the increased penetration capability of the table 3 liposomes compared to table 2 liposomes.

### • Liposomes interactions with fibroblasts

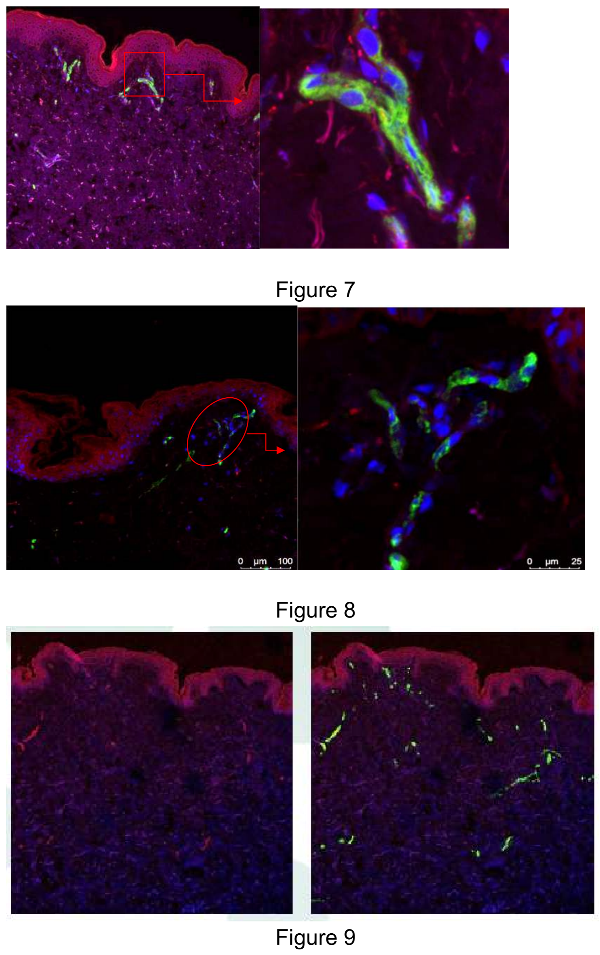

In Figure 7 and Figure 8, it can be observed the specificity shown by the liposomes towards the endothelial cells. In figure 7, table 2 liposomes were left to apply for 16 hours (endothelial cells (in green) with table 2 liposomes (in red), and in figure 8, for 24 hours.

In Figure 9, a comparison of the same image with (B) and without (A) the green channel (corresponding to the endothelial cells) can be observed. As the liposomes are attached to the endothelial tissue, a yellow color is observed in image B, product of the mixture of the red (liposomes) and green (endothelial cells) channel.

### • Quantitative analysis

To analyze the penetration capabilities of both delivery systems, ethyl ascorbic acid (EAA) was encapsulated in both table 3 and table 2 liposomes. EAA was chosen as a model analyte due to its stability and high UV-Vis absorbance, which make it a perfect molecule to work with and to analyze via HPLC.

EAA was quantified by HPLC using a Shimadzu® LC-20AD equipped with a UV detector and a Kromaphase 100 C18, 5.0 μm (150 x 4.6 mm). The chromatographic conditions were as follows:

- Mobile phase: 80% 25 mM NaH2PO4/20% MeOH
- Flow rate: 1 mL/min
- Detection wavelength: 245 nm
- Retention time: 4.5 min

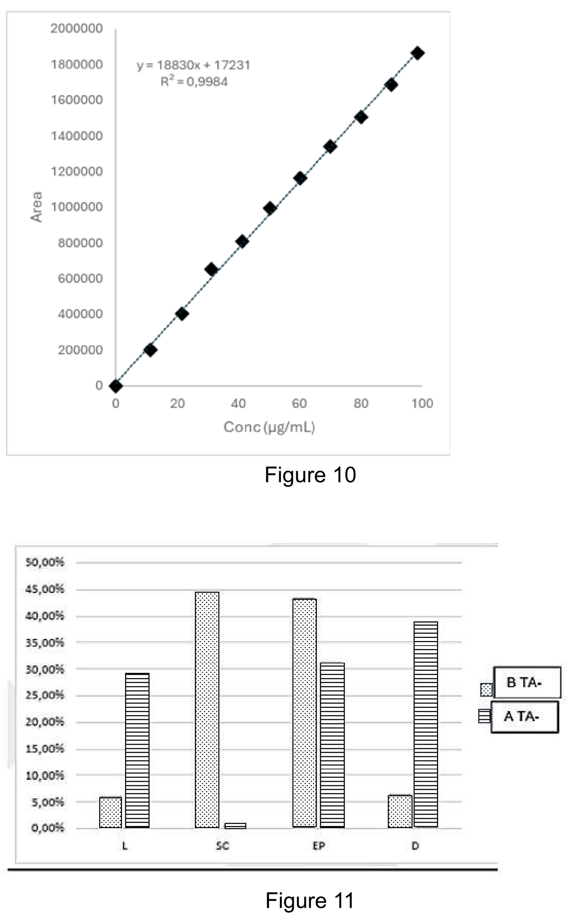

A calibration curve (Figure 10) was built between 0 and 100 ppm. The correlation coefficient (R2) obtained was 0.9984, and the limits of detection and quantification achieved were 4.6 μg/mL and 13.9 μg/mL.

EAA penetration profile was determined using Franz diffusion cells and dermatomized human skin samples. Skin samples were thawed and rehydrated on a 0,9% saline buffer for 20 minutes. After that time, skin discs were placed on top of the acceptor compartments of Franz diffusion cells, previously filled up to meniscus with the same saline buffer. The donor compartment was placed on top of the skin, and 100 μL of the tested product were added. The system was sealed up with Parafilm® M, to avoid evaporation, and the product was left to apply for 16 h.

The results are shown in Figure 11. This figure clearly demonstrates that the concentration in the dermis layer is much higher in the case of liposomes of composition A (table 2) than with liposomes of composition A (table 3) (around 8 times more)

### Claims

1.    Cosmetic non-therapeutic use of a topical cosmetic composition comprising tranexamic acid encapsulated in a liposome for treating melanin or hyperpigmented spot

2. Cosmetic non-therapeutic use of a topical cosmetic composition comprising tranexamic acid encapsulated in a liposome for one or more of the following applications: brightening the skin, reducing and/or treating the skin brown spots, reducing and/or treating hyperpigmented spots.

3. Cosmetic use according to claim 1 or 2 wherein the composition comprise % by weight relative to the total weight of the composition from 0.01 to 1% of tranexamic acid, preferably from 0.05 to 0.15%, more preferably from 0.05 to 0.015%.

4. Cosmetic use according to any one of the preceding claims, wherein the composition further comprises at least one ingredient selected from the group comprising ascorbic acid, zinc salt, olive leaf extract, oleuropeine, niacinamide, retinyl palmitate, hexylresorcinol, alpha hydroxy acid (aha), poly hydroxy acid, and phosphatase activator.

5. Cosmetic use according to claim 4 wherein the concentration of ascorbic acid is from 0.01 to 0.1% by weight relative to the total weight of the composition, preferably from 0.01 to 0.03%.

6. Cosmetic use according to claim 4 or 5 wherein the concentration of oleuropeine is from 0.01 to 0.1% by weight relative to the total weight of the composition, preferably from 0.01 to 0.09%.

7. Cosmetic use according any one any one of claims 4 to 6 wherein the concentration of niacinamide is from 1 to 10% by weight relative to the total weight of the composition, preferably from 0.4 to 4%.

8. Cosmetic use according any one of claims 4 to 7 wherein the concentration of retinyl palmitate is from 0.01 to 1% by weight relative to the total weight of the composition, preferably from 0.02 to 0.1%, more preferably from 0.01 to 0.02%.

9. Cosmetic use according any one of claims 4 to 8 wherein the concentration of zinc salt is from 0.01 to 1% by weight relative to the total weight of the composition, preferably from 0.05 to 1%.

10. Cosmetic use according any one of claims 4 to 9 wherein the concentration of hexylresorcinol is from 0.1 to 10% by weight relative to the total weight of the composition, preferably from 0.3 to 3%.

11. Cosmetic use according any one any one of claims 4 to 10 wherein the concentration of alpha hydroxy acid (aha) is from 0.1 to 10% by weight relative to the total weight of the composition, preferably from 0.1 to 5%, more preferably from 0.3 to 3%.

12. Cosmetic use according any one of claims 4 to 11 wherein the concentration of poly hydroxy acid (pha) is from 0.1 to 10% by weight relative to the total weight of the composition, preferably from 0.1 to 5%, more preferably from 0.3 to 3%.

13. Cosmetic use according any one of the preceding claims wherein the composition further comprises at least one phosphatase activator selected from the group comprising magnesium ions ($Mg^{2+}$), cupric ions ($Cu^{2+}$), zinc ions ($Zn^{2+}$), manganese ions ($Mn^{2+}$) and mixtures thereof.

14. Cosmetic use according to claim 13 wherein the phosphatase activator is the combination of at least two phosphatase activators selected from the group comprising magnesium sulfate, manganese chloride, magnesium chloride, manganese sulfate.

15. Cosmetic use according to claim 13 or 14 wherein the concentration of phosphatase activator is from 0.01 to 10% by weight relative to the total weight of the composition, preferably from 0.01 to 3%, more preferably from 0.03 to 3%.

16. Cosmetic use according any one of the preceding claims wherein the composition is a serum.

17. A non-therapeutic cosmetic method for brightening the skin, reducing and/or treating the skin brown spots, reducing and/or treating hyperpigmented spots, comprising applying the topical cosmetic composition as defined in any of claim 1 to 16 to skin

Figure 1

| | L | EP | D |
|---|---|---|---|
| B | 45,78% | 37,68% | 16,54% |
| A | 24,36% | 52,72% | 22,92% |
| Free TA | 71,96% | 25,06% | 2,98% |

Figure 2

SC

EP

D

Figure 3

L

F

Figure 4

Figure 5

A

B

C

Figure 6

Figure 7

Figure 8

Figure 9

Figure 10

Figure 11

Europäisches
Patentamt

European
Patent Office

Office européen
des brevets

## EUROPEAN SEARCH REPORT

**Application Number**

EP 26 15 1632

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/055741 A1 (ALASTIN SKINCARE INC [US]) 6 April 2023 (2023-04-06) * claims 1, 33-36, 50, 58-62 * * page 1, paragraph 3 - page 3, paragraph 4 * * page 14, paragraph 49 - page 15, paragraph 50 * | 1-17 | INV. A61K8/11 A61K8/44 A61Q19/00 A61Q19/02 |
| X | CN 109 481 321 B (GUANGZHOU CHENXI CHEMICAL CO LTD) 24 May 2022 (2022-05-24) * claims 1, 3, 4 * * paragraph [0028] * | 1-17 | |
| X | CN 113 679 631 A (CHANGZHOU KINYOND PHARMACEUTICAL MFG CO LTD) 23 November 2021 (2021-11-23) * paragraph [0001] * * paragraph [0007] * * claims 1, 9, 10 * | 1-17 | |
| X | CN 110 420 130 A (QU WEIHAN) 8 November 2019 (2019-11-08) * examples 4-7 * | 1-17 | **TECHNICAL FIELDS SEARCHED (IPC)** A61K A61Q |
| Y | US 2024/066086 A1 (WANG TONY [US] ET AL) 29 February 2024 (2024-02-29) * page 1, paragraph 6-12 * * page 2, paragraph 37 - page 3, paragraph 39 * * example 3 * * claims 26-38 * | 1-17 | |

-/--

The present search report has been drawn up for all claims

| Place of search | Date of completion of the search | Examiner |
|---|---|---|
| The Hague | 4 June 2026 | Collins, Sally |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .....................................................................................

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 26 15 1632

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CLIN TEY HL ET AL: "Skin Lightening Efficacy of Tranexamic Acid in Topical Delivery Systems", CLINICAL & EXPERIMENTAL DERMATOLOGY AND THERAPIES, vol. 9, no. 3, 26 September 2024 (2024-09-26), XP093402134, ISSN: 2575-8268, DOI: 10.29011/2575-8268.100229 Retrieved from the Internet: URL:https://www.gavinpublishers.com/assets/articles_pdf/Skin-Lightening-Efficacy-of-Tranexamic-Acid-in-Topical-Delivery-Systems.pdf> * the whole document * ----- | 1-17 | |
| | | | **TECHNICAL FIELDS SEARCHED (IPC)** |

The present search report has been drawn up for all claims

| Place of search | Date of completion of the search | Examiner |
|---|---|---|
| The Hague | 4 June 2026 | Collins, Sally |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 26 15 1632

This annex lists the patent family members relating to the patent documents cited in  the above-mentioned European search report.
The members are as contained in the European Patent Office EDP file on
The European Patent Office is in no way liable for these particulars which are merely given for the purpose of information.

04-06-2026

| Patent document cited in search report | | Publication date | Patent family member(s) | | Publication date |
|---|---|---|---|---|---|
| WO 2023055741 | A1 | 06-04-2023 | AU | 2022357284 A1 | 16-05-2024 |
| | | | CA | 3233323 A1 | 06-04-2023 |
| | | | CN | 118678945 A | 20-09-2024 |
| | | | CO | 2024005318 A2 | 30-05-2024 |
| | | | EP | 4408541 A1 | 07-08-2024 |
| | | | JP | 2024535424 A | 30-09-2024 |
| | | | KR | 20240082385 A | 10-06-2024 |
| | | | US | 2024225985 A1 | 11-07-2024 |
| | | | WO | 2023055741 A1 | 06-04-2023 |
| CN 109481321 | B | 24-05-2022 | NONE | | |
| CN 113679631 | A | 23-11-2021 | NONE | | |
| CN 110420130 | A | 08-11-2019 | NONE | | |
| US 2024066086 | A1 | 29-02-2024 | NONE | | |

EPO FORM P0459

For more details about this annex : see Official Journal of the European Patent Office, No. 12/82

**REFERENCES CITED IN THE DESCRIPTION**

**Non-patent literature cited in the description**

- **AKBARZADEH et al.** Liposome: classification, preparation, and applications. *Nanoscale Research Letters*, 2013, vol. 8 (1), 102
- **MOZAFARI, M.R.** Liposomes: an overview of manufacturing techniques. *Cellular and Molecular Biology Letters,*, 2005, vol. 10 (4), 711-719
- **FITZPATRICK, T. B.** The validity and practicality of sun-reactive skin types I through VI. *Archives of Dermatology*, 1988, vol. 124 (6), 869-871